# EUROPEAN PATENT SPECIFICATION

(11) **EP 2 069 403 B1**
(45) Date of publication and mention of the grant of the patent: **07.05.2014**
(21) Application number: 07867197.1
(22) Date of filing: 02.10.2007
(51) Int. Cl.: C07K 16/28

(54) **HIGH AFFINITY HUMAN ANTIBODIES TO HUMAN IL-4 RECEPTOR**
MENSCHLICHE ANTIKÖRPER MIT HOHER AFFINITÄT FÜR DEN MENSCHLICHEN IL-4-REZEPTOR
ANTICORPS HUMAINS À HAUTE AFFINITÉ VIS-À-VIS DU RÉCEPTEUR IL-4 HUMAIN

(30) Priority: 02.10.2006 US 848694 P; 24.08.2007 US 957738 P
(43) Date of publication of application: 17.06.2009
(60) Divisional application: 14162081.5
(73) Proprietor: Regeneron Pharmaceuticals, Inc., Tarrytown, NY 10591 (US)
(72) Inventor: STEVENS, Sean, Mohegan Lake, NY 10547 (US); HUANG, Tammy T., Golden Bridge, NY 10526 (US); MARTIN, Joel H., Putnam Valley, NY 10579 (US); FAIRHURST, Jeanette L., White Plains, NY 10603 (US); RAFIQUE, Ashique, Yonkers, NY 10705 (US); TORRES, Marcela, New Milford, NJ 07646 (US); POBURSKY, Kevin J., Beacon, NY 12508 (US); LEIDICH, Raymond W., Middletown, NY 10940 (US); WINDSOR, Joan A., Brooklyn, NY 11216 (US); MIKULKA, Warren R., Brewster, NY 10509 (US); AHRENS, Diana M., Mahopac, NY 10541 (US); SHI, Ergang, Ossining, NY 10562 (US); PAPADOPOULOS, Nicholas J., Lagrangeville, NY 12540 (US)
(74) Representative: Bentham, Andrew
(86) International application number: PCT/US2007/021210
(87) International publication number: WO 2008/054606

(56) References cited:
- EP-A- 0 604 693
- WO-A-01/92340
- WO-A-2005/047331
- WO-A-2005/085284
- WO-A-2006/072564
- US-A1- 2005 255 532
- US-A1- 2005 282 181
- US-A1- 2007 041 976
- ZURAWSKI S M ET AL: "THE PRIMARY BINDING SUBUNIT OF THE HUMAN INTERLEUKIN-4 RECEPTOR IS ALSO A COMPONENT OF THE INTERLEUKEN-13 RECEPTOR" JOURNAL OF BIOLOGICAL CHEMISTRY, AMERICAN SOCIETY OF BIOLOCHEMICAL BIOLOGISTS, BIRMINGHAM,; US, vol. 270, no. 23, 9 June 1995 (1995-06-09), pages 13869-13878, XP000986235 ISSN: 0021-9258
- HOLT L J ET AL: "Domain antibodies: proteins for therapy" TRENDS IN BIOTECHNOLOGY, ELSEVIER PUBLICATIONS, CAMBRIDGE, GB, vol. 21, no. 11, 1 November 2003 (2003-11-01), pages 484-490, XP004467495 ISSN: 0167-7799
- DAVIES J ET AL: "Affinity improvement of single antibody VH domains: residues in all three hypervariable regions affect antigen binding" IMMUNOTECHNOLOGY, ELSEVIER SCIENCE PUBLISHERS BV, NL, vol. 2, no. 3, 1 September 1996 (1996-09-01), pages 169-179, XP004070292 ISSN: 1380-2933

## Description

### BACKGROUND

Interleukin-4 (IL-4, also known as B cell stimulating factor or BSF-1) was originally characterized by its ability to stimulate the proliferation of B cells in response to low concentrations of antibodies directed to surface immunoglobulin. IL-4 has been shown to possess a broad spectrum of biological activities, including growth stimulation of T cells, mast cells, granulocytes, megakaryocytes and erythrocytes. IL-4 induces the expression of class II major histocompatibility complex molecules in resting B cells, and enhances the secretion of IgE and IgG1 isotypes by stimulated B cells.

The biological activities of IL-4 are mediated by specific cell surface receptors for IL-4. Human IL-4 receptor alpha (hIL-4R) (SEQ ID NO:1) is described in, for example, U.S. Patent No. 5,599,905, 5,767,065,'and 5,840,869. Antibodies to hIL-4R are described in U.S. Patent No. 5,717,072.

The biological activities of IL-4 are mediated by specific cell surface receptors for IL-4. Human IL-4 receptor alpha (hIL-4R) (SEQ ID NO:1) is described in, for example, U.S. Patent No. 5,599,905, 5,767,065, and 5,840,869. Antibodies to hIL-4R are described in U.S. Patent No. 5,717,072 and 7,186,809.

Methods to produce antibodies useful as human therapeutics include generating chimeric antibodies and humanized antibodies (see, for example, US 6,949,245). See, for example, WO 94/02602 (Abgenix) and US 6,596,541 (Regeneron Pharmaceuticals) describing methods of generating nonhuman transgenic mice capable of producing human antibodies.

Methods for using antibodies to hIL-4R are described in U.S. Patent Nos. 5,714,146; 5,985,280; and 6,716,587.

### BRIEF SUMMARY OF THE INVENTION

The invention provides:
- An antibody or antigen-binding fragment thereof, that specifically binds hIL-4R (SEQ ID NO:1) with a K_{D} of about 200 pM or less, as measured by surface plasmon resonance, comprising the CDRs from a HCVR and a LCVR sequence, wherein the HCVR/LCVR sequences are selected from SEQ ID NOs: 579/59 and 581/59.
- An isolated nucleic acid molecule encoding an antibody or antigen-binding fragment of the invention.
- A vector comprising such a nucleic acid sequence.
- A host-vector system for the production of an antibody or antigen-binding fragment of an antibody which specifically binds IL-4 receptor, comprising such a vector, in a suitable host cell.
- A method of producing an anti-IL-4R antibody or antigen-binding fragment thereof, comprising growing cells of a host-vector system of the invention under conditions permissing production of the antibody or fragment thereof and recovering the antibody or fragment so expressed.
- Use of an antibody or antigen-binding fragment of an antibody of the invention in the manufacture of a medicament for use to attenuate or inhibit an IL-4-mediated disease or disorder in a human.
- An antibody or antigen-binding fragment of the invention, for use in a method of treating a disease or disorder in a human, wherein the disease or disorder is improved, ameliorated or inhibited by removal, inhibition or reduction of human interleukin-4 (hIL-4) activity.

Disclosed herein are human antibodies, preferably recombinant human antibodies, that specifically bind human interleukin-4 receptor (hIL-4R). The human antibodies are characterized by binding to hIL-4R with high affinity and by the ability to neutralize hIL-4 activity. In specific instances, the human antibodies are capable of blocking hIL-13/hIL-13R1 complex binding to hIL-4R, and thus inhibit signaling by hIL-13. The antibodies can be full-length (for example, an IgG1 or IgG4 antibody) or may comprise only an antigen-binding portion (for example, a Fab, F(ab')₂ or scFv fragment), and may be modified to effect functionality, e.g., to eliminate residual effector functions (Reddy et al. (2000) J. Immunol. 164:1925-1933).

Also disclosed herein is an antibody or antigen-binding fragment thereof, that specifically binds hIL-4R (SEQ ID NO:1) with a K_{D} of about 200 pM or less, as measured by surface plasmon resonance. In a more specific embodiment, the antibody or antigen-binding portion thereof exhibits a K_{D} of less than about 150 pM, or less than about 50 pM, or less than about 20 pM. In various embodiments, the antibody or antigen-binding fragment blocks hIL-4 activity with an IC₅₀ of about 200 pM or less, as measured by STAT6 luciferase bioassay. In more specific embodiments, the antibody or antigen-binding fragment exhibits an IC₅₀ of about 150 pM or less, or about 100 pM or less, or even about 50 pM or less as measured by luciferase bioassay. In various embodiments, the antibody or antigen-binding fragment blocks hIL-13 activity with an IC₅₀ of about 100 pM or less, as measured by STAT6 luciferase bioassay. In more specific embodiments, the antibody or antigen-binding fragment exhibits an IC₅₀ of about 75 pM or less, or about 50 pM or less, or even about 20 pM or less.

In a second aspect, the antibody of the invention comprises a heavy chain variable region (HCVR) selected from the group consisting of SEQ ID NO: 579 and 581, or a substantially similar sequence thereof.

In a third aspect, the antibody of the invention comprises the light chain variable region (LCVR) of SEQ ID NO: 59 or a substantially similar sequence thereof.

In one embodiment, the antibody or antibody fragment of the invention comprises a HCVR and a LCVR (HCVR/LCVR) selected from the group consisting of SEQ ID NO: 579/59 and 581/59.

Also referred to is a nucleic acid molecule encoding an HCVR, wherein the nucleic acid molecule is a nucleotide sequence selected from the group consisting of SEQ ID NO: 578 and 580, or a substantially identical sequence having at least 95% homology thereof.

Also referred to is a nucleic acid molecule encoding a LCVR, wherein the nucleic acid molecule is SEQ ID NO: 58, or a substantially identical sequence having at least 95% homology thereof.

In one embodiment, the antibody of the invention comprises a HCVR and LCVR encoded by a nucleotide sequence pairs selected from the group consisting of SEQ ID NO: 578/58 and 580/58.

The antibody or antibody fragment comprises a light chain CDR1 of SEQ ID NO: 61; a light chain CDR2 domain of SEQ ID NO: 63; and a light chain CDR3 domain of SEQ ID NO: 65

In a further embodiment, the invention features a human antibody or antibody fragment comprising a light chain CDR1 domain encoded by a nucleotide sequence selected from the group consisting of SEQ ID NO: 60; a light chain CDR2 domain encoded by a nucleotide sequence of SEQ ID NO: 62; and a light chain CDR3 domain encoded by a nucleotide sequence selected SEQ ID NO: 64.

The invention encompasses anti-hIL-4R antibodies having a modified glycosylation pattern. In some applications, modification to remove undesirable glycosylation sites may be useful, or an antibody lacking a fucose moiety present on the oligosaccharide chain, for example, to increase antibody dependent cellular cytotoxicity (ADCC) function (see Shield et al. (2002) JBC 277:26733). In other applications, modification of a galactosylation can be made in order to modify complement dependent cytotoxicity (CDC).

Additionally described herein are recombinant expression vectors carrying the nucleic acid molecules of the invention, and host cells into which such vectors have been included, as are methods of making the antibodies or antigen-binding fragments of the invention obtained by culturing the host cells of the invention. The host cell may be a prokaryotic or eukaryotic cell, preferably the host cell is an *E. coli* cell or a mammalian cell, such as a CHO cell.

Further described herein is a composition comprising a recombinant human antibody that specifically binds hIL-4R and an acceptable carrier.

Disclosed herein are methods for inhibiting hIL-4 activity using an antibody, or antigen-binding portion thereof, disclosed herein. In specific instances, the antibodies disclosed herein also block hIL-13/hIL-13R1 complex binding to hIL-4R. In one instance, the method comprises contacting hIL-4R with the antibody disclosed herein, or antigen-binding portion thereof, such that hIL-4 or hIL-4/hIL-13 activity is inhibited. In another instance, the method comprises administering an antibody disclosed herein, or antigen-binding portion thereof, to a human subject suffering from a disorder that is ameliorated by inhibition of hIL-4 or hIL-4/hIL-13 activity. The disorder treated is any disease or condition that is improved, ameliorated, inhibited or prevented by removal, inhibition or reduction of hIL-4 or hIL-4/hIL-13 activity.

Further encompassed by the invention is the use of an antibody or antigen-binding fragment of an antibody of the invention in the manufacture of a medicament for use to attenuate or inhibit an IL-4-mediated disease or disorder in a human.

IL-4-mediated or related disorders which are treated by the antibodies or antibody fragments of the invention include, for example, arthritis (including septic arthritis), herpetiformis, chronic idiopathic urticaria, scleroderma, hypertrophic scarring, Whipple's Disease, benign prostate hyperplasia, lung disorders, such as mild, moderate or severe asthma, inflammatory disorders such as inflammatory bowel disease, allergic reactions, Kawasaki disease, sickle cell disease, Churg-Strauss syndrome, Grave's disease, prè-eclampsia, Sjogren's syndrome, autoimmune lymphoproliferative syndrome, autoimmune hemolytic anemia, Barrett's esophagus, autoimmune uveitis, tuberculosis, and nephrosis.

Other objects and advantages will become apparent from a review of the ensuing detailed description.

### BRIEF DESCRIPTION OF FIGURES

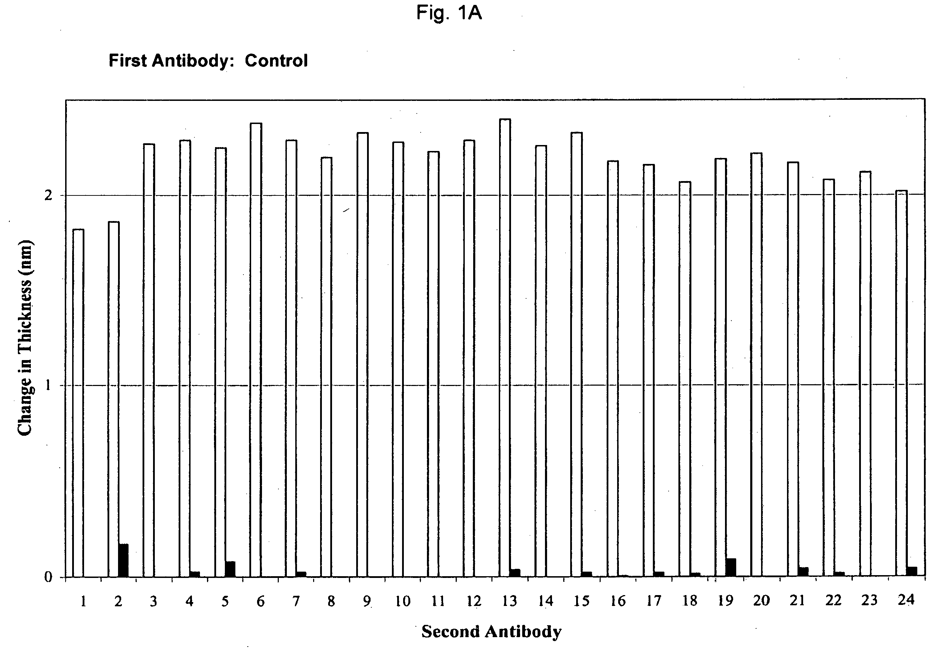

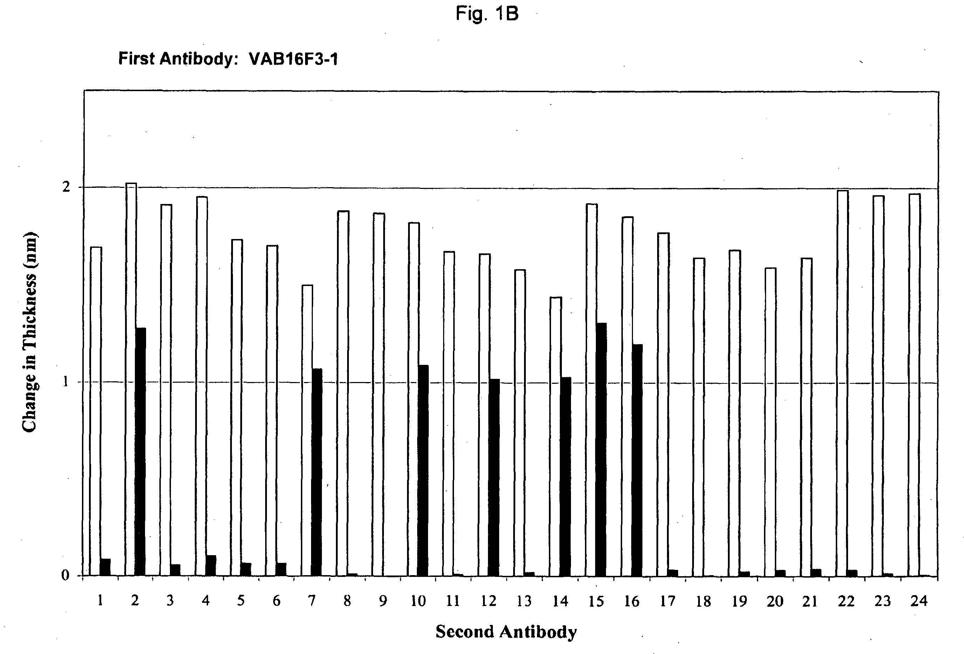

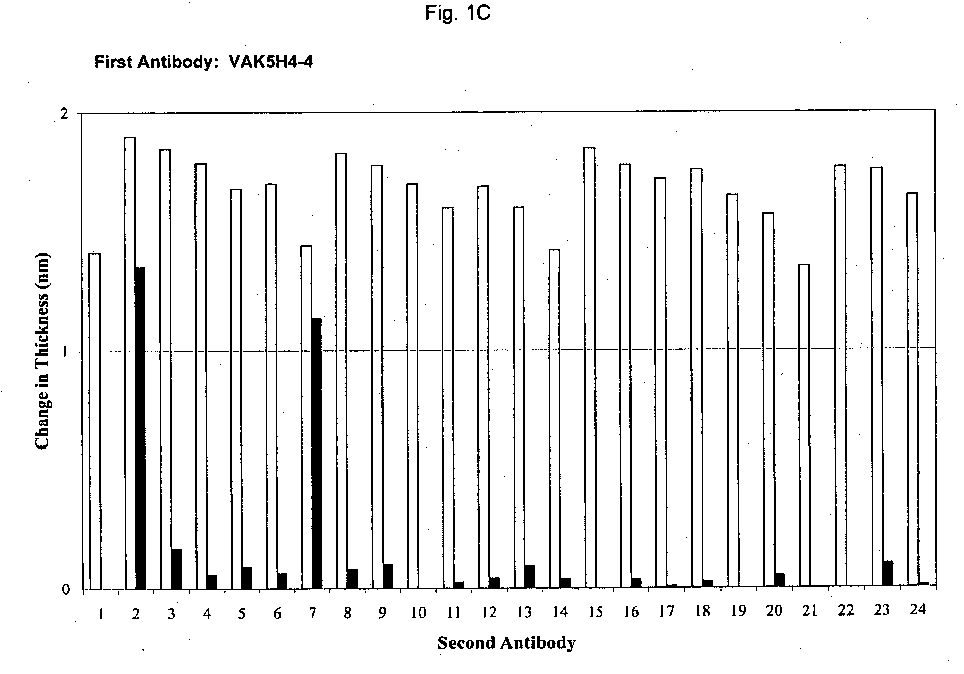

Fig. 1(A-C). Antibody binding profile assessment generated with OCTET™-based sequential binding assays. Fig. 1(A) is a bar graph showing results obtained when the first antibody exposed to bound antigen is the control antibody. Fig. 1(B) the first antibody exposed to bound antigen is VAB16F3-1. Fig. 1(C) the first antibody exposed to bound antigen is VAK5H4-4. Second antibody: 1= control; 2=VX4E7-9; 3= VX3F7-6; 4=VAB16G-1;5= VAB16F3-1; 6=VAB15C8-17; 7= VAB11G8-1; 8= VAB10C1-5; 9= VAB10G8-19; 10=VAB8G10-1; 11=VAB7B9-3; 12=VAB6C10-14; 13= VAB5C5-11; 14=VAB3B4-10; 15=VAB4D5-3; 16= VAB1H1-2; 17= VAK5H4-4; 18= VAK7G8-5; 19=VAK8G11-13; 20= VAK9C6-11; 21= VAK10G6-7; 22=VAK11D4-1; 23=VAK12B11-9; and 24=VAK10G12-5. Open bars show binding level of the first antibody, filled bars show additional binding by a second antibody.

### DETAILED DESCRIPTION

Before the present methods are described, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

### Definitions

The term "human IL4R" (hIL-4R), as used herein, is intended to refer to a human cytokine receptor that specifically binds interleukin-4 (IL-4), IL-4Rα (SEQ ID NO:1). The term "human interleukin-13" (hIL-13) refers to a cytokine that specifically binds IL-13 receptor, and "hIL-13/hIL-13R1 complex" refers to the complex formed by hIL-13 binding to hIL-13R1 complex, which complex binds hIL-4 receptor to initiate biological activity.

The term "antibody", as used herein, is intended to refer to immunoglobulin molecules comprising four polypeptide chains, two heavy (H) chains and two light (L) chains interconnected by disulfide bonds. Each heavy chain comprises a heavy chain variable region (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain (CL1). The VH and VL regions can be further subdivided into regions of hypervariability, termed complementary determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

The term "antigen-binding portion" of an antibody (or simply "antibody portion" or "antibody fragment"), as used herein, refers to one or more fragments of an antibody that retain the ability to specifically bind to an antigen (e.g., hIL-4R). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL1 and CH1 domains; (ii) a F(ab')₂ fragment, a bivalent fragment comprising two F(ab)' fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al. (1989) Nature 241:544-546), which consists of a VH domain; and (vi) an isolated complementary determining region (CDR). Furthermore, although the two domains of the Fv fragment, VL and VH, are coded for by separate genes, they can be joined, using recombinant methods, by a synthetic linker that enables them to be made as a single contiguous chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883. Such single chain antibodies are also intended to be encompassed within the term "antigen-binding portion" of an antibody. Other forms of single chain antibodies, such as diabodies, are also encompassed (see e.g., Holliger et al. (1993) Proc. Natl. Acad Sci. USA 90:6444-6448).

A "neutralizing" or "blocking" antibody, as used herein, is intended to refer to an antibody whose binding to hIL-4R results in inhibition of the biological activity of hIL-4 and/or hIL-13. This inhibition of the biological activity of hIL-4 and/or IL-13 can be assessed by measuring one or more indicators of hIL-4 and/or hIL-13 biological activity known to the art, such as hIL-4- and/or IL-13-induced cellular activation and hIL-4 binding to hIL-4R (see examples below).

A "CDR" or complementary determining region is a region of hypervariability interspersed within regions that are more conserved, termed "framework regions" (FR). In different embodiments of the anti-hIL-4R antibody or fragment of the invention, the FRs may be identical to the human germline sequences, or may be naturally or artificially modified.

The term "surface plasmon resonance", as used herein, refers to an optical phenomenon that allows for the analysis of real-time interactions by detection of alterations in protein concentrations within a biosensor matrix, for example using the BIAcore™ system (Pharmacia Biosensor AB).

The term "epitope" is an antigenic determinant that interacts with a specific antigen binding site in the variable region of an antibody molecule known as a paratope. A single antigen may have more than one epitope. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sufonyl groups on the antigen.

The term "substantial identity" or "substantially identical," when referring to a nucleic acid or fragment thereof, indicates that, when optimally aligned with appropriate nucleotide insertions or deletions with another nucleic acid (or its complementary strand), there is nucleotide sequence identity in at least about 95%, and more preferably at least about 96%, 97%, 98% or 99% of the nucleotide bases, as measured by any well-known algorithm of sequence identity, such as FASTA, BLAST or Gap, as discussed below.

As applied to polypeptides, the term "substantial similarity" or "substantially similar" means that two peptide sequences, when optimally aligned, such as by the programs GAP or BESTFIT using default gap weights, share at least 95% sequence identity, even more preferably at least 98% or 99% sequence identity. Preferably, residue positions which are not identical differ by conservative amino acid substitutions. A "conservative amino acid substitution" is one in which an amino acid residue is substituted by another amino acid residue having a side chain (R group) with similar chemical properties (e.g., charge or hydrophobicity). In general, a conservative amino acid substitution will not substantially change the functional properties of a protein. In cases where two or more amino acid sequences differ from each other by conservative substitutions, the percent sequence identity or degree of similarity may be adjusted upwards to correct for the conservative nature of the substitution. Means for making this adjustment are well-known to those of skill in the art. See, e.g., Pearson (1994) Methods Mol. Biol. 24: 307-331. Examples of groups of amino acids that have side chains with similar chemical properties include (1) aliphatic side chains: glycine, alanine, valine, leucine and isoleucine; (2) aliphatic-hydroxyl side chains: serine and threonine; (3) amide-containing side chains: asparagine and glutamine; (4) aromatic side chains: phenylalanine, tyrosine, and tryptophan; (5) basic side chains: lysine, arginine, and histidine; (6) acidic side chains: aspartate and glutamate, and (7) sulfur-containing side chains are cysteine and methionine. Preferred conservative amino acids substitution groups are: valine-leucine-isoleucine, phenylalanine-tyrosine, lysine-arginine, alanine-valine, glutamate-aspartate, and asparagine-glutamine. Alternatively, a conservative replacement is any change having a positive value in the PAM250 log-likelihood matrix disclosed in Gonnet et al. (1992) Science 256: 1443 45. A "moderately conservative" replacement is any change having a nonnegative value in the PAM250 log-likelihood matrix.

Sequence similarity for polypeptides, which is also referred to as sequence identity, is typically measured using sequence analysis software. Protein analysis software matches similar sequences using measures of similarity assigned to various substitutions, deletions and other modifications, including conservative amino acid substitutions. For instance, GCG software contains programs such as Gap and Bestfit which can be used with default parameters to determine sequence homology or sequence identity between closely related polypeptides, such as homologous polypeptides from different species of organisms or between a wild type protein and a mutein thereof. See, e.g., GCG Version 6.1. Polypeptide sequences also can be compared using FASTA using default or recommended parameters, a program in GCG Version 6.1. FASTA (e.g., FASTA2 and FASTA3) provides alignments and percent sequence identity of the regions of the best overlap between the query and search sequences (Pearson (2000) *supra*). Another preferred algorithm when comparing a sequence of the invention to a database containing a large number of sequences from different organisms is the computer program BLAST, especially BLASTP or TBLASTN, using default parameters. See, e.g., Altschul et al. (1990) J. Mol. Biol. 215:403-410 and Altschul et al. (1997) Nucleic Acids Res. 25:3389-402.

### Preparation of Human Antibodies

Methods for generating human antibodies include, for example, Veloclmmune™ (Regeneron Pharmaceuticals), XenoMouse™ technology (Green et al. (1994) Nature Genetics 7:13-21; Abgenix), the "minilocus" approach, and phage display (and see, for example, US 5,545,807, US 6,787,637). The VelocImmune™ technology (US 6, 596,541) encompasses a method of generating a high specificity fully human antibody to a select antigen.

Rodents can be immunized by any method known in the art (see, for example, Harlow and Lane (1988) *supra*; Malik and Lillehoj (1994) Antibody techniques, Academic Press, CA). In a preferred embodiment, hIL-4R antigen is administered directly to mice that comprise DNA loci encoding both a human Ig heavy chain variable region and a Kappa light chain variable region (VelocImmune™, Regeneron Pharmaceuticals, Inc.; US 6,596,541), with an adjuvant to stimulate the immune response, for example, complete and incomplete Freund's adjuvant, MPL+TDM adjuvant system (Sigma), or RIBI (muramyl dipeptides) (see O'Hagan (2000) Vaccine Adjuvant, Human Press, NJ). An adjuvant can prevent rapid dispersal of polypeptide by sequestering the antigen in a local depot, and may contain factors that can stimulate host immune response. VelocImmune™ technology involves generating a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody. In a specific embodiment, the cell is a CHO cell.

Antibodies may be therapeutically useful in blocking a ligand-receptor interaction or inhibiting receptor component interaction, rather than by killing cells through fixation of complement (complement-dependent cytotoxicity) (CDC) and participation antibody-dependent cell-mediated cytotoxicity (ADCC). The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

Human immunoglobulins can exist in two forms that are associated with hinge heterogeneity. In one form, an immunoglobulin molecule comprises a stable four-chain construct of approximately 150-160 kDa in which the dimers are held together by an interchain heavy chain disulfide bond. In a second form, the dimers are not linked via interchain disulfide bonds and a molecule of about 75-80 kDa is formed composed of a covalently coupled light and heavy chain (half-antibody). These forms have been extremely difficult to separate, even after affinity purification. The frequency of appearance of the second form in various intact IgG isotypes is due to, but not limited to, structural differences associated with the hinge region isotype of the antibody. In fact, a single amino acid substitution in the hinge region of the human IgG4 hinge can significantly reduce the appearance of the second form (Angal et al. (1993) Molecular Immunology 30: 105) to levels typically observed using a human IgG1 hinge. The instant invention encompasses antibodies having one or more mutations in the hinge, CH2 or CH3 region that may be desirable, for example, in production, to improve the yield of the desired antibody form.

Antibodies of the invention are preferably prepared with the use of VelocImmune™ technology. A transgenic mouse in which the endogenous immunoglobulin heavy and light chain variable regions are replaced with the corresponding human variable regions is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable regions of the light and heavy chains may be isolated directly from antigen-specific lymphocytes.

In one instance, the transgenic mouse comprises up to 18 functional human variable heavy chain genes and 12 functional human variable kappa light chain genes. In another instance, the transgenic mouse comprises up to 39 human variable heavy chain genes and 30 human variable kappa light chain genes. In yet another embodiment, the transgenic mouse comprises up to 80 human variable heavy chain genes and 40 human variable kappa light chain genes.

In general, the antibodies of the instant invention possess very high affinities, typically possessing K_{D}s of from about 10⁻⁹ through about 10⁻¹² M, when measured by binding to antigen either immobilized on a solid phase or measured in solution.

Initially, high affinity chimeric antibodies are isolated having a human variable region and a mouse constant region. As described below, the antibodies are characterized and selected for desirable characteristics, including binding affinity to hIL-4R, ability to block hIL-4 binding to hIL-4R, and/or selectivity for the human protein. The mouse constant regions are replaced with desired human constant regions to generate the fully human antibodies of the invention, for example wild-type or modified IgG4 or igG1 (for example, SEQ ID NO:588, 589, 590). While the constant region selected may vary according to specific use, high affinity antigen-binding and target specificity characteristics reside in the variable region.

### Epitope Mapping and Related Technologies

To screen for antibodies that bind to a particular epitope, a routine cross-blocking assay such as that described in Antibodies: A Laboratory Manual 1988 Cold Spring Harbor Laboratory, Harlow and Lane, eds. can be performed. Other methods include alanine scanning mutants, peptide blots (Reineke (2004) Methods Mol Biol 248:443-63), or peptide cleavage analysis. In addition, methods such as epitope excision, epitope extraction and chemical modification of antigens can be employed (Tomer (2000) Protein Science: 9:487-496).

Modification-Assisted, Profiling (MAP), also known as Antigen Structure-based Antibody Profiling (ASAP) is a method that categorizes large numbers of monoclonal antibodies (mAbs) directed against the same antigen according to the similarities of the binding profile of each antibody to chemically or enzymatically modified antigen surfaces (US Patent Application Publication No. 2004/0101920). Each category may reflect a unique epitope either distinctly different from, or partially overlapping with, an epitope represented by another category. This technology allows rapid filtering of genetically identical antibodies, such that characterization can be focused on genetically distinct antibodies. When applied to hybridoma screening, MAP may facilitate identification of rare hybridoma clones with desired characteristics. MAP may be used to sort the hIL-4R antibodies of the invention into groups of antibodies binding different epitopes.

Agents useful for altering the structure of the immobilized antigen are enzymes, such as, for example, proteolytic enzymes and chemical agents. The antigen protein may be immobilized on either biosensor chip surfaces or polystyrene beads. The latter can be processed with, for example, an assay such as a multiplex Luminex™ detection assay (Luminex Corp., TX). Because of the capacity of Luminex™ to handle multiplex analysis with up to 100 different types of beads, Luminex™ provides almost unlimited antigen surfaces with various modifications, resulting in improved resolution in antibody epitope profiling over a biosensor assay.

### Therapeutic Administration and Formulations

Administration of therapeutic entities in accordance with the invention can be achieved with suitable carriers, excipients, and other agents that are incorporated into formulations to provide improved transfer, delivery, tolerance, and the like. A multitude of appropriate formulations can be found in the formulary known to all pharmaceutical chemists: Remington: The Science and Practice of Pharmacy (2003, 20th ed, Lippincott Williams & Wilkins). These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing mixtures may be appropriate in treatments and therapies in accordance with the present invention, provided that the active ingredient in the formulation is not inactivated by the formulation and the formulation is physiologically compatible and tolerable with the route of administration. See also Powell et al. "Compendium of excipients for parenteral formulations" PDA (1998) J Pharm Sci Technol. 52:238-311 and the citations therein for additional information related to excipients and carriers well known to pharmaceutical chemists.

The therapeutic molecules of the invention may be administered to a patient in a manner appropriate to the indication, for example, parenterally, topically, or by inhalation. If injected, the antagonist can be administered, for example, via intra-articular, intravenous, intramuscular, intralesional, intraperitoneal or subcutaneous routes, by bolus injection, or continuous infusion. Localized administration at a site of disease or injury is contemplated, as are transdermal delivery and sustained release from implants. Delivery by inhalation includes, for example, nasal or oral inhalation, use of a nebulizer, inhalation of the antagonist in aerosol form, and the like. Other alternatives include eyedrops; oral preparations including pills, syrups, lozenges or chewing gum; and topical preparations such as lotions, gels, sprays, and ointments.

Specific dosages and the frequency of administration may vary according to such factors as the route of administration, the nature and severity of the disease to be treated, whether the condition is acute or chronic, and the size and general condition of the patient. Appropriate dosages can be determined by procedures known in the pertinent art, e.g. in clinical trials that may involve dose escalation studies. The therapeutic molecules of the invention may be once, or repeatedly. In particular embodiments, the antibody or antibody fragment is administered over a period of at least a month or more, e.g., for one, two, or three months or even indefinitely. For treating chronic conditions, long-term treatment is generally most effective. However, for treating acute conditions, administration for shorter periods, e.g. from one to six weeks, may be sufficient. In general, the therapeutic is administered until the patient manifests a medically relevant degree of improvement over baseline for the chosen indicator or indicators. The level of IL-4 may be monitored during and/or after treatment with the therapeutic molecule of the invention. Methods for measuring IL-4 serum levels are known in the art, for example by ELISA, etc.

### Therapeutic Use and Combination Therapies

The antibodies and antibody fragments of the invention are useful for treating diseases and disorders which are improved, inhibited or ameliorated by reducing IL-4 activity. These disorders include those characterized by abnormal or excess expression of IL-4, or by an abnormal host response to IL-4 production. IL-4 related disorders which are treated by the antibodies or antibody fragments of the include, for example, arthritis (including septic arthritis), herpetiformis, chronic idiopathic urticaria, scleroderma, hypertrophic scarring, Whipple's Disease, benign prostate hyperplasia, pulmonary disorders such as asthma (mild, moderate or severe), inflammatory disorders such as inflammatory bowel disease, allergic reactions, Kawasaki disease, sickle cell disease, Churg-Strauss syndrome, Grave's disease, pre-eclampsia, Sjogren's syndrome, autoimmune lymphoproliferative syndrome, autoimmune hemolytic anemia, Barrett's esophagus, autoimmune uveitis, tuberculosis, atopic dermatatis, ulcerative colitis, fibrosis, and nephrosis (see U.S. 7,186,809).

The invention encompasses combination therapies in which the anti-IL-4R antibody or antibody fragment is administered in combination with a second therapeutic agent. Coadministration and combination therapy are not limited to simultaneous administration, but include treatment regimens in which an anti-IL-4R antibody or antibody fragment is administered at least once during a course of treatment that involves administering at least one other therapeutic agent to the patient. A second therapeutic agent may be another IL-4 antagonist, such as another antibody/antibody fragment, or a soluble cytokine receptor, an IgE antagonist, an anti-asthma medication (corticosteroids, non-steroidal agents, beta agonists, leukotriene antagonists, xanthines, fluticasone, salmeterol, albuterol) which may be delivered by inhalation or other appropriate means. In a specific embodiment, the anti-IL-4R antibody or antibody fragment of the invention may be administered with an IL-1 antagonist, such as rilonacept, or an IL-13 antagonist. The second agent may include one or more leukotriene receptor antagonists to treat disorders such as allergic inflammatory diseases, e.g., asthma and allergies. Examples of leukotriene receptor antagonists include but are not limited to montelukast, pranlukast, and zafirlukast. The second agent may include a cytokine inhibitor such as one or more of a TNF (etanercept, ENBREL™), IL-9, IL-5 or IL-17 antagonist.

### EXAMPLES

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human IL-4 Receptor.

Mice comprising DNA loci encoding both a human Ig heavy chain variable region and a kappa light chain variable region (VelocImmune™, Regeneron Pharmaceuticals, Inc.; US Patent No. 6,596,541) were immunized with human IL-4R (hIL-4R, SEQ ID NO:1). The hIL-4R was administered by direct injection of purified antigen in an adjuvant or indirectly by providing the DNA sequence of hIL-4R in a DNA plasmid that contains the hIL-4R gene and expresses hIL-4R using the host's cellular protein expression machinery to produce antigen polypeptide *in vivo.* To obtain optimal immune response, animals were subsequently boosted every 3-4 weeks and bleeds obtained 10 after each boost for assessment of progression of anti-antigen response.

When the mice attained maximum immune response, antibody-expressing B cells were harvested and fused with mouse myeloma cells to form hybridomas. Functionally desirable monoclonal antibodies were selected by screening conditioned media of the hybridomas or transfected cells for specificity, antigen-binding affinity, and potency in blocking hIL-4 binding to hIL-4R (described below).

Selected antibodies that displayed desirable antigen binding affinity, potency, and/or ability to block hIL-4 binding to hIL-4R included (HCVR/LCVR): VX 4E7-9 (SEQ ID NO:3/11; 387/389; 391/393); VX 3F7-6 (SEQ ID NO:19/27; 395/397; 399/401); VAB 16G1-1 (SEQ ID NO:35/43; 403/405; 407/409); VAB 16F3-1 (SEQ ID NO:51/59, 411/413, 415/417, 579/59 and 581/59); VAB 15C8-17 (SEQ ID NO:67/75, 419/421, 423/425); VAB 13A1-6 (SEQ ID NO:83/91, 427/429, 431/433); VAB 11 G8-1 (SEQ I D NO:99/107, 435/437, 439/441); VAB 10G8-19 (SEQ ID NO:115/123, 443/445, 447/449); VAB 10C1-5 (SEQ ID NO:131/139, 451/453, 455/457); VAB 8G10-1 (SEQ ID NO:147/155, 459/461, 463/465); VAB 7B9-3 (SEQ ID NO:163/171, 467/469, 471/473); VAB 6C10-14 (SEQ ID NO:179/187, 475/477, 479/481); VAB 5C5-1 (SEQ ID NO:195/203, 483/485, 487/489); VAB 3B4-10 (SEQ ID NO:211/219, 491/493, 495/497); VAB 4D5-3 (SEQ ID NO:227/235, 499/501, 503/505); VAB 1 H1-2 (SEQ ID NO:243/251, 507/509, 511/513); VAK5H4-4 (SEQ ID NO:259/267, 515/517, 519/521); VAK7G8-5 (SEQ ID NO:275/283, 523/525, 527/529); VAK8G11-13 (SEQ ID NO:291/299, 531/533, 535/537); VAK9C6-11 (SEQ ID NO:307/315, 539/541, 543/545); VAK10G6-7 (SEQ ID NO:323/331, 547/549, 551/553); VAK11D4-1 (SEQ ID NO:339/347, 555/557, 559/561); VAK12B11-9 (SEQ ID NO:355/363, 563/565, 567/569); and VAK10G12-5 (SEQ ID NO:371/379, 571/573, 575/577).

### Example 2. Antigen Binding Affinity Determination.

Binding affinity (K_{D}) of selected antibodies with respect to hIL-4R was determined using a real-time biosensor surface plasmon resonance assay (BIAcore™ 2000). Briefly, antibody was captured on a goat anti-mouse (GAM) IgG polyclonal antibody surface created through direct chemical coupling of the GAM IgG to a BIAcore™ chip to form a captured antibody surface. Various concentrations (ranging from 12.5 nM to 0.625 nM) of monomeric hIL-4R (R&D Systems) or dimeric hIL-4R-hFc were injected over the captured antibody surface. Binding of antigen to antibody, and dissociation of the bound complex, were monitored in real time. Equilibrium dissociation constants (K_{D}) and dissociation rate constants were ascertained by performing kinetic analysis using BIA evaluation software. BIA evaluation software was also used to calculate the half-life of antigen/antibody complex dissociation (T_{1/2}). Results are shown in Table I. Control: a fully human anti-IL-4R antibody (U.S. Patent No. 7,186, 809; SEQ ID NOs:10 and 12). The results of the kinetic analyses revealed that the selected antibodies included high affinity antibodies that were capable of binding to dimeric receptor with K_{D}s under 1 nM. The binding affinity of anti-hIL-4R antibodies to either mouse or monkey (*Macaca fascicularis*) IL-4R was also tested. The selected antibodies (1) did not cross-react with mouse IL-4R; and (2) either failed to bind monkey IL-4R or bound monkey IL-4R with very low affinity.

**Table 1**

| **Antibody** | **K_{D} dimeric (pM)** | **T_{1/2} dimeric (hr)** | **K_{D} Monomeric (nM)** | **T_{1/2} monomeric (min)** |
|---|---|---|---|---|
| VX 4E7-9 | 111 | 19.1 | 1.43 | 48 |
| VX 3F7-6 | 654 | 2.7 | 3.91 | 16 |
| VAB 8G10-1 | 5,510 | 4.1 | 7.71 | 28 |
| VAB 7B9-3 | 52 | 6.1 | 1.01 | 13 |
| VAB 6C10-14 | 286 | 6.2 | 1.77 | 73 |
| VAB 5C5-11 | 27 | 7.7 | 0.13 | 47 |
| VAB 4D5-3 | 32 | 20.4 | 2.02 | 35 |
| VAB 3B4-10 | 53 | 20.4 | 0.59 | 102 |
| VAB 1H1-2 | 139 | 8.2 | 34.90 | 8 |
| VAB 16G1-1 | 38 | 7.3 | 0.93 | 15 |
| VAB 16F3-1 | 3 | 41 | 0.02 | 254 |
| VAB 15C8-17 | 45 | 3.6 | 0.82 | 15 |
| VAB 11G8-1 | 108 | 8.7 | 1.63 | 25 |
| VAB 10G8-19 | 52 | 2 | 11.40 | 2 |
| VAB 10C1-5 | 51 | 6.7 | 4.47 | 6 |
| VAK8G11-13 | 2 | 63 | 0.03 | 840 |
| VAK5H4-4 | 4 | 30 | 0.02 | 960 |
| VAK10G6-7 | 11 | 24 | 0.23 | 144 |
| VAK9C6-11 | 68 | 7 | 2.05 | 28 |
| VAK7G8-5 | 9 | 25 | 0.06 | 480 |
| VAK11D4-1 | 6 | 21 | 0.05 | 480 |
| VAK12B11-9 | 36 | 12 | 0.41 | 120 |
| VAK10G12-5 | 7 | 46 | 0.09 | 360 |
| Control | 71 | 2.1 | 1.23 | 16 |

Antibody-antigen binding affinity was also assessed using an ELISA-based solution competition assay. Briefly, antibodies (purified proteins at 1 or 3.3 ng/ml) were premixed with serial dilutions of antigen protein (monomeric or dimeric) ranging from 0 to 10 µg/ml. Solutions of the antibody and antigen mixture were then incubated for two to four hours at room temperature to reach binding equilibrium. Free antibody in the mixtures was then measured using a quantitative sandwich ELISA. Briefly, 96-well Maxisorp™ plates (VWR, West Chester, PA) were coated with 2 µg/ml hIL-4R-hFc protein in PBS overnight at 4° C followed by blocking nonspecific binding with BSA. The antibody-antigen mixture solutions were then transferred to the coated Maxisorb™ plates followed by a one-hour incubation. The plates were then washed with washing buffer and plate-bound antibodies were detected with an HRP-conjugated goat anti-mouse IgG polyclonal antibody reagent (Jackson ImmunoResearch) or an HRP-conjugated goat anti-human IgG polyclonal antibody reagent (Jackson ImmunoResearch) for control antibody and developed using colorimetric substrates such as BD OptEIA™ (BD Biosciences Pharmingen, San Diego, CA). After the reaction was stopped with 1 M phosphoric or sulfuric acid, absorbances at 450 nm were recorded and the data were analyzed using GraphPad™ Prism software. The dependency of the signals on the concentrations of antigen in solution was analyzed with a four parameter fit analysis and reported as IC₅₀, the antigen concentration required to achieve 50% reduction of the signal from the antibody samples without the presence of antigen in solution. The IC₅₀s were determined as described above and are summarized in Table 2. In a preferred embodiment, the antibody or antibody fragment of the invention exhibits an IC₅₀ for dimeric hIL-4R of about 20 pM or less, and an IC₅₀ for monomeric hIL-4R of about 150 pM or less, or about 100 pM or less, as measured by ELISA solution competition assay.

**Table 2**

| **Antibody** | **IC₅₀ Dimeric hIL4R (pM)** | **IC₅₀ Monomeric hIL4R (pM)** |
|---|---|---|
| VX 4E7-9 | 94 | 175 |
| VX 3F7-6 | 184 | 2,900 |
| VAB 8G10-1 | 85 | 517 |
| VAB 7B9-3 | 7 | 2,980 |
| VAB 6C10-14 | 203 | 489 |
| VAB 5C5-11 | 6 | 5,220 |
| VAB 4D5-3 | 35 | 285 |
| VAB 3B4-10 | 88 | 336 |
| VAB 1H1-2 | 70 | 779 |
| VAB 16G1-1 | 9 | 595 |
| VAB 16F3-1 | 5 | 7 |
| VAB 15C8-17 | 9 | 418 |
| VAB 11G8-1 | 76 | 473 |
| VAB 10G8-19 | 4 | 89 |
| VAB 10C1-5 | 12 | 12,900 |
| VAK 5H4-4 | 18 | 130 |
| VAK 7G8-5 | 18 | 92 |
| VAK 8G11-13 | 15 | 103 |
| VAK 9C6-11 | 46 | 1,400 |
| VAK 10G6-7 | 25 | 165 |
| VAK 11D4-1 | 23 | 109 |
| VAK 12B11-9 | 64 | 325 |
| VAK 10G12-5 | 41 | 169 |
| Control | 14 | 3,500 |

### Example 3. Inhibition of hIL-4 and hIL-4R Interaction

The ability of the selected antibodies to block hIL-4 binding to hIL-4R were screened by BIAcore™ hIL-4 blocking assay and potency was measured by a quantitative hIL-4 blocking immunoassay as described below. Results are summarized in Table 3.

The ability of the antibodies to block hIL-4 binding to the hIL-4R receptor was determined using surface plasmon resonance. Purified hIL-4R-hFc molecules were captured by goat anti-human IgG polyclonal antibody immobilized on CM-5 to a density of 260 RU, to prepare a receptor-coated surface. Human IL-4 (0.25 ml at 50 nM) was then injected over the receptor-coated surface and the amount of bound hIL-4 was recorded (first injection of hIL-4). The bound hIL-4 was then removed with a pulse of 3 M MgCl₂ followed by conditioning buffer. Purified anti-hIL4R antibodies were then injected over the receptor surface followed by a second injection of hIL-4 at the same concentration (second injection of hIL-4). The percent reduction in hIL-4 binding resulting from preformed antibody and receptor complex is reported in Table 3.

To further evaluate ability to block hIL-4 binding to hIL-4R, a quantitative immunoassay was performed. Briefly, solutions of 25 pM hIL-4R-Fc were premixed with antibody protein ranging from ∼ 50 nM to 0 nM in serial dilutions, followed by a one hour incubation at room temperature. Concentration of free hIL-4R-Fc (no antibody bound) was determined using a hIL-4R-specific sandwich ELISA. Detection plates for hIL-4R were prepared by binding biotinylated hIL-4 (0.5 µg/ml) on streptavidin-coated 96-well plates. Pre-bound antibody-antigen samples were transferred to the hIL-4-coated detection plate. After a one-hour incubation at room temperature, the detection plate was washed and plate-bound hIL-4R-hFc was detected using HRP conjugated goat anti-hFc polyclonal antibodies and developed using colorimetric substrates such as BD OptEIA^{™} (BD Biosciences Pharmingen, San Diego, CA). After the reaction was stopped with 1 M phosphoric or sulfuric acid, absorbances at 450 nm were recorded and the data were analyzed using GraphPad™ Prism software. IC₅₀s were determined as the amount of antibody required to reduce 50% of IL-4R-hFc detectable to plate bound hIL-4. In a preferred embodiment, the antibody or antibody fragment of the invention exhibits an IC₅₀ for blocking 25 pM hIL-4R of less than about 50 pM, or less than about 40 pM, or less than about 30 pM, or less than about 20 pM, as measured by ELISA.

**Table 3**

| **Antibody** | **% Inhibition (BIAcore™)** | **IC₅₀ (ELISA pM)** |
|---|---|---|
| VX 4E7-9 | 79 | 118 |
| VX 3F7-6 | 86 | 274 |
| VAB 8G10-1 | 74 | 244 |
| VAB 7B9-3 | 96 | 59 |
| VAB 6C10-14 | 79 | 441 |
| VAB 5C5-11 | 96 | 24 |
| VAB 4D5-3 | 82 | 240 |
| VAB 3B4-10 | 72 | 322 |
| VAB 1H1-2 | 78 | 146 |
| VAB 16G1-1 | 92 | 18 |
| VAB 16F3-1 | 97 | 19 |
| VAB 15C8-17 | 97 | 29 |
| VAB 11G8-1 | 77 | 240 |
| VAB 10G8-19 | 85 | 18 |
| VAB 10C1-5 | 93 | 34 |
| VAK 5H4-4 | 96 | 33 |
| VAK 7G8-5 | 95 | 27 |
| VAK 8G11-13 | 95 | 26 |
| VAK 9C6-11 | 96 | 67 |
| VAK 10G6-7 | 95 | 37 |
| VAK 11D4-1 | 95 | 35 |
| VAK 12B11-9 | 96 | 99 |
| VAK 10G12-5 | 94 | 59 |

### Example 4. Neutralization of Biological Effect of hIL-4 In Vitro

The IL-4-mediated signal transduction pathway has been documented extensively in the literature (for example, see review of Hebenstreit et al. 2006 Cytokine Growth Factor Rev.17(3):173-88, 2006). IL-4 can stimulate two receptor complexes, type I and type II. Type I receptor complexes are formed by the binding of IL-4 to IL-4R and the subsequent heterodimerization with the common gamma chain. Alternatively, IL4/IL4R complex can heterodimerize with the IL-13 receptor 1 to form type II receptor complexes. Both type I and type II complexes signal mainly through STAT6. Therefore, the ability of the selected antibodies to block signaling through STAT6 was assessed as described below.

A cell line with high sensitivity to hIL-4 and hIL-13 was established. HEK293 cells were stably transfected with human STAT6 and a STAT6 luciferase reporter plasmid, and was maintained in growth media (DMEM, 10% FBS, L-glutamine, penicillin, streptomycin). A robust IL-4 receptor-mediated response was achieved when 10 pM hIL-4 was added to the growth medium of the STAT6-transfected HEK293 cells. For bioassay of the hIL-4 response, cells were washed once in assay media (Optimem I (Gibco) plus 0.1 % FBS) and plated at 1 x 10⁴ cells/well (96 well plate) in 80 µl of assay media. Purified antibodies were serially diluted into assay media (final concentrations ranging from 20 nM to 0) and 10 µl of each of the test antibody was added to the cells along with 10 µl of hIL-4 (10 pM constant final concentration). Cells were then incubated at 37°C, 5% CO₂ for 6 hrs. The extent of cellular response was measured in a luciferase assay (Promega Biotech). Results are shown in Table 4.

Inhibition of IL-4-dependent biological activity *in vitro* was also confirmed using either a human erythroblast cell line, TF1, or a modified, II-13Rα overexpressing TF1 cell line (TF1/A12). In this bioassay, 20,000 cells were seeded in each well of a 96-well plate in RPMI 1640 medium containing 10% FBS, 2mM L-glutamine, and penicillin and streptomycin. Twenty-five µl of purified antibodies ranging from 0 to 50 nM (final concentration) were added together with 25 □I of hIL-4 recombinant protein to a final concentration of either 50 pM for cell line TF1 or 20 pM for cell line TF1/A12. Cells were then allowed to grow for 3 days at 37°C and final cell counts were measured using a CCK8 kit (Dojindo, Japan). Ability of antibody to block TF-1 cell growth is reported in Table 4 as the concentration of antibody required to achieve 50% reduction of cell proliferation.

**Table 4**

| **Antibody** | **STAT6 Bioassay IC₅₀ (nM)** | **TF1 Bioassay IC₅₀ (nM)** | **TF1/A12 Bioassay IC₅₀ (nM)** |
|---|---|---|---|
| VX 4E7-9 | 7.90 | | 10 |
| VX 3F7-6 | >10 | | >50 |
| VAB 8G10-1 | >10 | | >50 |
| VAB 7B9-3 | 3.40 | | 8.20 |
| VAB 6C10-14 | >10 | | 23 |
| VAB 5C5-11 | 0.14 | 0.21 | 0.37 |
| VAB 4D5-3 | 3.80 | | 21 |
| VAB 3B4-10 | >10 | | >50 |
| VAB 1H1-2 | >10 | | >50 |
| VAB 16G1-1 | 0.14 | 0.12 | 0.70 |
| VAB 16F3-1 | 0.032 | 0.046 | 0.067 |
| VAB 15C8-17 | 0.32 | 0.44 | 4.70 |
| VAB 11G8-1 | >10 | | >50 |
| VAB 10G8-19 | 1.80 | | 12.8 |
| VAB 10C1-5 | 1.90 | | 22.8 |
| VAK 5H4-4 | 0.132 | 0.150 | 0.182 |
| VAK 7G8-5 | 0.06 | 0.13 | |
| VAK 8G11-13 | 0.08 | 0.16 | |
| VAK 9C6-11 | 0.48 | 0.31 | |
| VAK 10G6-7 | 0.11 | 0.23 | |
| VAK 11D4-1 | 0.20 | 0.20 | |
| VAK 12B11-9 | 0.49 | 0.57 | |
| VAK 10G12-5 | 0.36 | 0.58 | |

### Example 5. Neutralization of Biological Effect of hIL-13 In Vitro

Since IL-4R has been shown to be a modulator for IL-13 activity through its binding to IL-13/IL-13R complex, the selected antibodies were tested for their ability to block IL-13 activity in a modification of the HEK293 STAT6 luciferase assay described above, with the modification being replacement of 10 pM IL-4 with 40 pM hIL-13. The antibodies were also assessed for potency in blocking hIL-13 activity in the TF-1 cell line assay described above, with hIL-13 at 150 pM in the presence of 0-50 nM antibodies. Results are shown in Table 5.

**Table 5**

| **Antibody** | **STAT6 Bioassay IC₅₀ (nM)** | **TF1 Bioassay IC₅₀ (nM)** |
|---|---|---|
| VX 4E7-9 | 6.30 | |
| VX 3F7-6 | >10 | |
| VAB 8G10-1 | >10 | |
| VAB 7B9-3 | 1.10 | |
| VAB 6C10-14 | >10 | |
| VAB 5C5-11 | 0.06 | 0.82 |
| VAB 4D5-3 | 3.30 | |
| VAB 3B4-10 | 8.30 | |
| VAB 1H1-2 | >10 | |
| VAB 16G1-1 | 0.09 | 0.44 |
| VAB 16F3-1 | 0.018 | 0.079 |
| VAB 15C8-17 | 0.20 | 2.60 |
| VAB 11G8-1 | 8.80 | |
| VAB 10G8-19 | 0.97 | |
| VAB 10C1-5 | 1.00 | |
| VAK 5H4-4 | 0.084 | 0.240 |
| VAK 7G8-5 | 0.04 | 0.36 |
| VAK 8G11-13 | 0.06 | 0.42 |
| VAK 9C6-11 | 0.30 | 1.00 |
| VAK 10G6-7 | 0.07 | 0.46 |
| VAK 11D4-1 | 0.14 | 0.44 |
| VAK 12B11-9 | 0.37 | 1.60 |
| VAK 10G12-5 | 0.26 | 1.90 |

### Example 6. Antibody Binding Profile Assessment

An antibody binding profile can be established by determining the effect that an antibody (bound to its antigen) can have on the ability of a panel of different antibodies to subsequent bind the same antigen. For example, the antigen can be immobilized on a support to form an antigen-coated surface, the antigen-coated surface can be saturated with the antibody, and then the antibody-saturated antigen-coated surface can be exposed to a panel of other antibodies. The extent of binding of the panel of other antibodies to the antibody-saturated antigen surface provides an antibody binding profile. OCTET^{™}-based sequential binding assays were employed to generate an antibody binding profile. Briefly, a group of 24 strepavidin High Binding FA Biosensors (ForteBio, Inc., Menlo Park, CA) were first incubated with antigen, biotin-hIL-4R-hFc, at 2 µg/ml for 10 min at 30°C to achieve saturation and the amount of bound antigen was measured as a change in thickness (nm) of the biological layer due to bound protein, which is directly measured by the wavelength shift. Biotin-hIL-4R-hFc-bound biosensors were then incubated with the first antibody (control antibody) at 50 µg/ml for 15 min at 30°C to achieve saturation and the amount of bound control antibody was measured as a change in thickness (nm) of the biological layer. Each control antibody-bound sensor was then incubated with one of a panel of 24 different anti-hIL-4R antibodies (second antibody) at 50 µg/ml for 15 min at 30°C, and the amount of second antibody bound was measured as a change in thickness of the biological layer. The same assay was repeated using either VAB16F3-1 or VAK5H4-4 anti-hIL-4R antibody as the first antibody. Results are shown in Fig. 1A-C.

Selected anti-hIL-4R antibodies were also assessed by Western blot (data not shown). Briefly, hIL-4R monomer (200 ng per lane) and mfIL-4R His-tagged monomer (200 ng per lane) were electrophoresed on SDS-PAGE gels using both reducing and non-reducing sample buffer. Four separate gels were each transferred to a PVDF membrane and each membrane was exposed to one of four primary antibodies: anti-His mAb (Qiagen), VAB 16F3-1, VAK 5H4-4, or control anti-hIL-4R antibody, with either HRP-conjugated goat anti-mIgG or anti-hIgG (Pierce) as secondary antibody. All three anti-hIL-4R antibodies recognized the non-reduced form of hIL-4R. Only VAB 16F3-1 and VAK 5H4-4 detected the reduced form of hIL-4R. None of the anti-hIL-4R antibodies detected monkey IL-4R.

## Claims

1. An antibody or antigen-binding fragment thereof, that specifically binds hIL-4R (SEQ ID NO: 1) with a K_{D} of about 200 pM or less, as measured by surface plasmon resonance, comprising the CDRs from a HCVR and a LCVR sequence, wherein the HCVR/LCVR sequences are selected from SEQ ID NOs: 579/59 and 581/59.

2. An antibody or antigen-binding fragment of claim 1, that exhibits a K_{D} for hIL-4R (SEQ ID NO: 1) of less than 150pM.

3. An antibody or antigen-binding fragment of claim 1, that exhibits a K_{D} for hIL-4R (SEQ ID NO: 1) of less than 50pM.

4. An isolated nucleic acid molecule encoding an antibody or antigen-binding fragment according to any one of the preceding claims.

5. A vector comprising the nucleic acid sequence according to claim 4.

6. A host-vector system for the production of an antibody or antigen-binding fragment of an antibody which specifically binds IL-4 receptor, comprising a vector according to claim 5, in a suitable host cell.

7. A host-vector system according to claim 6, wherein the host cell is a prokaryotic or eukaryotic cell selected from one of *E. coli* or a CHO cell.

8. A method of producing an anti-IL-4R antibody or antigen-binding fragment thereof, comprising growing cells of a host-vector system according to claim 6 or 7 under conditions permissing production of the antibody or fragment thereof and recovering the antibody or fragment so expressed.

9. Use of an antibody or antigen-binding fragment of an antibody according to any one of claims 1 to 3 in the manufacture of a medicament for use to attenuate or inhibit an IL-4-mediated disease or disorder in a human.

10. An antibody or antigen-binding fragment according to any one of claims 1 to 3, for use in a method of treating a disease or disorder in a human, wherein the disease or disorder is improved, ameliorated or inhibited by removal, inhibition or reduction of human interleukin-4 (hIL-4) activity.

11. Use according to claim 9, wherein the disease or disorder is selected from arthritis, herpetiformis, chronic idiopathic urticaria, scleroderma, hypertrophic scarring, Whipple's Disease, benign prostate hyperplasia, lung disorders, inflammatory disorders, allergic reactions, Kawasaki disease, sickle cell disease, Churg-Strauss syndrome, Grave's disease, pre-eclampsia, Sjogren's syndrome, autoimmune lymphoproliferative syndrome, autoimmune hemolytic anemia, Barrett's esophagus, autoimmune uveitis, tuberculosis, atopic dermatitis, ulcerative colitis, fibrosis and nephrosis.

12. Use according to claim 11, wherein said lung disorder is asthma, said inflammatory disorder is inflammatory bowel disease, or said arthritis is septic arthritis.

13. An antibody or antigen-binding fragment according to claim 10 for use according to claim 10, wherein the disease or disorder is selected from arthritis, herpetiformis, chronic idiopathic urticaria, scleroderma, hypertrophic scarring, Whipple's Disease, benign prostate hyperplasia, lung disorders, inflammatory disorders, allergic reactions, Kawasaki disease, sickle cell disease, Churg-Strauss syndrome, Grave's disease, pre-eclampsia, Sjogren's syndrome, autoimmune lymphoproliferative syndrome, autoimmune hemolytic anemia, Barrett's esophagus, autoimmune uveitis, tuberculosis, atopic dermatitis, ulcerative colitis, fibrosis and nephrosis.

14. An antibody or antigen-binding fragment according to claim 13 for use according to claim 13, wherein said lung disorder is asthma, said inflammatory disorder is inflammatory bowel disease, or said arthritis is septic arthritis.

15. A composition comprising an antibody or antigen-binding fragment of any one of claims 1 to 3 and an acceptable carrier.

## Patentansprüche

1. Antikörper oder Antigen-bindendes Fragment davon, der/das hIL-4R (SEQ ID NO: 1) spezifisch mit einer K_{D} von etwa 200 pM oder weniger, wie gemessen durch Oberflächenplasmonresonanz, bindet, umfassend die CDRs aus einer HCVR- und einer LCVR-Sequenz, wobei die HCVR/LCVR-Sequenzen aus SEQ ID NOs: 579/59 und 581/59 ausgewählt sind.

2. Antikörper oder Antigen-bindendes Fragment nach Anspruch 1, der/das eine K_{D} für hIL-4R (SEQ ID NO: 1) von weniger als 150 pM aufweist.

3. Antikörper oder Antigen-bindendes Fragment nach Anspruch 1, der/das eine K_{D} für hIL-4R (SEQ ID NO: 1) von weniger als 50 pM aufweist.

4. Isoliertes Nukleinsäuremolekül, das einen Antikörper oder ein Antigenbindendes Fragment nach einem der vorangehenden Ansprüche codiert.

5. Vektor, umfassend die Nukleinsäuresequenz nach Anspruch 4.

6. Wirt-Vektor-System für die Herstellung eines Antikörpers oder eines Antigenbindenden Fragments eines Antikörpers, der/das spezifisch IL-4-Rezeptor bindet, umfassend einen Vektor nach Anspruch 5 in einer geeigneten Wirtszelle.

7. Wirt-Vektor-System nach Anspruch 6, wobei die Wirtszelle eine prokaryotische oder eukaryotische Zelle, ausgewählt aus einer von *E. coli* oder einer CHO-Zelle, ist.

8. Verfahren zur Herstellung eines Anti-IL-4R-Antikörpers oder eines Antigenbindenden Fragments davon, umfassend Wachsenlassen von Zellen eines Wirt-Vektor-Systems nach Anspruch 6 oder 7 unter Bedingungen, die eine Herstellung des Antikörpers oder Fragments davon zulassen, und Gewinnen des Antikörpers oder Fragments, der/das so exprimiert wurde.

9. Verwendung eines Antikörpers oder eines Antigen-bindenden Fragments eines Antikörpers nach einem der Ansprüche 1 bis 3 bei der Herstellung eines Medikaments zur Verwendung, um eine IL-4-vermittelte Krankheit oder Störung bei einem Menschen abzuschwächen oder zu inhibieren.

10. Antikörper oder Antigen-bindendes Fragment nach einem der Ansprüche 1 bis 3 zur Verwendung in einem Verfahren zur Behandlung einer Krankheit oder Störung bei einem Menschen, wobei die Krankheit oder Störung durch Entfernung, Inhibierung oder Verringerung der humanen Interleukin-4 (hIL-4)-Aktivität verbessert, gelindert oder inhibiert wird.

11. Verwendung nach Anspruch 9, wobei die Krankheit oder Störung ausgewählt ist aus Arthritis, Herpetiformis, chronischer idiopathischer Urtikaria, Skleroderm, hypertropher Narbenbildung, Whipple-Krankheit, benigner Prostatahyperplasie, Lungenerkrankungen, Entzündungskrankheiten, allergischen Reaktionen, Kawasaki-Krankheit, Sichelzellenkrankheit, Churg-Strauss-Syndrom, Morbus Basedow, Prä-Eklampsie, Sjogren-Syndrom, autoimmunem lymphoproliferativem Syndrom, autoimmuner hämolytischer Anämie, Barrett-Oesophagus, autoimmuner Uveitis, Tuberkulose, atopischer Dermatitis, Colitis ulcerosa, Fibrose und Nephrose.

12. Verwendung nach Anspruch 11, wobei die Lungenerkrankung Asthma ist, die Entzündungskrankheit entzündliche Darmerkrankung ist oder die Arthritis septische Arthritis ist.

13. Antikörper oder Antigen-bindendes Fragment nach Anspruch 10 zur Verwendung nach Anspruch 10, wobei die Krankheit oder Störung ausgewählt ist aus Arthritis, Herpetiformis, chronischer idiopathischer Urtikaria, Skleroderm, hypertropher Narbenbildung, Whipple-Krankheit, benigner Prostatahyperplasie, Lungenerkrankungen, Entzündungskrankheiten, allergischen Reaktionen, Kawasaki-Krankheit, Sichelzellenkrankheit, Churg-Strauss-Syndrom, Morbus Basedow, Prä-Eklampsie, Sjogren-Syndrom, autoimmunem lymphoproliferativem Syndrom, autoimmuner hämolytischer Anämie, Barrett-Oesophagus, autoimmuner Uveitis, Tuberkulose, atopischer Dermatitis, Colitis ulcerosa, Fibrose und Nephrose.

14. Antikörper oder Antigen-bindendes Fragment nach Anspruch 13 zur Verwendung nach Anspruch 13, wobei die Lungenerkrankung Asthma ist, die Entzündungskrankheit entzündliche Darmerkrankung ist oder die Arthritis septische Arthritis ist.

15. Zusammensetzung, umfassend einen Antikörper oder ein Antigen-bindendes Fragment nach einem der Ansprüche 1 bis 3 und einen annehmbaren Träger.

## Revendications

1. Anticorps, ou fragment d'anticorps se liant à l'antigène, qui se lie spécifiquement au récepteur de l'interleukine 4 humaine (récepteur hIL-4R, Séquence N° 1) avec une constante K_{D}, mesurée par résonance de plasmons de surface, inférieure ou égale à environ 200 pM, et qui comprend les domaines CDR provenant d'une séquence de région variable de chaîne lourde (HCVR) et d'une séquence de région variable de chaîne légère (LCVR), lesquelles séquences HCVR/LCVR sont choisies parmi les Séquences N° 579/59 et N° 581/59.

2. Anticorps ou fragment d'anticorps se liant à l'antigène, conforme à la revendication 1, qui présente pour la liaison au récepteur hIL-4R (Séquence N° 1) une constante K_{D} inférieure à 150 pM.

3. Anticorps ou fragment d'anticorps se liant à l'antigène, conforme à la revendication 1, qui présente pour la liaison au récepteur hIL-4R (Séquence N° 1) une constante K_{D} inférieure à 50 pM.

4. Molécule d'acide nucléique isolée, qui code un anticorps ou un fragment se liant à l'antigène conforme à l'une des revendications précédentes.

5. Vecteur comprenant une séquence d'acide nucléique conforme à la revendication 4.

6. Système hôte-vecteur conçu pour la production d'un anticorps, ou d'un fragment d'anticorps se liant à l'antigène, qui se lie spécifiquement au récepteur hIL-4R, lequel système comprend un vecteur conforme à la revendication 5, dans une cellule hôte appropriée.

7. Système hôte-vecteur conforme à la revendication 6, dans lequel la cellule hôte est une cellule de procaryote ou d'eucaryote, choisie parmi une cellule d'*E*. *coli* et une cellule CHO.

8. Procédé de production d'un anticorps anti-IL-4R, ou d'un fragment se liant à l'antigène d'un tel anticorps, comprenant le fait de faire se multiplier des cellules d'un système hôte-vecteur conforme à la revendication 6 ou 7, dans des conditions permettant la production de l'anticorps ou de son fragment, et le fait de récupérer l'anticorps ou son fragment ainsi exprimé.

9. Utilisation d'un anticorps, ou d'un fragment d'anticorps se liant à l'antigène, conforme à l'une des revendications 1 à 3, dans la fabrication d'un médicament destiné à être utilisé pour atténuer ou inhiber, chez un humain, une maladie ou un trouble à médiation par l'interleukine 4.

10. Anticorps ou fragment d'anticorps se liant à l'antigène, conforme à l'une des revendications 1 à 3, pour utilisation dans un procédé de traitement d'une maladie ou d'un trouble chez un humain, laquelle maladie ou lequel trouble est amélioré(e) ou inhibé(e) par annihilation, inhibition ou réduction de l'activité d'interleukine 4 humaine (hIL-4).

11. Utilisation conforme à la revendication 9, la maladie ou le trouble étant choisi(e) parmi les suivant(e)s : arthrite, dermatite herpétiforme, urticaire idiopathique chronique, sclérodermie, cicatrice hypertrophique, lipodystrophie intestinale, hyperplasie bénigne de la prostate, affections pulmonaires, troubles inflammatoires, réactions allergiques, maladie de Kawasaki, drépanocytose, syndrome de Churg-Strauss, maladie de Basedow-Graves, pré-éclampsie, syndrome de Sjögren, syndrome lymphoprolifératif avec auto-immunité, anémie hémolytique auto-immune, ulcère de Barret, uvéite auto-immune, tuberculose, dermatite atopique, colite ulcéreuse, fibrose, et néphrose.

12. Utilisation conforme à la revendication 11, ladite affection pulmonaire étant un asthme, ledit trouble inflammatoire étant une affection intestinale inflammatoire, ou ladite arthrite étant une arthrite aiguë suppurée.

13. Anticorps ou fragment d'anticorps se liant à l'antigène, conforme à la revendication 10, pour utilisation conforme à la revendication 10, la maladie ou le trouble étant choisi(e) parmi les suivant(e)s : arthrite, dermatite herpétiforme, urticaire idiopathique chronique, sclérodermie, cicatrice hypertrophique, lipodystrophie intestinale, hyperplasie bénigne de la prostate, affections pulmonaires, troubles inflammatoires, réactions allergiques, maladie de Kawasaki, drépanocytose, syndrome de Churg-Strauss, maladie de Basedow-Graves, pré-éclampsie, syndrome de Sjögren, syndrome lymphoprolifératif avec auto-immunité, anémie hémolytique auto-immune, ulcère de Barret, uvéite auto-immune, tuberculose, dermatite atopique, colite ulcéreuse, fibrose, et néphrose.

14. Anticorps ou fragment d'anticorps se liant à l'antigène, conforme à la revendication 13, pour utilisation conforme à la revendication 13, ladite affection pulmonaire étant un asthme, ledit trouble inflammatoire étant une affection intestinale inflammatoire, ou ladite arthrite étant une arthrite aiguë suppurée.

15. Composition comprenant un anticorps ou un fragment d'anticorps se liant à l'antigène, conforme à l'une des revendications 1 à 3, et un véhicule acceptable.


### SEQUENCE LISTING

<110> Regeneron Pharmaceuticals, Inc.
<120> High Affinity Human Antibodies to Human IL-4 Receptor
<130> 6030A-WO
<140> To be assigned
   <141> 2007-10-02
<150> 60/848,694
   <151> 2006-10-02
<150> 60/957,738
   <151> 2007-08-24
<160> 590
<170> FastSEQ for Windows Version 4.0
<210> 1
   <211> 207
   <212> PRT
   <213> homo sapiens
<400> 1
   Met Lys Val Leu Gln Glu Pro Thr Cys Val Ser Asp Tyr Met Ser Ile
    1               5                  10                  15
   Ser Thr Cys Glu Trp Lys Met Asn Gly Pro Thr Asn Cys Ser Thr Glu
               20                  25                  30
   Leu Arg Leu Leu Tyr Gln Leu Val Phe Leu Leu Ser Glu Ala His Thr
           35                  40                  45
   Cys Ile Pro Glu Asn Asn Gly Gly Ala Gly Cys Val Cys His Leu Leu
       50                  55                  60
   Met Asp Asp Val Val Ser Ala Asp Asn Tyr Thr Leu Asp Leu Trp Ala
   65                  70                  75                  80
   Gly Gln Gln Leu Leu Trp Lys Gly Ser Phe Lys Pro Ser Glu His Val
                   85                  90                  95
   Lys Pro Arg Ala Pro Gly Asn Leu Thr Val His Thr Asn Val Ser Asp
               100                 105                 110
   Thr Leu Leu Leu Thr Trp Ser Asn Pro Tyr Pro Pro Asp Asn Tyr Leu
           115                 120                 125
   Tyr Asn His Leu Thr Tyr Ala Val Asn Ile Trp Ser Glu Asn Asp Pro
       130                 135                 140
   Ala Asp Phe Arg Ile Tyr Asn Val Thr Tyr Leu Glu Pro Ser Leu Arg
   145                 150                 155                 160
   Ile Ala Ala Ser Thr Leu Lys Ser Gly Ile Ser Tyr Arg Ala Arg Val
                   165                 170                 175
   Arg Ala Trp Ala Gln Cys Tyr Asn Thr Thr Trp Ser Glu Trp Ser Pro
               180                 185                 190
   Ser Thr Lys Trp His Asn Ser Tyr Arg Glu Pro Phe Glu Gln His
           195                 200                 205
<210> 2
   <211> 351
   <212> DNA
   <213> Artificial Sequence
<220>
   <223> Synthetic
<400> 2
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa aacaaactat 180
   gcacagaagc tccagggcag agtcaccatg accacagaca catccacgac cacagcctac 240
   atggaaatga ggagcctgag atctgacgac acggccgttt attactgtgc gagaggtagt 300
   ggctacgatt tggactactg gggccaggga accctggtct ccgtctcctc a          351
<210> 3
   <211> 117
   <212> PRT
   <213> Artificial Sequence
<220>
   <223> Synthetic
<400> 3
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Lys Thr Asn Tyr Ala Gln Lys Leu
       50                  55                  60
   Gln Gly Arg Val Thr Met Thr Thr Asp Thr Ser Thr Thr Thr Ala Tyr
   65                  70                  75                  80
   Met Glu Met Arg Ser Leu Arg Ser Asp Asp Thr Ala Val Tyr Tyr Cys
                   85                  90                  95
   Ala Arg Gly Ser Gly Tyr Asp Leu Asp Tyr Trp Gly Gln Gly Thr Leu
               100                 105                 110
   Val Ser Val Ser Ser
           115
<210> 4
   <211> 24
   <212> DNA
   <213> Artificial Sequence
<220>
   <223> Synthetic
<400> 4
   ggttacacct ttaccaacta tggt 24
<210> 5
   <211> 8
   <212> PRT
   <213> Artificial Sequence
<220>
   <223> Synthetic
<400> 5
   Gly Tyr Thr Phe Thr Asn Tyr Gly
    1               5
<210> 6
   <211> 24
   <212> DNA
   <213> Artificial Sequence
<220>
   <223> Synthetic
<400> 6
   atcagcgttt acaatggtaa aaca 24
<210> 7
   <211> 8
   <212> PRT
   <213> Artificial Sequence
<220>
   <223> Synthetic
<400> 7
   Ile Ser Val Tyr Asn Gly Lys Thr
   1 5
<210> 8
   <211> 30
   <212> DNA
   <213> Artificial Sequence
<220>
   <223> Synthetic
<400> 8
   gcgagaggta gtggctacga tttggactac 30
<210> 9
   <211> 10
   <212> PRT
   <213> Artificial Sequence
<220>
   <223> Synthetic
<400> 9
   Ala Arg Gly Ser Gly Tyr Asp Leu Asp Tyr
   1 5 10
<210> 10
   <211> 321
   <212> DNA
   <213> Artificial Sequence
<220>
   <223> Synthetic
<400> 10
   gccatccaga tgacccagtc tccatcctcc ctgtctgcat ctgtcggaga cagagtcacc 60
   atcacttgcc gggcaagtca gggcattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccttcagcag cctgcagcct 240
   gaagattttg caacttatta ctgtctacaa gatttcaatt acccgtacac ttttggccag 300
   gggaccaagc tggagatcaa a 321
<210> 11
   <211> 107
   <212> PRT
   <213> Artificial Sequence
<220>
   <223> Synthetic
<400> 11
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Phe Ser Ser Leu Gln Pro
   65                  70                  75                  80
   Glu Asp Phe Ala Thr Tyr Tyr Cys Leu Gln Asp Phe Asn Tyr Pro Tyr
                   85                  90                  95
   Thr Phe Gly Gln Gly Thr Lys Leu Glu Ile Lys
               100                 105
<210> 12
   <211> 18
   <212> DNA
   <213> Artificial Sequence
<220>
   <223> Synthetic
<400> 12
   cagggcatta gaaatgct 18
<210> 13
   <211> 6
   <212> PRT
   <213> Artificial Sequence
<220>
   <223> Synthetic
<400> 13
   Gln Gly Ile Arg Asn Ala
   1               5
<210> 14
   <211> 9
   <212> DNA
   <213> Artificial Sequence
<220>
   <223> Synthetic
<400> 14
   gctgcatcc 9
<210> 15
   <211> 3
   <212> PRT
   <213> Artificial Sequence
<220>
   <223> Synthetic
<400> 15
   Ala Ala Ser
   1
<210> 16
   <211> 27
   <212> DNA
   <213> Artificial Sequence
<220>
   <223> Synthetic
<400> 16
   ctacaagatt tcaattaccc gtacact 27
<210> 17
   <211> 9
   <212> PRT
   <213> Artificial Sequence
<220>
   <223> Synthetic
<400> 17
   Leu Gln Asp Phe Asn Tyr Pro Tyr Thr
   1 5
<210> 18
   <211> 384
   <212>.DNA
   <213> Artificial Sequence
<220>
   <223> Synthetic
<400> 18
   gaggtgcagc tggtggagtc tgggggaggc ttggtccagc ctggggggtc cctaagactc 60
   tcctgtgcag cctctggatt cacctttagt tccttctgga tgacctgggt ccgccaggct 120
   ccagggaagg ggctggagtg ggtggccaac ataaagcaag atggaagtga gaaatactat 180
   gtggactctg tgaagggccg attcaccatt tcccgagaca acgccaagaa ctcactgtat 240
   ctgcaaatga acagcctgag agccgaggac acggctgttt attactgtgc gagagatccc 300
   gggcgaacta tggttcgggg aggtataaga tattactatg gtatggacgt ctggggccaa 360
   gggaccacgg tcaccgtctc ctca 384
<210> 19
   <211> 128
   <212> PRT
   <213> Artificial Sequence
<220>
   <223> Synthetic
<400> 19
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Trp Met Thr Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Pro Gly Arg Thr Met Val Arg Gly Gly Ile Arg Tyr Tyr
               100                 105                 110
   Tyr Gly Met Asp Val Trp Gly Gln Gly Thr Thr Val Thr Val Ser Ser
           115                 120                 125
<210> 20
   <211> 24
   <212> DNA
   <213> Artificial Sequence
<220>
   <223> Synthetic
<400> 20
   ggattcacct ttagttcctt ctgg 24
<210> 21
   <211> 8
   <212> PRT
   <213> Artificial Sequence
<220>
   <223> Synthetic
<400> 21
   Gly Phe Thr Phe Ser Ser Phe Trp
    1               5
<210> 22
   <211> 24
   <212> DNA
   <213> Artificial Sequence
<220>
   <223> Synthetic
<400> 22
   ataaagcaag atggaagtga gaaa 24
<210> 23
   <211> 8
   <212> PRT
   <213> Artificial Sequence
<220>
   <223> Synthetic
<400> 23
   Ile Lys Gln Asp Gly Ser Glu Lys
   1 5
<210> 24
   <211> 63
   <212> DNA
   <213> Artificial Sequence
<220>
   <223> Synthetic
<400> 24
   gcgagagatc ccgggcgaac tatggttcgg ggaggtataa gatattacta tggtatggac 60
   gtc 63
<210> 25
   <211> 21
   <212> PRT
   <213> Artificial Sequence
<220>
   <223> Synthetic
<400> 25
   Ala Arg Asp Pro Gly Arg Thr Met Val Arg Gly Gly Ile Arg Tyr Tyr
   1 5 10 15
   Tyr Gly Met Asp Val
   20
<210> 26
   <211> 321
   <212> DNA
   <213> Artificial Sequence
<220>
   <223> Synthetic
<400> 26
   gacatccaga tgacccagtc tccatcttcc gtgtctgcat ctgtaggaga cagagtcacc 60
   atcagttgtc gggcgagtca gggtgttagt agctggttag cctggtatca gcagaaacca 120
   gggaacgccc ctaagctcct gatctctgct gcatccagta tacaaagtgg ggtcccatca 180
   aggttcagcg gcagtggatc tgggacagat ttcactctca ccatcagcag cctgcagcct 240
   gaagattttg caacttacta ttgtcaacag gctaacagtt tccctctcac tttcggcgga 300
   gggaccaagg tggagatcaa a                                           321
<210> 27
   <211> 107
   <212> PRT
   <213> Artificial Sequence
<220>
   <223> Synthetic
<400> 27
   Asp Ile Gln Met Thr Gln Ser Pro Ser Ser Val Ser Ala Ser Val Gly
    1               5                  10                  15
   Asp Arg Val Thr Ile Ser Cys Arg Ala Ser Gln Gly Val Ser Ser Trp
               20                  25                  30
   Leu Ala Trp Tyr Gln Gln Lys Pro Gly Asn Ala Pro Lys Leu Leu Ile
           35                  40                  45
   Ser Ala Ala Ser Ser Ile Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ser Phe Pro Leu
                   85                  90                  95
   Thr Phe Gly Gly Gly Thr Lys Val Glu Ile Lys
               100                 105
<210> 28
   <211> 18
   <212> DNA
   <213> Artificial Sequence
<220>
   <223> Synthetic
<400> 28
   cagggtgtta gtagctgg 18
<210> 29
   <211> 6
   <212> PRT
   <213> Artificial Sequence
<220>
   <223> Synthetic
<400> 29
   Gln Gly Val Ser Ser Trp
    1               5
<210> 30
   <211> 9
   <212> DNA
   <213> Artificial Sequence
<220>
   <223> Synthetic
<400> 30
   gctgcatcc 9
<210> 31
   <211> 3
   <212> PRT
   <213> Artificial Sequence
<220>
   <223> Synthetic
<400> 31
   Ala Ala Ser
   1
<210> 32
   <211> 27
   <212> DNA
   <213> Artificial Sequence
<220>
   <223> Synthetic
<400> 32
   caacaggcta acagtttccc tctcact 27
<210> 33
   <211> 9
   <212> PRT
   <213> Artificial Sequence
<220>
   <223> Synthetic
<400> 33
   Gln Gln Ala Asn Ser Phe Pro Leu Thr
   1 5
<210> 34
   <211> 366
   <212> DNA
   <213> Artificial Sequence
<220>
   <223> Synthetic
<400> 34
   gaggtgaagt tggcggagtc tgggggaggc ttggtccagc cgggggggtc cctgagactc 60
   tcctgtgcag cctctggatt cacctttagt agccattgga tgaactgggt ccgccaggct 120
   ccagggaagg ggctggagtg ggtggccaac ataaagcaag atggaagtga taaatactat 180
   gtggactctg tgaagggccg attcaccatc tccagagaca acgccaagaa ctcactctat 240
   ctgcaattga acagcctgat agccgaggac acggctgtgt attactgtgc gagagatcgg 300
   ggggttagac cacctcgtgg ggcttttgat atctggggcc aagggacaat ggtcaccgtc 360
   tcttca 366
<210> 35
   <211> 122
   <212> PRT
   <213> Artificial Sequence
<220>
   <223> Synthetic
<400> 35
   Glu Val Lys Leu Ala Glu Ser Gly Gly Gly Leu Val Gln Pro Gly Gly
   1 5 10 15
   Ser Leu Arg Leu Ser Cys Ala Ala Ser Gly Phe Thr Phe Ser Ser His
   20 25 30
   Trp Met Asn Trp Val Arg Gln Ala Pro Gly Lys Gly Leu Glu Trp Val
   35 40 45
   Ala Asn Ile Lys Gln Asp Gly Ser Asp Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Leu Asn Ser Leu Ile Ala Glu Asp Thr Ala Val Tyr Tyr Cys
   85 90 95
   Ala Arg Asp Arg Gly Val Arg Pro Pro Arg Gly Ala Phe Asp Ile Trp
   100 105 110
   Gly Gln Gly Thr Met Val Thr Val Ser Ser
   115 120
<210> 36
   <211> 24
   <212> DNA
   <213> Artificial Sequence
<220>
   <223> Synthetic
<400> 36
   ggattcacct ttagtagcca ttgg 24
<210> 37
   <211> 8
   <212> PRT
   <213> Artificial Sequence
<220>
   <223> Synthetic
<400> 37
   Gly Phe Thr Phe Ser Ser His Trp
   1 5
<210> 38
   <211> 24
   <212> DNA
   <213> Artificial Sequence
<220>
   <223> Synthetic
<400> 38
   ataaagcaag atggaagtga taaa 24
<210> 39
   <211> 8
   <212> PRT
   <213> Artificial Sequence
<220>
   <223> Synthetic
<400> 39
   Ile Lys Gln Asp Gly Ser Asp Lys
   1 5
<210> 40
   <211> 45
   <212> DNA
   <213> Artificial Sequence
<220>
   <223> Synthetic
<400> 40
   gcgagagatc ggggggttag accacctcgt ggggcttttg atatc 45
<210> 41
   <211> 15
   <212> PRT
   <213> Artificial Sequence
<220>
   <223> Synthetic
<400> 41
   Ala Arg Asp Arg Gly Val Arg Pro Pro Arg Gly Ala Phe Asp Ile
   1 5 10 15
<210> 42
   <211> 321
   <212> DNA
   <213> Artificial Sequence
<220>
   <223> Synthetic
<400> 42
   gacatccaga tgacccagtc tccatcttcc gtgtctgcat ctgtaggaga cagagtcacc 60
   atcacttgtc gggcgagtca gggtatcagc agctggttag cctggtatca gcagaaacca 120
   gggaaagccc ctaagctcct gatctatgct gcatccagtt ttcaaagtgg ggtcccatca 180
   aggttcagcg gcagtggatc tgggacagat ttcactctca ccatcagcag cctgcagcct 240
   gaagattttg caacttactt ttgtcaacag gctaacagtt tccctctcac tttcggcgga 300
   gggaccacgg tggagatcaa a                                           321
<210> 43
   <211> 107
   <212> PRT
   <213> Artificial Sequence
<220>
   <223> Synthetic
<400> 43
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Phe Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Phe Cys Gln Gln Ala Asn Ser Phe Pro Leu
                   85                  90                  95
   Thr Phe Gly Gly Gly Thr Thr Val Glu Ile Lys
               100                 105
<210> 44
   <211> 18
   <212> DNA
   <213> Artificial Sequence
<220>
   <223> Synthetic
<400> 44
   cagggtatca gcagctgg 18
<210> 45
   <211> 6
   <212> PRT
   <213> Artificial Sequence
<220>
   <223> Synthetic
<400> 45
   Gln Gly Ile Ser Ser Trp
   1 5
<210> 46
   <211> 9
   <212> DNA
   <213> Artificial Sequence
<220>
   <223> Synthetic
<400> 46
   gctgcatcc 9
<210> 47
   <211> 3
   <212> PRT
   <213> Artificial Sequence
<220>
   <223> Synthetic
<400> 47
   Ala Ala Ser
   1
<210> 48
   <211> 27
   <212> DNA
   <213> Artificial Sequence
<220>
   <223> Synthetic
<400> 48
   caacaggcta acagtttccc tctcact 27
<210> 49
   <211> 9
   <212> PRT
   <213> Artificial Sequence
<220>
   <223> Synthetic
<400> 49
   Gln Gln Ala Asn Ser Phe Pro Leu Thr
   1 5
<210> 50
   <211> 384
   <212> DNA
   <213> Artificial Sequence
<220>
   <223> Synthetic
<400> 50
   caggttcagc tggtgcagtc tggagctgag gtgaagaagc ctggggcctc agtgaaggtc 60
   tcctgcaagg cttctggtta cacctttaat agttatggaa tcagctgggt gcgacaggcc 120
   cctggacaag ggcttgagtg gatgggatgg atcagaactt acaatggtaa cacaaactat 180
   gcacagaagc tccagggcag agtcaccatg accacagaca catccacgag cacagcctac 240
   atggagctga ggagcctgag atctgacgac acggccgtgt attactgtgc gagagatgag 300
   gcccgtatag tagtggctgg tacaactcct tactactacg gtatggacgt ctggggccaa 360
   gggaccacgg tcaccgtctc ctca                                        384
<210> 51
   <211> 128
   <212> PRT
   <213> Artificial Sequence
<220>
   <223> Synthetic
<400> 51
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Asn Ser Tyr
               20                  25                  30
   Gly Ile Ser Trp Val Arg Gln Ala Pro Gly Gln Gly Leu Glu Trp Met
           35                  40                  45
   Gly Trp Ile Arg Thr Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Glu Ala Arg Ile Val Val Ala Gly Thr Thr Pro Tyr Tyr
               100                 105                 110
   Tyr Gly Met Asp Val Trp Gly Gln Gly Thr Thr Val Thr Val Ser Ser
           115                 120                 125
<210> 52
   <211> 24
   <212> DNA
   <213> Artificial Sequence
<220>
   <223> Synthetic
<400> 52
   ggttacacct ttaatagtta tgga 24
<210> 53
   <211> 8
   <212> PRT
   <213> Artificial Sequence
<220>
   <223> Synthetic
<400> 53
   Gly Tyr Thr Phe Asn Ser Tyr Gly
   1 5
<210> 54
   <211> 24
   <212> DNA
   <213> Artificial Sequence
<220>
   <223> Synthetic
<400> 54
   atcagaactt acaatggtaa caca 24
<210> 55
   <211> 8
   <212> PRT
   <213> Artificial Sequence
<220>
   <223> Synthetic
<400> 55
   Ile Arg Thr Tyr Asn Gly Asn Thr
   1 5
<210> 56
   <211> 63
   <212> DNA
   <213> Artificial Sequence
<220>
   <223> Synthetic
<400> 56
   gcgagagatg aggcccgtat agtagtggct ggtacaactc cttactacta cggtatggac 60
   gtc 63
<210> 57
   <211> 21
   <212> PRT
   <213> Artificial Sequence
<220>
   <223> Synthetic
<400> 57
   Ala Arg Asp Glu Ala Arg Ile Val Val Ala Gly Thr Thr Pro Tyr Tyr
    1               5                  10                  15
   Tyr Gly Met Asp Val
               20
<210> 58
   <211> 321
   <212> DNA
   <213> Artificial Sequence
<220>
   <223> Synthetic
<400> 58
   gacatccaga tgacccagtc tccatcttcc gtgtctgcat ctgtgggaga cagagtcacc 60
   atcacttgtc gggcgagtca ggatattagt atttggttag cctggtatca gcagagtcca 120
   gggaaagccc ctaaactcct gatcaatgtt gcatcccgtt tgcaaagtgg ggtcccatca 180
   aggttcagcg gcagtggatc tgggacagat ttcactctca ccatcaacag tctgcagcct 240
   gaagattttg taacttacta ttgtcaacag gctaacagtt tcccgatcac cttcggccaa 300
   gggacacgac tggcgaccaa a                                            321
<210> 59
   <211> 107
   <212> PRT
   <213> Artificial Sequence
<220>
   <223> Synthetic
<400> 59
   Asp Ile Gln Met Thr Gln Ser Pro Ser Ser Val Ser Ala Ser Val Gly
    1               5                  10                  15
   Asp Arg Val Thr Ile Thr Cys Arg Ala Ser Gln Asp Ile Ser Ile Trp
               20                  25                  30
   Leu Ala Trp Tyr Gln Gln Ser Pro Gly Lys Ala Pro Lys Leu Leu Ile
           35                  40                  45
   Asn Val Ala Ser Arg Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Val Thr Tyr Tyr Cys Gln Gln Ala Asn Ser Phe Pro Ile
                   85                  90                  95
   Thr Phe Gly Gln Gly Thr Arg Leu Ala Thr Lys
               100                 105
<210> 60
   <211> 18
   <212> DNA
   <213> Artificial Sequence
<220>
   <223> Synthetic
<400> 60
   caggatatta gtatttgg 18
<210> 61
   <211> 6
   <212> PRT
   <213> Artificial Sequence
<220>
   <223> Synthetic
<400> 61
   Gln Asp Ile Ser Ile Trp
   1 5
<210> 62
   <211> 9
   <212> DNA
   <213> Artificial Sequence
<220>
   <223> Synthetic
<400> 62
   gttgcatcc 9
<210> 63
   <211> 3
   <212> PRT
   <213> Artificial Sequence
<220>
   <223> Synthetic
<400> 63
   Val Ala Ser
   1
<210> 64
   <211> 27
   <212> DNA
   <213> Artificial Sequence
<220>
   <223> Synthetic
<400> 64
   caacaggcta acagtttccc gatcacc 27
<210> 65
   <211> 9
   <212> PRT
   <213> Artificial Sequence
<220>
   <223> Synthetic
<400> 65
   Gln Gln Ala Asn Ser Phe Pro Ile Thr
   1               5
<210> 66
   <211> 351
   <212> DNA
   <213> Artificial Sequence
<220>
   <223> Synthetic
<400> 66
   caggtgcagc tggtggagtc tgggggaggc ttggtccagc ctggagggtc cctgagactc 60
   tcctgtgcag tttctggatt cacaatcagt gaccactaca tgagctggat ccgccaggct 120
   ccagggaagg ggctggagtg gatttcatac attagtagta gtggtagtaa aatatactac 180
   gcagactctg tgaagggccg attcaccatc tccagggaca acgccaagaa ctcactgttt 240
   ctgcaaatga acagcctgag agccgaggac acggccgttt attactgtgc gagaacacga 300
   cagctcgtgg gggactactg gggccaggga accctggtca ccgtctcctc a          351
<210> 67
   <211> 117
   <212> PRT
   <213> Artificial Sequence
<220>
   <223> Synthetic
<400> 67
   Gln Val Gln Leu Val Glu Ser Gly Gly Gly Leu Val Gln Pro Gly Gly
   1               5                   10                  15
   Ser Leu Arg Leu Ser Cys Ala Val Ser Gly Phe Thr Ile Ser Asp His
               20                  25                  30
   Tyr Met Ser Trp Ile Arg Gln Ala Pro Gly Lys Gly Leu Glu Trp Ile
           35                  40                  45
   Ser Tyr Ile Ser Ser Ser Gly Ser Lys Ile Tyr Tyr Ala Asp Ser Val
       50                  55                  60
   Lys Gly Arg Phe Thr Ile Ser Arg Asp Asn Ala Lys Asn Ser Leu Phe
   65                  70                  75                  80
   Leu Gln Met Asn Ser Leu Arg Ala Glu Asp Thr Ala Val Tyr Tyr Cys
                   85                  90                  95
   Ala Arg Thr Arg Gln Leu Val Gly Asp Tyr Trp Gly Gln Gly Thr Leu
               100                 105                 110
   Val Thr Val Ser Ser
           115
<210> 68
   <211> 24
   <212> DNA
   <213> Artificial Sequence
<220>
   <223> Synthetic
<400> 68
   ggattcacaa tcagtgacca ctac 24
<210> 69
   <211> 8
   <212> PRT
   <213> Artificial Sequence
<220>
   <223> Synthetic
<400> 69
   Gly Phe Thr Ile Ser Asp His Tyr
   1 5
<210> 70
   <211> 24
   <212> DNA
   <213> Artificial Sequence
<220>
   <223> Synthetic
<400> 70
   attagtagta gtggtagtaa aata 24
<210> 71
   <211> 8
   <212> PRT
   <213> Artificial Sequence
<220>
   <223> Synthetic
<400> 71
   Ile Ser Ser Ser Gly Ser Lys Ile
   1 5
<210> 72
   <211> 30
   <212> DNA
   <213> Artificial Sequence
<220>
   <223> Synthetic
<400> 72
   gcgagaacac gacagctcgt gggggactac 30
<210> 73
   <211> 10
   <212> PRT
   <213> Artificial Sequence
<220>
   <223> Synthetic
<400> 73
   Ala Arg Thr Arg Gln Leu Val Gly Asp Tyr
   1 5 10
<210> 74
   <211> 321
   <212> DNA
   <213> Artificial Sequence
<220>
   <223> Synthetic
<400> 74
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaagctcct gatctttgct gcatccactt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt acccgctcac tttcggcgga 300
   gggaccaagg tggagatcag a                                           321
<210> 75
   <211> 107
   <212> PRT
   <213> Artificial Sequence
<220>
   <223> Synthetic
<400> 75
   Asp Ile Gln Leu Thr Gln Ser Pro Ser Phe Leu Ser Ala Ser Val Gly
   1               5                   10                  15
   Asp Arg Val Thr Ile Thr Cys Trp Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Phe Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Leu
                   85                  90                  95
   Thr Phe Gly Gly Gly Thr Lys Val Glu Ile Arg
               100                 105
<210> 76
   <211> 18
   <212> DNA
   <213> Artificial Sequence
<220>
   <223> Synthetic
<400> 76
   cagggcatta gcagttat 18
<210> 77
   <211> 6
   <212> PRT
   <213> Artificial Sequence
<220>
   <223> Synthetic
<400> 77
   Gln Gly Ile Ser Ser Tyr
   1 5
<210> 78
   <211> 9
   <212> DNA
   <213> Artificial Sequence
<220>
   <223> Synthetic
<400> 78
   gctgcatcc 9
<210> 79
   <211> 3
   <212> PRT
   <213> Artificial Sequence
<220>
   <223> Synthetic
<400> 79
   Ala Ala Ser
   1
<210> 80
   <211> 27
   <212> DNA
   <213> Artificial Sequence
<220>
   <223> Synthetic
<400> 80
   caacagctta atagttaccc gctcact 27
<210> 81
   <211> 9
   <212> PRT
   <213> Artificial Sequence
<220>
   <223> Synthetic
<400> 81
   Gln Gln Leu Asn Ser Tyr Pro Leu Thr
   1               5
<210> 82
   <211> 372
   <212> DNA
   <213> Artificial Sequence
<220>
   <223> Synthetic
<400> 82
   gaagtgcagc tggtggagtc tgggggaggc ttggtacagc ctggcaggtc cctgagactc 60
   tcctgtgcag cctctggatt cacctttgat aattatgcca tgcactgggt ccggcaagct 120
   ccagggaagg gcctggagtg ggtctcaggt attaggtgga atagtggtag cataggctat 180
   gcggactctg tgaagggccg attcaccatc tccagagaca acgccaagaa ctccctgtat 240
   ctgcaaatga atagtctgag agctgaggac acggccttgt attactgtgc aaaagaaggt 300
   ggatatagtg gctaccgccc cggacccttc tttgactact ggggccaggg aaccctggtc 360
   accgtctcct cc 372

<210> 83
   <211> 124
   <212> PRT
   <213> Artificial Sequence
<220>
   <223> Synthetic
<400> 83
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asn Tyr
               20                  25                  30
   Ala Met His Trp Val Arg Gln Ala Pro Gly Lys Gly Leu Glu Trp Val
           35                  40                  45
   Ser Gly Ile Arg Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Glu Gly Gly Tyr Ser Gly Tyr Arg Pro Gly Pro Phe Phe Asp
               100                 105                 110
   Tyr Trp Gly Gln Gly Thr Leu Val Thr Val Ser Ser
           115                 120
<210> 84
   <211> 24
   <212> DNA
   <213> Artificial Sequence
<220>
   <223> Synthetic
<400> 84
   ggattcacct ttgataatta tgcc 24
<210> 85
   <211> 8
   <212> PRT
   <213> Artificial Sequence
<220>
   <223> Synthetic
<400> 85
   Gly Phe Thr Phe Asp Asn Tyr Ala
    1               5
<210> 86
   <211> 24
   <212> DNA
   <213> Artificial Sequence
<220>
   <223> Synthetic
<400> 86
   attaggtgga atagtggtag cata 24
<210> 87
   <211> 8
   <212> PRT
   <213> Artificial Sequence
<220>
   <223> Synthetic
<400> 87
   Ile Arg Trp Asn Ser Gly Ser Ile
   1               5
<210> 88
   <211> 51
   <212> DNA
   <213> Artificial Sequence
<220>
   <223> Synthetic
<400> 88
   gcaaaagaag gtggatatag tggctaccgc cccggaccct tctttgacta c 51
<210> 89
   <211> 17
   <212> PRT
   <213> Artificial Sequence
<220>
   <223> Synthetic
<400> 89
   Ala Lys Glu Gly Gly Tyr Ser Gly Tyr Arg Pro Gly Pro Phe Phe Asp
   1               5                   10                  15
   Tyr
<210> 90
   <211> 321
   <212> DNA
   <213> Artificial Sequence
<220>
   <223> Synthetic
<400> 90
   gaaatagtga tgacgcagtc tccagccacc ctgtctgtgt ctccagggga aagagccacc 60
   ctctcctgca gggccagtca gagtgttaac tacaacttag cctggtacca gcataaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggccgctcac tttcggcgga 300
   gggaccaagg tggagatcaa a 321
<210> 91
   <211> 107
   <212> PRT
   <213> Artificial Sequence
<220>
   <223> Synthetic
<400> 91
   Glu Ile Val Met Thr Gln Ser Pro Ala Thr Leu Ser Val Ser Pro Gly
   1               5                   10                  15
   Glu Arg Ala Thr Leu Ser Cys Arg Ala Ser Gln Ser Val Asn Tyr Asn
               20                  25                  30
   Leu Ala Trp Tyr Gln His Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
               100                 105
<210> 92
   <211> 18
   <212> DNA
   <213> Artificial Sequence
<220>
   <223> Synthetic
<400> 92
   cagagtgtta actacaac 18
<210> 93
   <211> 6
   <212> PRT
   <213> Artificial Sequence
<220>
   <223> Synthetic
<400> 93
   Gln Ser Val Asn Tyr Asn
   1               5
<210> 94
   <211> 9
   <212> DNA
   <213> Artificial Sequence
<220>
   <223> Synthetic
<400> 94
   ggtgcatcc 9
<210> 95
   <211> 3
   <212> PRT
   <213> Artificial Sequence
<220>
   <223> Synthetic
<400> 95
   Gly Ala Ser
   1
<210> 96
   <211> 27
   <212> DNA
   <213> Artificial Sequence
<220>
   <223> Synthetic
<400> 96
   cagcagtata ataactggcc gctcact 27
<210> 97
   <211> 9
   <212> PRT
   <213> Artificial Sequence
<220>
   <223> Synthetic
<400> 97
   Gln Gln Tyr Asn Asn Trp Pro Leu Thr
   1 5
<210> 98
   <211> 351
   <212> DNA
   <213> Artificial Sequence
<220>
   <223> Synthetic
<400> 98
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtca cacaaactat 180
   gcacagaagc tccagggcag agtcaccatg accacagaca catccacgag cacagcctac 240
   atggagctga ggagcctcag atctgacgac acggccgtgt attactgtgc gagagggagt 300
   ggctacgatt ttgactcctg gggccaggga accctggtca ccgtctcctc a 351
<210> 99
   <211> 117
   <212> PRT
   <213> Artificial Sequence
<220>
   <223> Synthetic
<400> 99
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly His Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Ser Trp Gly Gln Gly Thr Leu
               100                 105                 110
   Val Thr Val Ser Ser
           115
<210> 100
   <211> 24
   <212> DNA
   <213> Artificial Sequence
<220>
   <223> Synthetic
<400> 100
   ggttacacct ttaccaacta tggt 24
<210> 101
   <211> 8
   <212> PRT
   <213> Artificial Sequence
<220>
   <223> Synthetic
<400> 101
   Gly Tyr Thr Phe Thr Asn Tyr Gly
    1               5
<210> 102
   <211> 24
   <212> DNA
   <213> Artificial Sequence
<220>
   <223> Synthetic
<400> 102
   atcagcgttt acaatggtca caca 24
<210> 103
   <211> 8
   <212> PRT
   <213> Artificial Sequence
<220>
   <223> Synthetic
<400> 103
   Ile Ser Val Tyr Asn Gly His Thr
   1 5
<210> 104
   <211> 30
   <212> DNA
   <213> Artificial Sequence
<220>
   <223> Synthetic
<400> 104
   gcgagaggga gtggctacga ttttgactcc 30
<210> 105
   <211> 10
   <212> PRT
   <213> Artificial Sequence
<220>
   <223> Synthetic
<400> 105
   Ala Arg Gly Ser Gly Tyr Asp Phe Asp Ser
   1 5 10
<210> 106
   <211> 321
   <212> DNA
   <213> Artificial Sequence
<220>
   <223> Synthetic
<400> 106
   gccatccaga tgacccagtc ttcatcctcc ctgtctgcat ctgttggaga cagagtcacc 60
   atcacttgcc gggcaagtca ggccattaga aatgctttag gctggtatca gcagaaaccg 120
   gggaaagccc ctaaggtcct gatctatgct gcatccagtt tacaaagtgg catcccatca 180
   aggttcagcg gcagtggatc tggcacagat ttcactctca ccatcagcag cctgcagcct 240
   gaagattttg caacttatta ctgtctacaa gattacgatt acccgtacac ttttggccag 300
   gggaccaagc tggagatcaa a 321
<210> 107
   <211> 107
   <212> PRT
   <213> Artificial Sequence
<220>
   <223> Synthetic
<400> 107
   Ala Ile Gln Met Thr Gln Ser Ser Ser Ser Leu Ser Ala Ser Val Gly
    1               5                  10                  15
   Asp Arg Val Thr Ile Thr Cys Arg Ala Ser Gln Ala Ile Arg Asn Ala
               20                  25                  30
   Leu Gly Trp Tyr Gln Gln Lys Pro Gly Lys Ala Pro Lys Val Leu Ile
           35                  40                  45
   Tyr Ala Ala Ser Ser Leu Gln Ser Gly Ile Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asp Tyr Pro Tyr
                   85                  90                  95
   Thr Phe Gly Gln Gly Thr Lys Leu Glu Ile Lys
               100                 105
<210> 108
   <211> 18
   <212> DNA
   <213> Artificial Sequence
<220>
   <223> Synthetic
<400> 108
   caggccatta gaaatgct 18
<210> 109
   <211> 6
   <212> PRT
   <213> Artificial Sequence
<220>
   <223> Synthetic
<400> 109
   Gln Ala Ile Arg Asn Ala
    1               5
<210> 110
   <211> 9
   <212> DNA
   <213> Artificial Sequence
<220>
   <223> Synthetic
<400> 110
   gctgcatcc 9
<210> 111
   <211> 3
   <212> PRT
   <213> Artificial Sequence
<220>
   <223> Synthetic
<400> 111
   Ala Ala Ser
   1
<210> 112
   <211> 27
   <212> DNA
   <213> Artificial Sequence
<220>
   <223> Synthetic
<400> 112
   ctacaagatt acgattaccc gtacact 27
<210> 113
   <211> 9
   <212> PRT
   <213> Artificial Sequence
<220>
   <223> Synthetic
<400> 113
   Leu Gln Asp Tyr Asp Tyr Pro Tyr Thr
   1 5
<210> 114
   <211> 348
   <212> DNA
   <213> Artificial Sequence
<220>
   <223> Synthetic
<400> 114
   caggtgcagc tggtacagtc tggggctgag gtgaagaagc ctggggcctc agtgaaggtc 60
   tcctgcaagg cttctagata caccttcacc agttatgata tcaactgggt gcgacaggcc 120
   actggacaag ggcttgagtg gatgggatgg atgaacccaa acagtggtaa cacaggctat 180
   gcacagaagt tccagggcag agtcaccatg accaggaaca cctccacaag cacagcctac 240
   atggagctga gcagcctgag atctgaggac acggccgtgt attactgtgc gagagtacga 300
   cgcttttttg actactgggg ccagggaacc ctggtcaccg tctcctca             348
<210> 115
   <211> 116
   <212> PRT
   <213> Artificial Sequence
<220>
   <223> Synthetic
<400> 115
   Gln Val Gln Leu Val Gln Ser Gly Ala Glu Val Lys Lys Pro Gly Ala
   1               5                   10                  15
   Ser Val Lys Val Ser Cys Lys Ala Ser Arg Tyr Thr Phe Thr Ser Tyr
               20                  25                  30
   Asp Ile Asn Trp Val Arg Gln Ala Thr Gly Gln Gly Leu Glu Trp Met
           35                  40                  45
   Gly Trp Met Asn Pro Asn Ser Gly Asn Thr Gly Tyr Ala Gln Lys Phe
       50                  55                  60
   Gln Gly Arg Val Thr Met Thr Arg Asn Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Val Arg Arg Phe Phe Asp Tyr Trp Gly Gln Gly Thr Leu Val
               100                 105                 110
   Thr Val Ser Ser
           115
<210> 116
   <211> 24
   <212> DNA
   <213> Artificial Sequence
<220>
   <223> Synthetic
<400> 116
   agatacacct tcaccagtta tgat 24
<210> 117
   <211> 8
   <212> PRT
   <213> Artificial Sequence
<220>
   <223> Synthetic
<400> 117
   Arg Tyr Thr Phe Thr Ser Tyr Asp
   1 5
<210> 118
   <211> 24
   <212> DNA
   <213> Artificial Sequence
<220>
   <223> Synthetic
<400> 118
   atgaacccaa acagtggtaa caca 24
<210> 119
   <211> 8
   <212> PRT
   <213> Artificial Sequence
<220>
   <223> Synthetic
<400> 119
   Met Asn Pro Asn Ser Gly Asn Thr
   1 5
<210> 120
   <211> 27
   <212> DNA
   <213> Artificial Sequence
<220>
   <223> Synthetic
<400> 120
   gcgagagtac gacgcttttt tgactac 27
<210> 121
   <211> 9
   <212> PRT
   <213> Artificial Sequence
<220>
   <223> Synthetic
<400> 121
   Ala Arg Val Arg Arg Phe Phe Asp Tyr
   1 5
<210> 122
   <211> 321
   <212> DNA
   <213> Artificial Sequence
<220>
   <223> Synthetic
<400> 122
   gacatccagt tgacccagtc tccatccttc ctgtctgcat ctgtaggaga cagagtcacc 60
   atcacttgct gggccagtca gggcattatc agttatttag cctggtatca gcaaaaacca 120
   gggaaagccc ctaagctcct gatctatgct gcatccactt tgcacagtgg ggtcccatca 180
   aggttcagcg gcagtggatc tgggacagaa ttcactctca caatcagcag cctgcagcct 240
   gaagattttg caacttatta ctgtcaccag cttaaaagtt acccgatcac cttcggccaa 300
   gggacacgac tggagattaa a 321
<210> 123
   <211> 107
   <212> PRT
   <213> Artificial Sequence
<220>
   <223> Synthetic
<400> 123
   Asp Ile Gln Leu Thr Gln Ser Pro Ser Phe Leu Ser Ala Ser Val Gly
   1 5 10 15
   Asp Arg Val Thr Ile Thr Cys Trp Ala Ser Gln Gly Ile Ile Ser Tyr
   20 25 30
   Leu Ala Trp Tyr Gln Gln Lys Pro Gly Lys Ala Pro Lys Leu Leu Ile
   35 40 45
   Tyr Ala Ala Ser Thr Leu His Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Glu Asp Phe Ala Thr Tyr Tyr Cys His Gln Leu Lys Ser Tyr Pro Ile
   85 90 95
   Thr Phe Gly Gln Gly Thr Arg Leu Glu Ile Lys
   100 105
<210> 124
   <211> 18
   <212> DNA
   <213> Artificial Sequence
<220>
   <223> Synthetic
<400> 124
   cagggcatta tcagttat 18
<210> 125
   <211> 6
   <212> PRT
   <213> Artificial Sequence
<220>
   <223> Synthetic
<400> 125
   Gln Gly Ile Ile Ser Tyr
   1 5
<210> 126
   <211> 9
   <212> DNA
   <213> Artificial Sequence
<220>
   <223> Synthetic
<400> 126
   gctgcatcc 9
<210> 127
   <211> 3
   <212> PRT
   <213> Artificial Sequence
<220>
   <223> Synthetic
<400> 127
   Ala Ala Ser
   1
<210> 128
   <211> 27
   <212> DNA
   <213> Artificial Sequence
<220>
   <223> Synthetic
<400> 128
   caccagctta aaagttaccc gatcacc 27
<210> 129
   <211> 9
   <212> PRT
   <213> Artificial Sequence
<220>
   <223> Synthetic
<400> 129
   His Gln Leu Lys Ser Tyr Pro Ile Thr
   1 5
<210> 130
   <211> 348
   <212> DNA
   <213> Artificial Sequence
<220>
   <223> Synthetic
<400> 130
   caggtgcagt tggtggagac tgggggagac ttggtcaggc ctggagggtc cctgagactc 60
   tcctgtacag cctctggatt caccctcagt gactactaca tgagctggat ccgtcaggct 120
   ccagggaagg ggctggagtg ggtttcatac attaggagaa gtggtaatac catatactac 180
   gcagactctg tgaagggccg attcaccatc tccagggaca acgccaagaa ctcactgtat 240
   ctgcaaatga acagcctgag agccgaggac acggccgtat attactgtgc gagagggggg 300
   gactgggcgg acgtctgggg ccaagggacc acggtcaccg tctcctca             348
<210> 131
   <211> 116
   <212> PRT
   <213> Artificial Sequence
<220>
   <223> Synthetic
<400> 131
   Gln Val Gln Leu Val Glu Thr Gly Gly Asp Leu Val Arg Pro Gly Gly
    1               5                  10                  15
   Ser Leu Arg Leu Ser Cys Thr Ala Ser Gly Phe Thr Leu Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Arg Arg Ser Gly Asn Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Trp Ala Asp Val Trp Gly Gln Gly Thr Thr Val
               100                 105                 110
   Thr Val Ser Ser
           115
<210> 132
   <211> 24
   <212> DNA
   <213> Artificial Sequence
<220>
   <223> Synthetic
<400> 132
   ggattcaccc tcagtgacta ctac 24
<210> 133
   <211> 8
   <212> PRT
   <213> Artificial Sequence
<220>
   <223> Synthetic
<400> 133
   Gly Phe Thr Leu Ser Asp Tyr Tyr
   1 5
<210> 134
   <211> 24
   <212> DNA
   <213> Artificial Sequence
<220>
   <223> Synthetic
<400> 134
   attaggagaa gtggtaatac cata 24
<210> 135
   <211> 8
   <212> PRT
   <213> Artificial Sequence
<220>
   <223> Synthetic
<400> 135
   Ile Arg Arg Ser Gly Asn Thr Ile
   1 5
<210> 136
   <211> 27
   <212> DNA
   <213> Artificial Sequence
<220>
   <223> Synthetic
<400> 136
   gcgagagggg gggactgggc ggacgtc 27
<210> 137
   <211> 9
   <212> PRT
   <213> Artificial Sequence
<220>
   <223> Synthetic
<400> 137
   Ala Arg Gly Gly Asp Trp Ala Asp Val
   1 5
<210> 138
   <211> 321
   <212> DNA
   <213> Artificial Sequence
<220>
   <223> Synthetic
<400> 138
   gacatccaga tgacccagtc tccatcctca ctgtctgcct ctgtgggaga cagagtcacc 60
   atcacttgtc gggcgagtca gggcattagc atttatttag cctggtttca gcagaaacca 120
   gggaaagccc ctaagtccct gatctatgct gcatccagtt tgcaacgtgg ggtcccatca 180
   aagttcagcg gcagtggatc tgggacagat ttcactctca ccatcagtag cctgcagcct 240
   gaagattttg caacttatta ctgccaacag tataatagtt acccgtacac ttttggccag 300
   gggaccaaac tggaaatcaa a                                           321
<210> 139
   <211> 107
   <212> PRT
   <213> Artificial Sequence
<220>
   <223> Synthetic
<400> 139
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Ser Ile Tyr
               20                  25                  30
   Leu Ala Trp Phe Gln Gln Lys Pro Gly Lys Ala Pro Lys Ser Leu Ile
           35                  40                  45
   Tyr Ala Ala Ser Ser Leu Gln Arg Gly Val Pro Ser Lys Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 140
   <211> 18
   <212> DNA
   <213> Artificial Sequence
<220>
   <223> Synthetic
<400> 140
   cagggcatta gcatttat 18
<210> 141
   <211> 6
   <212> PRT
   <213> Artificial Sequence
<220>
   <223> Synthetic
<400> 141
   Gln Gly Ile Ser Ile Tyr
   1 5
<210> 142
   <211> 9
   <212> DNA
   <213> Artificial Sequence
<220>
   <223> Synthetic
<400> 142
   gctgcatcc 9
<210> 143
   <211> 3
   <212> PRT
   <213> Artificial Sequence
<220>
   <223> Synthetic
<400> 143
   Ala Ala Ser
   1
<210> 144
   <211> 27
   <212> DNA
   <213> Artificial Sequence
<220>
   <223> Synthetic
<400> 144
   caacagtata atagttaccc gtacact 27
<210> 145
   <211> 9
   <212> PRT
   <213> Artificial Sequence
<220>
   <223> Synthetic
<400> 145
   Gln Gln Tyr Asn Ser Tyr Pro Tyr Thr
   1 5
<210> 146
   <211> 351
   <212> DNA
   <213> Artificial Sequence
<220>
   <223> Synthetic
<400> 146
   caggttcagc tggtgcagtc tggacctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa cataaactat 180
   gcacagaagc tccagggcag agtcaccatg accacagaca catccacgag cacagcctac 240
   atggacctga ggagcctgag atctgacgac acggccgtgt attactgtgc gagagggagt 300
   ggctacgatt ttgactactg gggccaggga accctggtca ccgtctcctc a         351
<210> 147
   <211> 117
   <212> PRT
   <213> Artificial Sequence
<220>
   <223> Synthetic
<400> 147
   Gln Val Gln Leu Val Gln Ser Gly Pro Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Ile Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Asp Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 148
   <211> 24
   <212> DNA
   <213> Artificial Sequence
<220>
   <223> Synthetic
<400> 148
   ggttacacct ttaccaacta tggt 24
<210> 149
   <211> 8
   <212> PRT
   <213> Artificial Sequence
<220>
   <223> Synthetic
<400> 149
   Gly Tyr Thr Phe Thr Asn Tyr Gly
   1 5
<210> 150
   <211> 24
   <212> DNA
   <213> Artificial Sequence
<220>
   <223> Synthetic
<400> 150
   atcagcgttt acaatggtaa cata 24
<210> 151
   <211> 8
   <212> PRT
   <213> Artificial Sequence
<220>
   <223> Synthetic
<400> 151
   Ile Ser Val Tyr Asn Gly Asn Ile
   1 5
<210> 152
   <211> 30
   <212> DNA
   <213> Artificial Sequence
<220>
   <223> Synthetic
<400> 152
   gcgagaggga gtggctacga ttttgactac 30
<210> 153
   <211> 10
   <212> PRT
   <213> Artificial Sequence
<220>
   <223> Synthetic
<400> 153
   Ala Arg Gly Ser Gly Tyr Asp Phe Asp Tyr
   1               5                   10
<210> 154
   <211> 321
   <212> DNA
   <213> Artificial Sequence
<220>
   <223> Synthetic
<400> 154
   gccatccaga tgacccagtc tccatcctcc ctgtctgcat ctgttggaga cagagtcacc 60
   atcacttgcc gggcaagtca ggacattaga aatgctttag gctggtatca gcagaaacca 120
   gggaaagccc ctaagctcct gatctatgct gcatccagtt tacaaagtgg ggtcccatca 180
   aggttcagcg gcagtgcttc tggcacagat ttcactctca ccatcagcag cctgcagcct 240
   gaagattttg cagcttatta ctgtctacaa gattacaatt acccgtacac ttttggccag 300
   gggaccaagc tggagatcaa a                                           321
<210> 155
   <211> 107
   <212> PRT
   <213> Artificial Sequence
<220>
   <223> Synthetic
<400> 155
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Ala Ser Gly Thr Asp Phe Thr Leu Thr Ile Ser Ser Leu Gln Pro
   65                  70                  75                  80
   Glu Asp Phe Ala Ala Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 156
   <211> 18
   <212> DNA
   <213> Artificial Sequence
<220>
   <223> Synthetic
<400> 156
   caggacatta gaaatgct 18
<210> 157
   <211> 6
   <212> PRT
   <213> Artificial Sequence
<220>
   <223> Synthetic
<400> 157
   Gln Asp Ile Arg Asn Ala
   1 5
<210> 158
   <211> 9
   <212> DNA
   <213> Artificial Sequence
<220>
   <223> Synthetic
<400> 158
   gctgcatcc 9
<210> 159
   <211> 3
   <212> PRT
   <213> Artificial Sequence
<220>
   <223> Synthetic
<400> 159
   Ala Ala Ser
   1
<210> 160
   <211> 27
   <212> DNA
   <213> Artificial Sequence
<220>
   <223> Synthetic
<400> 160
   ctacaagatt acaattaccc gtacact 27
<210> 161
   <211> 9
   <212> PRT
   <213> Artificial Sequence
<220>
   <223> Synthetic
<400> 161
   Leu Gln Asp Tyr Asn Tyr Pro Tyr Thr
   1 5
<210> 162
   <211> 366
   <212> DNA
   <213> Artificial Sequence
<220>
   <223> Synthetic
<400> 162
   caggttcagc tggtgcagtc tggagctgag gtgaagaagc ctggggcctc agtgaaggtc 60
   tcctgcaagg attctgctta cacctttaat agatatggta tcagctgggt gcgacaggcc 120
   cctggacaag gccttgagtg gatgggatgg atcagcgctt acactggtaa cacagtctat 180
   gcacagaagc tccagggcag agtcaccatg accacagaca attccacgag cacagcttac 240
   atggagctga ggagcctgag atctgacgac acggccgtgt attactgtgc gagagataag 300
   tcgatttttg gagtggttag agggtttgac tactggggcc agggaaccct ggtcaccgtc 360
   tcctca                                                            366
<210> 163
   <211> 122
   <212> PRT
   <213> Artificial Sequence
<220>
   <223> Synthetic
<400> 163
   Gln Val Gln Leu Val Gln Ser Gly Ala Glu Val Lys Lys Pro Gly Ala
   1               5                   10                  15
   Ser Val Lys Val Ser Cys Lys Asp Ser Ala Tyr Thr Phe Asn Arg Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Thr Gly Asn Thr Val Tyr Ala Gln Lys Leu
       50                  55                  60
   Gln Gly Arg Val Thr Met Thr Thr Asp Asn Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Lys Ser Ile Phe Gly Val Val Arg Gly Phe Asp Tyr Trp
               100                 105                 110
   Gly Gln Gly Thr Leu Val Thr Val Ser Ser
           115                 120
<210> 164
   <211> 24
   <212> DNA
   <213> Artificial Sequence
<220>
   <223> Synthetic
<400> 164
   gcttacacct ttaatagata tggt 24
<210> 165
   <211> 8
   <212> PRT
   <213> Artificial Sequence
<220>
   <223> Synthetic
<400> 165
   Ala Tyr Thr Phe Asn Arg Tyr Gly
   1 5
<210> 166
   <211> 24
   <212> DNA
   <213> Artificial Sequence
<220>
   <223> Synthetic
<400> 166
   atcagcgctt acactggtaa caca 24
<210> 167
   <211> 8
   <212> PRT
   <213> Artificial Sequence
<220>
   <223> Synthetic
<400> 167
   Ile Ser Ala Tyr Thr Gly Asn Thr
   1 5

<210> 168
   <211> 45
   <212> DNA
   <213> Artificial Sequence
<220>
   <223> Synthetic
<400> 168
   gcgagagata agtcgatttt tggagtggtt agagggtttg actac 45
<210> 169
   <211> 15
   <212> PRT
   <213> Artificial Sequence
<220>
   <223> Synthetic
<400> 169
   Ala Arg Asp Lys Ser Ile Phe Gly Val Val Arg Gly Phe Asp Tyr
    1               5                  10                  15
<210> 170
   <211> 321
   <212> DNA
   <213> Artificial Sequence
<220>
   <223> Synthetic
<400> 170
   gaaatagtga tgacgcagtc tccagtcacc ctgtctctgt ctccagggga aagagccacc 60
   ctcccctgca gggccagtca gagtgttagc agcagcttag cctggtacca gcagaaagct 120
   ggccagtctc ccaggctcct catctatggt gcatctacca gggccactgg tatcccagcc 180
   aggttcagtg gcagtgggtc tgggacggag ttcactctca ccatcagcaa cctgcagtct 240
   gaagattttg cagtttatta ctgtcagcag tataataact ggcctctcac tttcggcgga 300
   gggaccaagg tggagatcaa a                                            321
<210> 171
   <211> 107
   <212> PRT
   <213> Artificial Sequence
<220>
   <223> Synthetic
<400> 171
   Glu Ile Val Met Thr Gln Ser Pro Val Thr Leu Ser Leu Ser Pro Gly
    1               5                  10                  15
   Glu Arg Ala Thr Leu Pro Cys Arg Ala Ser Gln Ser Val Ser Ser Ser
               20                  25                  30
   Leu Ala Trp Tyr Gln Gln Lys Ala Gly Gln Ser Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Asn Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
               100                 105
<210> 172
   <211> 18
   <212> DNA
   <213> Artificial Sequence
<220>
   <223> Synthetic
<400> 172
   cagagtgtta gcagcagc 18
<210> 173
   <211> 6
   <212> PRT
   <213> Artificial Sequence
<220>
   <223> Synthetic
<400> 173
   Gln Ser Val Ser Ser Ser
   1 5
<210> 174
   <211> 9
   <212> DNA
   <213> Artificial Sequence
<220>
   <223> Synthetic
<400> 174
   ggtgcatct 9
<210> 175
   <211> 3
   <212> PRT
   <213> Artificial Sequence
<220>
   <223> Synthetic
<400> 175
   Gly Ala Ser
   1
<210> 176
   <211> 27
   <212> DNA
   <213> Artificial Sequence
<220>
   <223> Synthetic
<400> 176
   cagcagtata ataactggcc tctcact 27
<210> 177
   <211> 9
   <212> PRT
   <213> Artificial Sequence
<220>
   <223> Synthetic
<400> 177
   Gln Gln Tyr Asn Asn Trp Pro Leu Thr
   1 5
<210> 178
   <211> 351
   <212> DNA
   <213> Artificial Sequence
<220>
   <223> Synthetic
<400> 178
   caggttcagc tggtgcagtc tggagctgaa gtgaagaagc ctggggcctc agtgaaggtc 60
   tcctgcaagg cttctggtta cacctttacc aactatggcc tcacttgggt gcgacaggcc 120
   ccgggacgag gtcttgagtg gatgggatgg atcagtgttt acaatggcaa cacaaactat 180
   gcacagacgc tcaagggcag agtcaccatg accacagaca catccacgag cacagcctac 240
   atggaactga agagcctgag atctgacgac acggccgttt attactgtgc gagagggagt 300
   ggctacgatt ttgactactg gggccaggga accctggtca ccgtctcctc a          351
<210> 179
   <211> 117
   <212> PRT
   <213> Artificial Sequence
<220>
   <223> Synthetic
<400> 179
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Leu Thr Trp Val Arg Gln Ala Pro Gly Arg Gly Leu Glu Trp Met
           35                  40                  45
   Gly Trp Ile Ser Val Tyr Asn Gly Asn Thr Asn Tyr Ala Gln Thr Leu
       50                  55                  60
   Lys Gly Arg Val Thr Met Thr Thr Asp Thr Ser Thr Ser Thr Ala Tyr
   65                  70                  75                  80
   Met Glu Leu Lys Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 180
   <211> 24
   <212> DNA
   <213> Artificial Sequence
<220>
   <223> Synthetic
<400> 180
   ggttacacct ttaccaacta tggc 24
<210> 181
   <211> 8
   <212> PRT
   <213> Artificial Sequence
<220>
   <223> Synthetic
<400> 181
   Gly Tyr Thr Phe Thr Asn Tyr Gly
    1               5
<210> 182
   <211> 24
   <212> DNA
   <213> Artificial Sequence
<220>
   <223> Synthetic
<400> 182
   atcagtgttt acaatggcaa caca 24
<210> 183
   <211> 8
   <212> PRT
   <213> Artificial Sequence
<220>
   <223> Synthetic
<400> 183
   Ile Ser Val Tyr Asn Gly Asn Thr
   1 5
<210> 184
   <211> 30
   <212> DNA
   <213> Artificial Sequence
<220>
   <223> Synthetic
<400> 184
   gcgagaggga gtggctacga ttttgactac 30
<210> 185
   <211> 10
   <212> PRT
   <213> Artificial Sequence
<220>
   <223> Synthetic
<400> 185
   Ala Arg Gly Ser Gly Tyr Asp Phe Asp Tyr
   1 5 10
<210> 186
   <211> 321
   <212> DNA
   <213> Artificial Sequence
<220>
   <223> Synthetic
<400> 186
   gccatccaga tgacccagtc tccttcctcc ctgtctgcat ctgttggaga cagagtcgcc 60
   atcacttgcc gggcaagtca ggacattaga aatgatttag gctggtatca gcagaaacca 120
   gggaaagccc ctaagcttct gatccatact gcttccagtt tacaaagtgg ggtcccatca 180
   aggttcagcg gcagtggatc tggcacagat ttcactctca ccatcagcaa cctgcagcct 240
   gaagattttg ccacttatta ctgtctccaa gataatagtt acccgtacac ttttggccag 300
   gggaccaagc tggagatcaa t 321
<210> 187
   <211> 107
   <212> PRT
   <213> Artificial Sequence
<220>
   <223> Synthetic
<400> 187
   Ala Ile Gln Met Thr Gln Ser Pro Ser Ser Leu Ser Ala Ser Val Gly
    1               5                  10                  15
   Asp Arg Val Ala Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   His Thr Ala Ser Ser Leu Gln Ser Gly Val Pro Ser Arg Phe Ser Gly
       50                  55                  60
   Ser Gly Ser Gly Thr Asp Phe Thr Leu Thr Ile Ser Asn Leu Gln Pro
   65                  70                  75                  80
   Glu Asp Phe Ala Thr Tyr Tyr Cys Leu Gln Asp Asn Ser Tyr Pro Tyr
                   85                  90                  95
   Thr Phe Gly Gln Gly Thr Lys Leu Glu Ile Asn
               100                 105
<210> 188
   <211> 18
   <212> DNA
   <213> Artificial Sequence
<220>
   <223> Synthetic
<400> 188
   caggacatta gaaatgat 18
<210> 189
   <211> 6
   <212> PRT
   <213> Artificial Sequence
<220>
   <223> Synthetic
<400> 189
   Gln Asp Ile Arg Asn Asp
    1               5
<210> 190
   <211> 9
   <212> DNA
   <213> Artificial Sequence
<220>
   <223> Synthetic
<400> 190
   actgcttcc 9
<210> 191
   <211> 3
   <212> PRT
   <213> Artificial Sequence
<220>
   <223> Synthetic
<400> 191
   Thr Ala Ser
   1
<210> 192
   <211> 27
   <212> DNA
   <213> Artificial Sequence
<220>
   <223> Synthetic
<400> 192
   ctccaagata atagttaccc gtacact 27
<210> 193
   <211> 9
   <212> PRT
   <213> Artificial Sequence
<220>
   <223> Synthetic
<400> 193
   Leu Gln Asp Asn Ser Tyr Pro Tyr Thr
   1 5
<210> 194
   <211> 348
   <212> DNA
   <213> Artificial Sequence
<220>
   <223> Synthetic
<400> 194
   caggtgcaac tggtgcagtc tggggctgag gtgcagaagc ctggggcctc agtgaaggtc 60
   tcctgcaagg cttctggata caccttcacc ggccagtata ttcactgggt tcgacaggcc 120
   cctggacaag gacttgagtg gatgggatgg atcaacccta acagtggtgg cacaaacttt 180
   gcacagaagt ttcaggacag ggtcaccatg accagggaca cgtccatcgg cacagtatac 240
   atggaactga ggaggctgac atctgacgac acggccgtat tttattgtgt gagagaaaag 300
   agaggttttg atatctgggg ccaaggcaca atggtcaccg tttcttca 348
<210> 195
   <211> 116
   <212> PRT
   <213> Artificial Sequence
<220>
   <223> Synthetic
<400> 195
   Gln Val Gln Leu Val Gln Ser Gly Ala Glu Val Gln Lys Pro Gly Ala
   1 5 10 15
   Ser Val Lys Val Ser Cys Lys Ala Ser Gly Tyr Thr Phe Thr Gly Gln
   20 25 30
   Tyr Ile His Trp Val Arg Gln Ala Pro Gly Gln Gly Leu Glu Trp Met
   35 40 45
   Gly Trp Ile Asn Pro Asn Ser Gly Gly Thr Asn Phe Ala Gln Lys Phe
   50 55 60
   Gln Asp Arg Val Thr Met Thr Arg Asp Thr Ser Ile Gly Thr Val Tyr
   65 70 75 80
   Met Glu Leu Arg Arg Leu Thr Ser Asp Asp Thr Ala Val Phe Tyr Cys
   85 90 95
   Val Arg Glu Lys Arg Gly Phe Asp Ile Trp Gly Gln Gly Thr Met Val
   100 105 110
   Thr Val Ser Ser
   115
<210> 196
   <211> 24
   <212> DNA
   <213> Artificial Sequence
<220>
   <223> Synthetic
<400> 196
   ggatacacct tcaccggcca gtat 24
<210> 197
   <211> 8
   <212> PRT
   <213> Artificial Sequence
<220>
   <223> Synthetic
<400> 197
   Gly Tyr Thr Phe Thr Gly Gln Tyr
   1 5
<210> 198
   <211> 24
   <212> DNA
   <213> Artificial Sequence
<220>
   <223> Synthetic
<400> 198
   atcaacccta acagtggtgg caca 24
<210> 199
   <211> 8
   <212> PRT
   <213> Artificial Sequence
<220>
   <223> Synthetic
<400> 199
   Ile Asn Pro Asn Ser Gly Gly Thr
   1               5
<210> 200
   <211> 27
   <212> DNA
   <213> Artificial Sequence
<220>
   <223> Synthetic
<400> 200
   gtgagagaaa agagaggttt tgatatc 27
<210> 201
   <211> 9
   <212> PRT
   <213> Artificial Sequence
<220>
   <223> Synthetic
<400> 201
   Val Arg Glu Lys Arg Gly Phe Asp Ile
   1               5
<210> 202
   <211> 321
   <212> DNA
   <213> Artificial Sequence
<220>
   <223> Synthetic
<400> 202
   gaaatagtga tgacgcagtc tccagccacc ctgtctgtgt ctccagggga aagagccacc 60
   ctctcctgca gggccagtca gagtgttagc agcaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggccattcac tttcggccct 300
   gggaccaaag tggatatcaa a 321
<210> 203
   <211> 107
   <212> PRT
   <213> Artificial Sequence
<220>
   <223> Synthetic
<400> 203
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Phe
                   85                  90                  95
   Thr Phe Gly Pro Gly Thr Lys Val Asp Ile Lys
               100                 105
<210> 204
   <211> 18
   <212> DNA
   <213> Artificial Sequence
<220>
   <223> Synthetic
<400> 204
   cagagtgtta gcagcaac 18
<210> 205
   <211> 6
   <212> PRT
   <213> Artificial Sequence
<220>
   <223> Synthetic
<400> 205
   Gln Ser Val Ser Ser Asn
    1               5
<210> 206
   <211> 9
   <212> DNA
   <213> Artificial Sequence
<220>
   <223> Synthetic
<400> 206
   ggtgcatcc 9
<210> 207
   <211> 3
   <212> PRT
   <213> Artificial Sequence
<220>
   <223> Synthetic
<400> 207
   Gly Ala Ser
   1
<210> 208
   <211> 27
   <212> DNA
   <213> Artificial Sequence
<220>
   <223> Synthetic
<400> 208
   cagcagtata ataactggcc attcact 27
<210> 209
   <211> 9
   <212> PRT
   <213> Artificial Sequence
<220>
   <223> Synthetic
<400> 209
   Gln Gln Tyr Asn Asn Trp Pro Phe Thr
   1 5
<210> 210
   <211> 351
   <212> DNA
   <213> Artificial Sequence
<220>
   <223> Synthetic
<400> 210
   caggttcatc tggtgcagtc tggagctgag gtgaagaagc ctggggcctc agtgaaggtc 60
   tcctgcaagg cttctggtta cacctttacc aactatggta tcggctgggt gcgacaggcc 120
   cctggacaag ggcttgagtg gatgggatgg atcagtattt acaatggtaa cacaaattat 180
   gcacagaggc tccagggcag agtcaccatg accacagaca catccacgag cacagccttc 240
   atggacctga ggagcctgaa atctgacgac acggccgttt attactgtgc gagaggtagt 300
   ggctacaatt tcgaccactg gggccaggga accctggtca ccgtctcctc c 351
<210> 211
   <211> 117
   <212> PRT
   <213> Artificial Sequence
<220>
   <223> Synthetic
<400> 211
   Gln Val His Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Gly Ile Gly Trp Val Arg Gln Ala Pro Gly Gln Gly Leu Glu Trp Met
   35 40 45
   Gly Trp Ile Ser Ile Tyr Asn Gly Asn Thr Asn Tyr Ala Gln Arg Leu
   50 55 60
   Gln Gly Arg Val Thr Met Thr Thr Asp Thr Ser Thr Ser Thr Ala Phe
   65 70 75 80
   Met Asp Leu Arg Ser Leu Lys Ser Asp Asp Thr Ala Val Tyr Tyr Cys
   85 90 95
   Ala Arg Gly Ser Gly Tyr Asn Phe Asp His Trp Gly Gln Gly Thr Leu
   100 105 110
   Val Thr Val Ser Ser
   115
<210> 212
   <211> 24
   <212> DNA
   <213> Artificial Sequence
<220>
   <223> Synthetic
<400> 212
   ggttacacct ttaccaacta tggt 24
<210> 213
   <211> 8
   <212> PRT
   <213> Artificial Sequence
<220>
   <223> Synthetic
<400> 213
   Gly Tyr Thr Phe Thr Asn Tyr Gly
   1 5
<210> 214
   <211> 24
   <212> DNA
   <213> Artificial Sequence
<220>
   <223> Synthetic
<400> 214
   atcagtattt acaatggtaa caca 24
<210> 215
   <211> 8
   <212> PRT
   <213> Artificial Sequence
<220>
   <223> Synthetic
<400> 215
   Ile Ser Ile Tyr Asn Gly Asn Thr
   1 5
<210> 216
   <211> 30
   <212> DNA
   <213> Artificial Sequence
<220>
   <223> Synthetic
<400> 216
   gcgagaggta gtggctacaa tttcgaccac 30
<210> 217
   <211> 10
   <212> PRT
   <213> Artificial Sequence
<220>
   <223> Synthetic
<400> 217
   Ala Arg Gly Ser Gly Tyr Asn Phe Asp His
   1 5 10
<210> 218
   <211> 321
   <212> DNA
   <213> Artificial Sequence
<220>
   <223> Synthetic
<400> 218
   gccatccaga tgacccagtc tccatcctcc ctgtctgcat ctgtaggaga cagagtcacc 60
   atcacttgcc gggcaagtca gggcattaga aatgatttag gctggtatca gcagataccg 120
   gggaaagccc ctaaactcct gatctatgct gcatccagtt tacaaagtgg ggtcccgtca 180
   aggttcagcg gcagtggatc tggcacagat ttcactctca ccatcagcaa cctgcagcct 240
   gaagattttg cagtttattt ctgtctacaa gataacactt atccgtacac ttttggccag 300
   gggaccaatc tggagagcaa a                                           321
<210> 219
   <211> 107
   <212> PRT
   <213> Artificial Sequence
<220>
   <223> Synthetic
<400> 219
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Asp
               20                  25                  30
   Leu Gly Trp Tyr Gln Gln Ile Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Asn Leu Gln Pro
   65                  70                  75                  80
   Glu Asp Phe Ala Val Tyr Phe Cys Leu Gln Asp Asn Thr Tyr Pro Tyr
                   85                  90                  95
   Thr Phe Gly Gln Gly Thr Asn Leu Glu Ser Lys
               100                 105
<210> 220
   <211> 18
   <212> DNA
   <213> Artificial Sequence
<220>
   <223> Synthetic
<400> 220
   cagggcatta gaaatgat 18
<210> 221
   <211> 6
   <212> PRT
   <213> Artificial Sequence
<220>
   <223> Synthetic
<400> 221
   Gln Gly Ile Arg Asn Asp
    1               5
<210> 222
   <211> 9
   <212> DNA
   <213> Artificial Sequence
<220>
   <223> Synthetic
<400> 222
   gctgcatcc 9
<210> 223
   <211> 3
   <212> PRT
   <213> Artificial Sequence
<220>
   <223> Synthetic
<400> 223
   Ala Ala Ser
   1
<210> 224
   <211> 27
   <212> DNA
   <213> Artificial Sequence
<220>
   <223> Synthetic
<400> 224
   ctacaagata acacttatcc gtacact 27
<210> 225
   <211> 9
   <212> PRT
   <213> Artificial Sequence
<220>
   <223> Synthetic
<400> 225
   Leu Gln Asp Asn Thr Tyr Pro Tyr Thr
   1 5
<210> 226
   <211> 351
   <212> DNA
   <213> Artificial Sequence
<220>
   <223> Synthetic
<400> 226
   caggttcagt tggtgcagtc tggacctgag gtgaagaagc ctggggcctc agtgaaggtc 60
   tcctgcaagg cttctggtta cacctttacc aactatggta tcagttgggt gcgacaggcc 120
   cctggacaag ggcttgagtg gatgggatgg atcagcgttt acaatggtaa aacaaactat 180
   gcacagaagt tccagggcag agtcaccatg accacagaca catccacgag cacagcctac 240
   atggacctga ggagcctgag atctgacgac acggccgtat attactgtgc gagagggagt 300
   ggctacgatt ttgactactg gggccaggga accctggtca ccgtctcctc a          351
<210> 227
   <211> 117
   <212> PRT
   <213> Artificial Sequence
<220>
   <223> Synthetic
<400> 227
   Gln Val Gln Leu Val Gln Ser Gly Pro Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Lys Thr Asn Tyr Ala Gln Lys Phe
       50                  55                  60
   Gln Gly Arg Val Thr Met Thr Thr Asp Thr Ser Thr Ser Thr Ala Tyr
   65                  70                  75                  80
   Met Asp Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 228
   <211> 24
   <212> DNA
   <213> Artificial Sequence
<220>
   <223> Synthetic
<400> 228
   ggttacacct ttaccaacta tggt 24
<210> 229
   <211> 8
   <212> PRT
   <213> Artificial Sequence
<220>
   <223> Synthetic
<400> 229
   Gly Tyr Thr Phe Thr Asn Tyr Gly
   1 5
<210> 230
   <211> 24
   <212> DNA
   <213> Artificial Sequence
<220>
   <223> Synthetic
<400> 230
   atcagcgttt acaatggtaa aaca 24
<210> 231
   <211> 8
   <212> PRT
   <213> Artificial Sequence
<220>
   <223> Synthetic
<400> 231
   Ile Ser Val Tyr Asn Gly Lys Thr
   1 5
<210> 232
   <211> 30
   <212> DNA
   <213> Artificial Sequence
<220>
   <223> Synthetic
<400> 232
   gcgagaggga gtggctacga ttttgactac 30
<210> 233
   <211> 10
   <212> PRT
   <213> Artificial Sequence
<220>
   <223> Synthetic
<400> 233
   Ala Arg Gly Ser Gly Tyr Asp Phe Asp Tyr
   1 5 10
<210> 234
   <211> 321
   <212> DNA
   <213> Artificial Sequence
<220>
   <223> Synthetic
<400> 234
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctttgct tcatccagtt tacaaagtgg ggtcccatca 180
   aggttcagcg gcagtggatc tggcacagat ttcactctca ccatccgcag cctgcagcct 240
   gaagattttg cagcttatta ctgtctacaa gattataatt acccgtacac ttttggccag 300
   gggaccaagc tggagatcaa a                                           321
<210> 235
   <211> 107
   <212> PRT
   <213> Artificial Sequence
<220>
   <223> Synthetic
<400> 235
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Phe Ala Ser Ser Ser Leu Gln Ser Gly Val Pro Ser Arg Phe Ser Gly
       50                  55                  60
   Ser Gly Ser Gly Thr Asp Phe Thr Leu Thr Ile Arg Ser Leu Gln Pro
   65                  70                  75                  80
   Glu Asp Phe Ala Ala Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 236
   <211> 18
   <212> DNA
   <213> Artificial Sequence
<220>
   <223> Synthetic
<400> 236
   cagggcatta gaaatgct 18
<210> 237
   <211> 6
   <212> PRT
   <213> Artificial Sequence
<220>
   <223> Synthetic
<400> 237
   Gln Gly Ile Arg Asn Ala
   1 5
<210> 238
   <211> 9
   <212> DNA
   <213> Artificial Sequence
<220>
   <223> Synthetic
<400> 238
   gcttcatcc 9
<210> 239
   <211> 3
   <212> PRT
   <213> Artificial Sequence
<220>
   <223> Synthetic
<400> 239
   Ala Ser Ser
   1
<210> 240
   <211> 27
   <212> DNA
   <213> Artificial Sequence
<220>
   <223> Synthetic
<400> 240
   ctacaagatt ataattaccc gtacact 27
<210> 241
   <211> 9
   <212> PRT
   <213> Artificial Sequence
<220>
   <223> Synthetic
<400> 241
   Leu Gln Asp Tyr Asn Tyr Pro Tyr Thr
   1 5
<210> 242
   <211> 351
   <212> DNA
   <213> Artificial Sequence
<220>
   <223> Synthetic
<400> 242
   caggttcagc tggtgcagtc tggagctgag gtgaagaagc ctggggcctc agtgaaggtc 60
   tcctgcaagg cttctggtta cacctttacc agctatggta tcagctgggt gcgacaggcc 120
   cctggacaag ggcttgagtg gatgggatgg atcagtgttt acaatggtaa cacaaactat 180
   gcacagaagc tccagggcag agtcaccatg accacagaca catccacgag cacagcctac 240
   atggacctga ggagcctgag atctgacgac gcggccgtgt attactgtgc gagagggagt 300
   ggctacgatt ttgactactg gggccaggga accctggtca ccgtctcctc a          351
<210> 243
   <211> 117
   <212> PRT
   <213> Artificial Sequence
<220>
   <223> Synthetic
<400> 243
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Ser Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Asp Leu Arg Ser Leu Arg Ser Asp Asp Ala Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 244
   <211> 24
   <212> DNA
   <213> Artificial Sequence
<220>
   <223> Synthetic
<400> 244
   ggttacacct ttaccagcta tggt 24
<210> 245
   <211> 8
   <212> PRT
   <213> Artificial Sequence
<220>
   <223> Synthetic
<400> 245
   Gly Tyr Thr Phe Thr Ser Tyr Gly
   1 5
<210> 246
   <211> 24
   <212> DNA
   <213> Artificial Sequence
<220>
   <223> Synthetic
<400> 246
   atcagtgttt acaatggtaa caca 24
<210> 247
   <211> 8
   <212> PRT
   <213> Artificial Sequence
<220>
   <223> Synthetic
<400> 247
   Ile Ser Val Tyr Asn Gly Asn Thr
   1 5
<210> 248
   <211> 30
   <212> DNA
   <213> Artificial Sequence
<220>
   <223> Synthetic
<400> 248
   gcgagaggga gtggctacga ttttgactac 30
<210> 249
   <211> 10
   <212> PRT
   <213> Artificial Sequence
<220>
   <223> Synthetic
<400> 249
   Ala Arg Gly Ser Gly Tyr Asp Phe Asp Tyr
   1               5                   10
<210> 250
   <211> 321
   <212> DNA
   <213> Artificial Sequence
<220>
   <223> Synthetic
<400> 250
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaaactcct gatctatgct gcatccagtt tacaaagtgg ggtcccatca 180
   aggttcagcg gcagtggatc tggtacagat ttcactctca ccatcagcag cctgcagtct 240
   gaagattttg caacttatta ctgtctacaa gattacaatt acccgtacac ttttggccag 300
   gggaccaagc tggagatcaa a                                           321
<210> 251
   <211> 107
   <212> PRT
   <213> Artificial Sequence
<220>
   <223> Synthetic
<400> 251
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Ser
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 252
   <211> 18
   <212> DNA
   <213> Artificial Sequence
<220>
   <223> Synthetic
<400> 252
   cagggcatta gaaatgct 18
<210> 253
   <211> 6
   <212> PRT
   <213> Artificial Sequence

<220>
   <223> Synthetic
<400> 253
   Gln Gly Ile Arg Asn Ala
   1 5
<210> 254
   <211> 9
   <212> DNA
   <213> Artificial Sequence
<220>
   <223> Synthetic
<400> 254
   gctgcatcc 9
<210> 255
   <211> 3
   <212> PRT
   <213> Artificial Sequence
<220>
   <223> Synthetic
<400> 255
   Ala Ala Ser
   1
<210> 256
   <211> 27
   <212> DNA
   <213> Artificial Sequence
<220>
   <223> Synthetic
<400> 256
   ctacaagatt acaattaccc gtacact 27
<210> 257
   <211> 9
   <212> PRT
   <213> Artificial Sequence
<220>
   <223> Synthetic
<400> 257
   Leu Gln Asp Tyr Asn Tyr Pro Tyr Thr
   1 5
<210> 258
   <211> 357
   <212> DNA
   <213> Artificial-Sequence
<220>
   <223> Synthetic
<400> 258
   caggtgcgac tggtggagtc tgggggaggc gtggtccagc ctggagggtc cctgagactc 60
   tcctgtgtag cctctggatt caccttcaga agctatggca tgcactgggt ccgccaggct 120
   ccaggcaggg gactggagtg ggtggcagtt atatcatatg atggcagtaa aacattctac 180
   gtagattccg tgaaggtccg attcaccatc tccagagaca attccaagaa cacgctttat 240
   ttggaaatga acagcctgag agttgacgac acggctgtgt atttctgtgt gaaagaagga 300
   agaagtggga gttggttcga cccctggggc cagggaaccc tggtcgccgt ctcctca    357
<210> 259
   <211> 119
   <212> PRT
   <213> Artificial Sequence
<220>
   <223> Synthetic
<400> 259
   Gln Val Arg Leu Val Glu Ser Gly Gly Gly Val Val Gln Pro Gly Gly
   1               5                   10                  15
   Ser Leu Arg Leu Ser Cys Val Ala Ser Gly Phe Thr Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Thr Phe Tyr Val Asp Ser Val
       50                  55                  60
   Lys Val Arg Phe Thr Ile Ser Arg Asp Asn Ser Lys Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Ala Val Ser Ser
           115
<210> 260
   <211> 24
   <212> DNA
   <213> Artificial Sequence
<220>
   <223> Synthetic
<400> 260
   ggattcacct tcagaagcta tggc 24
<210> 261
   <211> 8
   <212> PRT
   <213> Artificial Sequence
<220>
   <223> Synthetic
<400> 261
   Gly Phe Thr Phe Arg Ser Tyr Gly
   1 5
<210> 262
   <211> 24
   <212> DNA
   <213> Artificial Sequence
<220>
   <223> Synthetic
<400> 262
   atatcatatg atggcagtaa aaca 24
<210> 263
   <211> 8
   <212> PRT
   <213> Artificial Sequence
<220>
   <223> Synthetic
<400> 263
   Ile Ser Tyr Asp Gly Ser Lys Thr
   1 5
<210> 264
   <211> 36
   <212> DNA
   <213> Artificial Sequence
<220>
   <223> Synthetic
<400> 264
   gtgaaagaag gaagaagtgg gagttggttc gacccc 36
<210> 265
   <211> 12
   <212> PRT
   <213> Artificial Sequence
<220>
   <223> Synthetic
<400> 265
   Val Lys Glu Gly Arg Ser Gly Ser Trp Phe Asp Pro
    1              5                   10
<210> 266
   <211> 321
   <212> DNA
   <213> Artificial Sequence
<220>
   <223> Synthetic
<400> 266
   gacatccaga tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaactccct gatctttgct acatccagtt tacaaagtgg ggtcccatca 180
   aggttcagcg ccggtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa a                                           321
<210> 267
   <211> 107
   <212> PRT
   <213> Artificial Sequence
<220>
   <223> Synthetic
<400> 267
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Asn Ser Leu Ile
           35                  40                  45
   Phe Ala Thr Ser Ser Leu Gln Ser Gly Val Pro Ser Arg Phe Ser Ala
       50                  55                  60
   Gly Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 268
   <211> 18
   <212> DNA
   <213> Artificial Sequence
<220>
   <223> Synthetic
<400> 268
   caggccatta acaatttt 18
<210> 269
   <211> 6
   <212> PRT
   <213> Artificial Sequence
<220>
   <223> Synthetic
<400> 269
   Gln Ala Ile Asn Asn Phe
   1 5
<210> 270
   <211> 9
   <212> DNA
   <213> Artificial Sequence
<220>
   <223> Synthetic
<400> 270
   gctacatcc 9
<210> 271
   <211> 3
   <212> PRT
   <213> Artificial Sequence
<220>
   <223> Synthetic
<400> 271
   Ala Thr Ser
   1
<210> 272
   <211> 27
   <212> DNA
   <213> Artificial Sequence
<220>
   <223> Synthetic
<400> 272
   cagcagtata atagtcaccc gtggacg 27
<210> 273
   <211> 9
   <212> PRT
   <213> Artificial Sequence
<220>
   <223> Synthetic
<400> 273
   Gln Gln Tyr Asn Ser His Pro Trp Thr
   1               5
<210> 274
   <211> 357
   <212> DNA
   <213> Artificial Sequence
<220>
   <223> Synthetic
<400> 274
   caggtgcgac tggtggagtc tgggggaggc gtggtccagc ctggagggtc cctgagactc 60
   tcctgtactg cctctggatt catcttcaga agttatggca tgcactgggt ccgccaggct 120
   ccaggcaggg gactggagtg ggtggcagtc atatcatatg atggcagtaa aacattctac 180
   gtagattccg tgaaggtccg attcaccatc tccagagaca actccaagaa cacgctttat 240
   ttggagatga acagcctgag agttgacgac acggctgtat atttctgtgt gaaagaagga 300
   agaagtggga gttggttcga cccctggggc cagggaaccc tggtcgccgt ctcctca    357
<210> 275
   <211> 119
   <212> PRT
   <213> Artificial Sequence
<220>
   <223> Synthetic
<400> 275
   Gln Val Arg Leu Val Glu Ser Gly Gly Gly Val Val Gln Pro Gly Gly
   1               5                   10                  15
   Ser Leu Arg Leu Ser Cys Thr Ala Ser Gly Phe Ile Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Thr Phe Tyr Val Asp Ser Val
       50                  55                  60
   Lys Val Arg Phe Thr Ile Ser Arg Asp Asn Ser Lys Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Ala Val Ser Ser
           115
<210> 276
   <211> 24
   <212> DNA
   <213> Artificial Sequence
<220>
   <223> Synthetic
<400> 276
   ggattcatct tcagaagtta tggc 24
<210> 277
   <211> 8
   <212> PRT
   <213> Artificial Sequence
<220>
   <223> Synthetic
<400> 277
   Gly Phe Ile Phe Arg Ser Tyr Gly
   1 5
<210> 278
   <211> 24
   <212> DNA
   <213> Artificial Sequence
<220>
   <223> Synthetic
<400> 278
   atatcatatg atggcagtaa aaca 24
<210> 279
   <211> 8
   <212> PRT
   <213> Artificial Sequence
<220>
   <223> Synthetic
<400> 279
   Ile Ser Tyr Asp Gly Ser Lys Thr
   1 5
<210> 280
   <211> 36
   <212> DNA
   <213> Artificial Sequence
<220>
   <223> Synthetic
<400> 280
   gtgaaagaag gaagaagtgg gagttggttc gacccc 36
<210> 281
   <211> 12
   <212> PRT
   <213> Artificial Sequence
<220>
   <223> Synthetic
<400> 281
   Val Lys Glu Gly Arg Ser Gly Ser Trp Phe Asp Pro
   1 5 10
<210> 282
   <211> 321
   <212> DNA
   <213> Artificial Sequence
<220>
   <223> Synthetic
<400> 282
   gacatccaca tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcag a 321
<210> 283
   <211> 107
   <212> PRT
   <213> Artificial Sequence
<220>
   <223> Synthetic
<400> 283
   Asp Ile His Met Thr Gln Ser Pro Ser Ser Leu Ser Ala Ser Val Gly
   1 5 10 15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Arg
               100                 105
<210> 284
   <211> 18
   <212> DNA
   <213> Artificial Sequence
<220>
   <223> Synthetic
<400> 284
   caggccatta acaatttt 18
<210> 285
   <211> 6
   <212> PRT
   <213> Artificial Sequence
<220>
   <223> Synthetic
<400> 285
   Gln Ala Ile Asn Asn Phe
   1               5
<210> 286
   <211> 9
   <212> DNA
   <213> Artificial Sequence
<220>
   <223> Synthetic
<400> 286
   gctacatcc 9
<210> 287
   <211> 3
   <212> PRT
   <213> Artificial Sequence
<220>
   <223> Synthetic
<400> 287
   Ala Thr Ser
   1
<210> 288
   <211> 27
   <212> DNA
   <213> Artificial Sequence
<220>
   <223> Synthetic
<400> 288
   caacagtata atagtcaccc gtggacg 27
<210> 289
   <211> 9
   <212> PRT
   <213> Artificial Sequence
<220>
   <223> Synthetic
<400> 289
   Gln Gln Tyr Asn Ser His Pro Trp Thr
   1 5
<210> 290
   <211> 357
   <212> DNA
   <213> Artificial Sequence
<220>
   <223> Synthetic
<400> 290
   caggtgcgac tggtggagtc tgggggaggc gtggtccagc ctggagggtc cctgagactc 60
   tcctgtgctg cctctggatt catcttcaga agctatggca tgcactgggt ccgccaggct 120
   ccaggcaggg gactggagtg ggtggcagtc atatcatatg atggcagtaa aacattctac 180
   gtagattccg tgaaggtccg attcaccata tccagagaca actccaagaa cacgctttat 240
   ttggaaatga acagcctgag agttgacgac acggctgtat atttctgtgt gaaagaagga 300
   agaagtggga gttggttcga cccctggggc cagggaaccc tggtcgccgt ctcctca 357
<210> 291
   <211> 119
   <212> PRT
   <213> Artificial Sequence
<220>
   <223> Synthetic
<400> 291
   Gln Val Arg Leu Val Glu Ser Gly Gly Gly Val Val Gln Pro Gly Gly
   1               5                   10                  15
   Ser Leu Arg Leu Ser Cys Ala Ala Ser Gly Phe Ile Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Thr Phe Tyr Val Asp Ser Val
       50                  55                  60
   Lys Val Arg Phe Thr Ile Ser Arg Asp Asn Ser Lys Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Ala Val Ser Ser
           115
<210> 292
   <211> 24
   <212> DNA
   <213> Artificial Sequence
<220>
   <223> Synthetic
<400> 292
   ggattcatct tcagaagcta tggc 24
<210> 293
   <211> 8
   <212> PRT
   <213> Artificial Sequence
<220>
   <223> Synthetic
<400> 293
   Gly Phe Ile Phe Arg Ser Tyr Gly
   1               5
<210> 294
   <211> 24
   <212> DNA
   <213> Artificial Sequence
<220>
   <223> Synthetic
<400> 294
   atatcatatg atggcagtaa aaca 24
<210> 295
   <211> 8
   <212> PRT
   <213> Artificial Sequence
<220>
   <223> Synthetic
<400> 295
   Ile Ser Tyr Asp Gly Ser Lys Thr
   1 5
<210> 296
   <211> 36
   <212> DNA
   <213> Artificial Sequence
<220>
   <223> Synthetic
<400> 296
   gtgaaagaag gaagaagtgg gagttggttc gacccc 36
<210> 297
   <211> 12
   <212> PRT
   <213> Artificial Sequence
<220>
   <223> Synthetic
<400> 297
   Val Lys Glu Gly Arg Ser Gly Ser Trp Phe Asp Pro
   1 5 10
<210> 298
   <211> 321
   <212> DNA
   <213> Artificial Sequence
<220>
   <223> Synthetic
<400> 298
   gacatccaca tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa a 321
<210> 299
   <211> 107
   <212> PRT
   <213> Artificial Sequence
<220>
   <223> Synthetic
<400> 299
   Asp Ile His Met Thr Gln Ser Pro Ser Ser Leu Ser Ala Ser Val Gly
   1               5                   10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 300
   <211> 18
   <212> DNA
   <213> Artificial Sequence
<220>
   <223> Synthetic
<400> 300
   caggccatta acaatttt 18
<210> 301
   <211> 6
   <212> PRT
   <213> Artificial Sequence
<220>
   <223> Synthetic
<400> 301
   Gln Ala Ile Asn Asn Phe
   1               5
<210> 302
   <211> 9
   <212> DNA
   <213> Artificial Sequence
<220>
   <223> Synthetic
<400> 302
   gctacatcc 9
<210> 303
   <211> 3
   <212> PRT
   <213> Artificial Sequence
<220>
   <223> Synthetic
<400> 303
   Ala Thr Ser
   1
<210> 304
   <211> 27
   <212> DNA
   <213> Artificial Sequence
<220>
   <223> Synthetic
<400> 304
   caacagtata atagtcaccc gtggacg 27
<210> 305
   <211> 9
   <212> PRT
   <213> Artificial Sequence
<220>
   <223> Synthetic
<400> 305
   Gln Gln Tyr Asn Ser His Pro Trp Thr
   1 5
<210> 306
   <211> 354
   <212> DNA
   <213> Artificial Sequence
<220>
   <223> Synthetic
<400> 306
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg gtctggagtg ggtgacagtt atatcatatg atggaagtat taaatattat 180
   gtagactccg tgaagggccg attcaccatc tccagagaca attccaagaa cacgctgttt 240
   ctgcaaatga atagcctgcg agttgaggac acggctgtgt attactgtgc gaaggaaggt 300
   aggaagtacg gtatggacgt ctggggccaa gggaccacgg tcaccgtctc ctca 354
<210> 307
   <211> 118
   <212> PRT
   <213> Artificial Sequence
<220>
   <223> Synthetic
<400> 307
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Thr Val Ile Ser Tyr Asp Gly Ser Ile Lys Tyr Tyr Val Asp Ser Val
       50                  55                  60
   Lys Gly Arg Phe Thr Ile Ser Arg Asp Asn Ser Lys Asn Thr Leu Phe
   65                  70                  75                  80
   Leu Gln Met Asn Ser Leu Arg Val Glu Asp Thr Ala Val Tyr Tyr Cys
                   85                  90                  95
   Ala Lys Glu Gly Arg Lys Tyr Gly Met Asp Val Trp Gly Gln Gly Thr
               100                 105                 110
   Thr Val Thr Val Ser Ser
           115
<210> 308
   <211> 24
   <212> DNA
   <213> Artificial Sequence
<220>
   <223> Synthetic
<400> 308
   ggattcacct tcagtagcta tggc 24
<210> 309
   <211> 8
   <212> PRT
   <213> Artificial Sequence
<220>
   <223> Synthetic
<400> 309
   Gly Phe Thr Phe Ser Ser Tyr Gly
   1               5
<210> 310
   <211> 24
   <212> DNA
   <213> Artificial Sequence
<220>
   <223> Synthetic
<400> 310
   atatcatatg atggaagtat taaa 24
<210> 311
   <211> 8
   <212> PRT
   <213> Artificial Sequence
<220>
   <223> Synthetic
<400> 311
   Ile Ser Tyr Asp Gly Ser Ile Lys
   1 5
<210> 312
   <211> 33
   <212> DNA
   <213> Artificial Sequence
<220>
   <223> Synthetic
<400> 312
   gcgaaggaag gtaggaagta cggtatggac gtc 33
<210> 313
   <211> 11
   <212> PRT
   <213> Artificial Sequence
<220>
   <223> Synthetic
<400> 313
   Ala Lys Glu Gly Arg Lys Tyr Gly Met Asp Val
   1 5 10
<210> 314
   <211> 321
   <212> DNA
   <213> Artificial Sequence
<220>
   <223> Synthetic
<400> 314
   gacatccaga tgacccagtc tccttccacc ctgtctgcat ctgtaggaga cagagtcacc 60
   atcacttgcc gggccagtca gattattagt agcttattgg tctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatcatagtt acatgtacac ttttggccag 300
   gggaccaagc tggagatcaa a                                           321
<210> 315
   <211> 107
   <212> PRT
   <213> Artificial Sequence
<220>
   <223> Synthetic
<400> 315
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ile Ile Ser Ser Leu
               20                  25                  30
   Leu Val Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr His Ser Tyr Met Tyr
                   85                  90                  95
   Thr Phe Gly Gln Gly Thr Lys Leu Glu Ile Lys
               100                 105
<210> 316
   <211> 18
   <212> DNA
   <213> Artificial Sequence
<220>
   <223> Synthetic
<400> 316
   cagattatta gtagctta 18
<210> 317
   <211> 6
   <212> PRT
   <213> Artificial Sequence
<220>
   <223> Synthetic
<400> 317
   Gln Ile Ile Ser Ser Leu
    1               5
<210> 318
   <211> 9
   <212> DNA
   <213> Artificial Sequence
<220>
   <223> Synthetic
<400> 318
   aaggcgtct 9
<210> 319
   <211> 3
   <212> PRT
   <213> Artificial Sequence
<220>
   <223> Synthetic
<400> 319
   Lys Ala Ser
   1
<210> 320
   <211> 27
   <212> DNA
   <213> Artificial Sequence
<220>
   <223> Synthetic
<400> 320
   caacagtatc atagttacat gtacact 27
<210> 321
   <211> 9
   <212> PRT
   <213> Artificial Sequence
<220>
   <223> Synthetic
<400> 321
   Gln Gln Tyr His Ser Tyr Met Tyr Thr
   1 5
<210> 322
   <211> 357
   <212> DNA
   <213> Artificial Sequence
<220>
   <223> Synthetic
<400> 322
   caggtgcagc tggtggagtc tgggggaggc gtggtccagc ctgggaggtc cctgagactc 60
   tcctgtacag cctctggatt caccttcagt agctttggca tgcactgggt ccgccaggct 120
   ccaggcaggg gactggagtg ggtggcagtt atatcatatg atggcagtaa aaaatactac 180
   gcagactccg tgaaggtccg attcaccatc tccagagaca actccaagaa cacgctttat 240
   ctgcaaatga acagcctgag agttgacgac acggctgtgt atttctgtgc gaaagaagga 300
   agaactggga attggttcga cccctggggc caggggaccc tggtcgccgt ctcctca 357
<210> 323
   <211> 119
   <212> PRT
   <213> Artificial Sequence
<220>
   <223> Synthetic
<400> 323
   Gln Val Gln Leu Val Glu Ser Gly Gly Gly Val Val Gln Pro Gly Arg
    1               5                  10                  15
   Ser Leu Arg Leu Ser Cys Thr Ala Ser Gly Phe Thr Phe Ser Ser Phe
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Lys Tyr Tyr Ala Asp Ser Val
       50                  55                  60
   Lys Val Arg Phe Thr Ile Ser Arg Asp Asn Ser Lys Asn Thr Leu Tyr
   65                  70                  75                  80
   Leu Gln Met Asn Ser Leu Arg Val Asp Asp Thr Ala Val Tyr Phe Cys
                   85                  90                  95
   Ala Lys Glu Gly Arg Thr Gly Asn Trp Phe Asp Pro Trp Gly Gln Gly
               100                 105                 110
   Thr Leu Val Ala Val Ser Ser
           115
<210> 324
   <211> 24
   <212> DNA
   <213> Artificial Sequence
<220>
   <223> Synthetic
<400> 324
   ggattcacct tcagtagctt tggc 24
<210> 325
   <211> 8
   <212> PRT
   <213> Artificial Sequence
<220>
   <223> Synthetic
<400> 325
   Gly Phe Thr Phe Ser Ser Phe Gly
    1               5
<210> 326
   <211> 24
   <212> DNA
   <213> Artificial Sequence
<220>
   <223> Synthetic
<400> 326
   atatcatatg atggcagtaa aaaa 24
<210> 327
   <211> 8
   <212> PRT
   <213> Artificial Sequence
<220>
   <223> Synthetic
<400> 327
   Ile Ser Tyr Asp Gly Ser Lys Lys
    1               5
<210> 328
   <211> 36
   <212> DNA
   <213> Artificial Sequence
<220>
   <223> Synthetic
<400> 328
   gcgaaagaag gaagaactgg gaattggttc gacccc 36
<210> 329
   <211> 12
   <212> PRT
   <213> Artificial Sequence
<220>
   <223> Synthetic
<400> 329
   Ala Lys Glu Gly Arg Thr Gly Asn Trp Phe Asp Pro
    1               5                  10
<210> 330
   <211> 321
   <212> DNA
   <213> Artificial Sequence
<220>
   <223> Synthetic
<400> 330
   gacatccaga tgacccagtc tccatcctca ctgtctgcat ctgtaggaga cagagtcacc 60
   atcacttgtc gggcgagtca ggccattaac aattatttag cctggtttca gcagaaacca 120
   gggaaaaccc ctaagtccct gatctttgct acatccagtt tacaaagtgg ggtcccatca 180
   aggttcagcg gcactggatc tgggacagac ttcactctca ccatcagcag cctgcagcct 240
   gaagattttg caacttatta ctgccaacag tataatagtc acccgtggac gttcggccaa 300
   gggaccaagg tggaaatcag a 321
<210> 331
   <211> 107
   <212> PRT
   <213> Artificial Sequence
<220>
   <223> Synthetic
<400> 331
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Ala Ile Asn Asn Tyr
   20 25 30
   Leu Ala Trp Phe Gln Gln Lys Pro Gly Lys Thr Pro Lys Ser Leu Ile
   35 40 45
   Phe Ala Thr Ser Ser Leu Gln Ser Gly Val Pro Ser Arg Phe Ser Gly
   50 55 60
   Thr Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser His Pro Trp
   85 90 95
   Thr Phe Gly Gln Gly Thr Lys Val Glu Ile Arg
   100 105
<210> 332
   <211> 18
   <212> DNA
   <213> Artificial Sequence
<220>
   <223> Synthetic
<400> 332
   caggccatta acaattat 18
<210> 333
   <211> 6
   <212> PRT
   <213> Artificial Sequence
<220>
   <223> Synthetic
<400> 333
   Gln Ala Ile Asn Asn Tyr
   1 5
<210> 334
   <211> 9
   <212> DNA
   <213> Artificial Sequence
<220>
   <223> Synthetic
<400> 334
   gctacatcc 9
<210> 335
   <211> 3
   <212> PRT
   <213> Artificial Sequence
<220>
   <223> Synthetic
<400> 335
   Ala Thr Ser
   1
<210> 336
   <211> 27
   <212> DNA
   <213> Artificial Sequence
<220>
   <223> Synthetic
<400> 336
   caacagtata atagtcaccc gtggacg 27
<210> 337
   <211> 9
   <212> PRT
   <213> Artificial Sequence
<220>
   <223> Synthetic
<400> 337
   Gln Gln Tyr Asn Ser His Pro Trp Thr
   1 5
<210> 338
   <211> 357
   <212> DNA
   <213> Artificial Sequence
<220>
   <223> Synthetic
<400> 338
   caggtgcgac tggtggagtc tgggggaggc gtggtccagc ctggaaggtc cctgagactc 60
   tcctgtgtag cctctggatt caccttcaga agttatggca tgcactgggt ccgccaggct 120
   ccaggcaggg gactggagtg ggtggcagtc atatcatatg atggcagtca aacattctat 180
   gtagattccg tgaaggtccg attcaccatc tccagagaca actccaacaa cacgctttat 240
   ttggaaatga acagcctgag agttgacgac acggctgttt atttctgtgt gaaagaagga 300
   agaagtggga gttggttcga cccctggggc cagggaaccc tggtcgccgt ctcctca    357
<210> 339
   <211> 119
   <212> PRT
   <213> Artificial Sequence
<220>
   <223> Synthetic

<400> 339
   Gln Val Arg Leu Val Glu Ser Gly Gly Gly Val Val Gln Pro Gly Arg
   1               5                   10                  15
   Ser Leu Arg Leu Ser Cys Val Ala Ser Gly Phe Thr Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Gln Thr Phe Tyr Val Asp Ser Val
       50                  55                  60
   Lys Val Arg Phe Thr Ile Ser Arg Asp Asn Ser Asn Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Ala Val Ser Ser
           115
<210> 340
   <211> 24
   <212> DNA
   <213> Artificial Sequence
<220>
   <223> Synthetic
<400> 340
   ggattcacct tcagaagtta tggc 24
<210> 341
   <211> 8
   <212> PRT
   <213> Artificial Sequence
<220>
   <223> Synthetic
<400> 341
   Gly Phe Thr Phe Arg Ser Tyr Gly
   1 5
<210> 342
   <211> 24
   <212> DNA
   <213> Artificial Sequence
<220>
   <223> Synthetic
<400> 342
   atatcatatg atggcagtca aaca 24
<210> 343
   <211> 8
   <212> PRT
   <213> Artificial Sequence
<220>
   <223> Synthetic
<400> 343
   Ile Ser Tyr Asp Gly Ser Gln Thr
   1 5
<210> 344
   <211> 36
   <212> DNA
   <213> Artificial Sequence
<220>
   <223> Synthetic
<400> 344
   gtgaaagaag gaagaagtgg gagttggttc gacccc 36
<210> 345
   <211> 12
   <212> PRT
   <213> Artificial Sequence
<220>
   <223> Synthetic
<400> 345
   Val Lys Glu Gly Arg Ser Gly Ser Trp Phe Asp Pro
   1 5 10
<210> 346
   <211> 321
   <212> DNA
   <213> Artificial Sequence
<220>
   <223> Synthetic
<400> 346
   gacatccaca tgacccagtc tccatcctca ctgtctgcgt ctgttggaga cagagtcacc 60
   atcacttgtc gggcgagtca ggccattaat aattatttag cctggtttca gcagaaacca 120
   gggagaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa a                                           321
<210> 347
   <211> 107
   <212> PRT
   <213> Artificial Sequence
<220>
   <223> Synthetic
<400> 347
   Asp Ile His Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Tyr
               20                  25                  30
   Leu Ala Trp Phe Gln Gln Lys Pro Gly Arg Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 348
   <211> 18
   <212> DNA
   <213> Artificial Sequence
<220>
   <223> Synthetic
<400> 348
   caggccatta ataattat 18
<210> 349
   <211> 6
   <212> PRT
   <213> Artificial Sequence
<220>
   <223> Synthetic
<400> 349
   Gln Ala Ile Asn Asn Tyr
   1 5
<210> 350
   <211> 9
   <212> DNA
   <213> Artificial Sequence
<220>
   <223> Synthetic
<400> 350
   gctacatcc 9
<210> 351
   <211> 3
   <212> PRT
   <213> Artificial Sequence
<220>
   <223> Synthetic
<400> 351
   Ala Thr Ser
   1
<210> 352
   <211> 27
   <212> DNA
   <213> Artificial Sequence
<220>
   <223> Synthetic
<400> 352
   caacagtata atagtcaccc gtggacg 27
<210> 353
   <211> 9
   <212> PRT
   <213> Artificial Sequence
<220>
   <223> Synthetic
<400> 353
   Gln Gln Tyr Asn Ser His Pro Trp Thr
   1 5
<210> 354
   <211> 354
   <212> DNA
   <213> Artificial Sequence
<220>
   <223> Synthetic
<400> 354
   caggtgcagt tggtggagtc agggggaggc gtggtccagc ctgggaggtc cctgagactc 60
   tcctgtgcag cctctggatt caccttcagt agttatgcca tgtactgggt ccgccaggct 120
   ccaggcaagg ggctggagtg ggtggcagtg atatcatatg atggaagtaa taaatactat 180
   acagactccg tgaagggccg attcaccgtc tccagagaca attccaaaaa cacgctgttt 240
   ctgcaaatga acagcctgag agctgaggac acggctctgt attactgtgc gaaagaaagt 300
   tcttcgtacg gtttggacgt ctggggtcaa gggaccacgg tcaccgtctc ctca 354
<210> 355
   <211> 118
   <212> PRT
   <213> Artificial Sequence
<220>
   <223> Synthetic
<400> 355
   Gln Val Gln Leu Val Glu Ser Gly Gly Gly Val Val Gln Pro Gly Arg
   1 5 10 15
   Ser Leu Arg Leu Ser Cys Ala Ala Ser Gly Phe Thr Phe Ser Ser Tyr
   20 25 30
   Ala Met Tyr Trp Val Arg Gln Ala Pro Gly Lys Gly Leu Glu Trp Val
   35 40 45
   Ala Val Ile Ser Tyr Asp Gly Ser Asn Lys Tyr Tyr Thr Asp Ser Val
   50 55 60
   Lys Gly Arg Phe Thr Val Ser Arg Asp Asn Ser Lys Asn Thr Leu Phe
   65 70 75 80
   Leu Gln Met Asn Ser Leu Arg Ala Glu Asp Thr Ala Leu Tyr Tyr Cys
   85 90 95
   Ala Lys Glu Ser Ser Ser Tyr Gly Leu Asp Val Trp Gly Gln Gly Thr
   100 105 110
   Thr Val Thr Val Ser Ser
   115
<210> 356
   <211> 24
   <212> DNA
   <213> Artificial Sequence
<220>
   <223> Synthetic
<400> 356
   ggattcacct tcagtagtta tgcc 24
<210> 357
   <211> 8
   <212> PRT
   <213> Artificial Sequence
<220>
   <223> Synthetic
<400> 357
   Gly Phe Thr Phe Ser Ser Tyr Ala
   1 5
<210> 358
   <211> 24
   <212> DNA
   <213> Artificial Sequence
<220>
   <223> Synthetic
<400> 358
   atatcatatg atggaagtaa taaa 24
<210> 359
   <211> 8
   <212> PRT
   <213> Artificial Sequence
<220>
   <223> Synthetic
<400> 359
   Ile Ser Tyr Asp Gly Ser Asn Lys
   1 5
<210> 360
   <211> 33
   <212> DNA
   <213> Artificial Sequence
<220>
   <223> Synthetic
<400> 360
   gcgaaagaaa gttcttcgta cggtttggac gtc 33
<210> 361
   <211> 11
   <212> PRT
   <213> Artificial Sequence
<220>
   <223> Synthetic
<400> 361
   Ala Lys Glu Ser Ser Ser Tyr Gly Leu Asp Val
   1 5 10
<210> 362
   <211> 321
   <212> DNA
   <213> Artificial Sequence
<220>
   <223> Synthetic
<400> 362
   gacatccaga tgacccagtc tccttccacc ctgtctgcat ctgtaggaga cagagtcgcc 60
   atcacttgcc gggccagtca gagtattagt aactggttgg cctggtatca gcagaaacca 120
   gggaaagccc ctaaactcct gatctctaag gcgtctagtt taaaaagtgg ggtcccatca 180
   aggttcagcg gcagtggatc tgggacagaa ttcactctca ccatcagcag cctgcagcct 240
   gatgattttg cagcttatta ctgccaacag tataatagtt attcgtacac ttttggccag 300
   gggaccaagc tggagataaa a 321
<210> 363
   <211> 107
   <212> PRT
   <213> Artificial Sequence
<220>
   <223> Synthetic
<400> 363
   Asp Ile Gln Met Thr Gln Ser Pro Ser Thr Leu Ser Ala Ser Val Gly
   1 5 10 15
   Asp Arg Val Ala Ile Thr Cys Arg Ala Ser Gln Ser Ile Ser Asn Trp
   20 25 30
   Leu Ala Trp Tyr Gln Gln Lys Pro Gly Lys Ala Pro Lys Leu Leu Ile
   35 40 45
   Ser Lys Ala Ser Ser Leu Lys Ser Gly Val Pro Ser Arg Phe Ser Gly
   50 55 60
   Ser Gly Ser Gly Thr Glu Phe Thr Leu Thr Ile Ser Ser Leu Gln Pro
   65 70 75 80
   Asp Asp Phe Ala Ala Tyr Tyr Cys Gln Gln Tyr Asn Ser Tyr Ser Tyr
   85 90 95
   Thr Phe Gly Gln Gly Thr Lys Leu Glu Ile Lys
   100 105
<210> 364
   <211> 18
   <212> DNA
   <213> Artificial Sequence
<220>
   <223> Synthetic
<400> 364
   cagagtatta gtaactgg 18
<210> 365
   <211> 6
   <212> PRT
   <213> Artificial Sequence
<220>
   <223> Synthetic
<400> 365
   Gln Ser Ile Ser Asn Trp
   1 5
<210> 366
   <211> 9
   <212> DNA
   <213> Artificial Sequence
<220>
   <223> Synthetic
<400> 366
   aaggcgtct 9
<210> 367
   <211> 3
   <212> PRT
   <213> Artificial Sequence
<220>
   <223> Synthetic
<400> 367
   Lys Ala Ser
   1
<210> 368
   <211> 27
   <212> DNA
   <213> Artificial Sequence
<220>
   <223> Synthetic
<400> 368
   caacagtata atagttattc gtacact 27
<210> 369
   <211> 9
   <212> PRT
   <213> Artificial Sequence
<220>
   <223> Synthetic
<400> 369
   Gln Gln Tyr Asn Ser Tyr Ser Tyr Thr
   1 5
<210> 370
   <211> 357
   <212> DNA
   <213> Artificial Sequence
<220>
   <223> Synthetic
<400> 370
   caggtccaac tggtggagtc tgggggaggc gtggtccagc ctggggggtc cctgagactc 60
   tcctgtgcag cctctggatt caccttcagt agctatggca tgcactgggt ccgccaggct 120
   ccaggcaggg gactggagtg ggtggcagtt ttatcatatg atggcagtaa agaattctac 180
   gcagactccg tgaaggtccg attcaccgtc tccagagaca actccaagaa cacgctttat 240
   ctgcaaatga gcagcctgag agttgaggac acggctatat atttctgtgc gaaagaagga 300
   agaactggga attggttcga cccctggggc cagggaaccc tggtcgccgt ctcctca 357
<210> 371
   <211> 119
   <212> PRT
   <213> Artificial Sequence
<220>
   <223> Synthetic
<400> 371
   Gln Val Gln Leu Val Glu Ser Gly Gly Gly Val Val Gln Pro Gly Gly
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Arg Gly Leu Glu Trp Val
           35                  40                  45
   Ala Val Leu Ser Tyr Asp Gly Ser Lys Glu Phe Tyr Ala Asp Ser Val
       50                  55                  60
   Lys Val Arg Phe Thr Val Ser Arg Asp Asn Ser Lys Asn Thr Leu Tyr
   65                  70                  75                  80
   Leu Gln Met Ser Ser Leu Arg Val Glu Asp Thr Ala Ile Tyr Phe Cys
                   85                  90                  95
   Ala Lys Glu Gly Arg Thr Gly Asn Trp Phe Asp Pro Trp Gly Gln Gly
               100                 105                 110
   Thr Leu Val Ala Val Ser Ser
           115
<210> 372
   <211> 24
   <212> DNA
   <213> Artificial Sequence
<220>
   <223> Synthetic
<400> 372
   ggattcacct tcagtagcta tggc 24
<210> 373
   <211> 8
   <212> PRT
   <213> Artificial Sequence
<220>
   <223> Synthetic
<400> 373
   Gly Phe Thr Phe Ser Ser Tyr Gly
   1 5
<210> 374
   <211> 24
   <212> DNA
   <213> Artificial Sequence
<220>
   <223> Synthetic
<400> 374
   ttatcatatg atggcagtaa agaa 24
<210> 375
   <211> 8
   <212> PRT
   <213> Artificial Sequence
<220>
   <223> Synthetic
<400> 375
   Leu Ser Tyr Asp Gly Ser Lys Glu
   1 5
<210> 376
   <211> 36
   <212> DNA
   <213> Artificial Sequence
<220>
   <223> Synthetic
<400> 376
   gcgaaagaag gaagaactgg gaattggttc gacccc 36
<210> 377
   <211> 12
   <212> PRT
   <213> Artificial Sequence
<220>
   <223> Synthetic
<400> 377
   Ala Lys Glu Gly Arg Thr Gly Asn Trp Phe Asp Pro
    1               5                  10
<210> 378
   <211> 321
   <212> DNA
   <213> Artificial Sequence
<220>
   <223> Synthetic
<400> 378
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   ggtaaaaccc ctaagtccct gatctttgct acatccagtt tacaaagtgg ggtcccatca 180
   aggttcagcg gcagtgaatc tgggacagac ttcactctca ccatcagcag cctgcagcct 240
   gaagattttg caacttatta ctgccaacag tataatagtc acccgtggac gttcggccaa 300
   gggaccaagg tggaaatcaa a                                           321
<210> 379
   <211> 107
   <212> PRT
   <213> Artificial Sequence
<220>
   <223> Synthetic
<400> 379
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Glu Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 380
   <211> 18
   <212> DNA
   <213> Artificial Sequence
<220>
   <223> Synthetic
<400> 380
   caggccatta acaatttt 18
<210> 381
   <211> 6
   <212> PRT
   <213> Artificial Sequence
<220>
   <223> Synthetic
<400> 381
   Gln Ala Ile Asn Asn Phe
   1 5
<210> 382
   <211> 9
   <212> DNA
   <213> Artificial Sequence
<220>
   <223> Synthetic
<400> 382
   gctacatcc 9
<210> 383
   <211> 3
   <212> PRT
   <213> Artificial Sequence
<220>
   <223> Synthetic
<400> 383
   Ala Thr Ser
   1
<210> 384
   <211> 27
   <212> DNA
   <213> Artificial Sequence
<220>
   <223> Synthetic
<400> 384
   caacagtata atagtcaccc gtggacg 27
<210> 385
   <211> 9
   <212> PRT
   <213> Artificial Sequence
<220>
   <223> Synthetic
<400> 385
   Gln Gln Tyr Asn Ser His Pro Trp Thr
    1               5
<210> 386
   <211> 352
   <212> DNA
   <213> Artificial Sequence
<220>
   <223> Synthetic
<400> 386
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa aacaaactat 180
   gcacagaagc tccagggcag agtcaccatg accacagaca catccacgag cacagcctac 240
   atggagctga ggagcctgag atctgacgac acggccgtgt attactgtgc gagaggtagt 300
   ggctacgatt tggactactg gggccaagga accctggtca ccgtctcctc ag         352
<210> 387
   <211> 117
   <212> PRT
   <213> Artificial Sequence
<220>
   <223> Synthetic
<400> 387
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Lys Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Leu Asp Tyr Trp Gly Gln Gly Thr Leu
               100                 105                 110
   Val Thr Val Ser Ser
           115
<210> 388
   <211> 322
   <212> DNA
   <213> Artificial Sequence
<220>
   <223> Synthetic
<400> 388
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gatttcaatt acccgtacac ttttggccag 300
   gggaccaagc tggagatcaa ac                                          322
<210> 389
   <211> 107
   <212> PRT
   <213> Artificial Sequence
<220>
   <223> Synthetic
<400> 389
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Ala
                   20                      25                      30
   Leu Gly Trp Tyr Gln Gln Lys Pro Gly Lys Ala Pro Lys Leu Leu Ile
               35                      40                      45
   Tyr Ala Ala Ser Ser Leu Gln Ser Gly Val Pro Ser Arg Phe Ser Gly
       50                      55                      60
   Ser Gly Ser Gly Thr Asp Phe Thr Leu Thr Ile Ser Ser Leu Gln Pro
   65                      70                      75                      80
   Glu Asp Phe Ala Thr Tyr Tyr Cys Leu Gln Asp Phe Asn Tyr Pro Tyr
                   85                      90                      95
   Thr Phe Gly Gln Gly Thr Lys Leu Glu Ile Lys
               100                     105
<210> 390
   <211> 352
   <212> DNA
   <213> Artificial Sequence
<220>
   <223> Synthetic
<400> 390
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa aacaaactat 180
   gcacagaagc tccagggcag agtcaccatg accacagaca catccacgac cacagcctac 240
   atggaaatga ggagcctgag atctgacgac acggccgttt attactgtgc gagaggtagt 300
   ggctacgatt tggactactg gggccaggga accctggtca ccgtctcctc ag         352
<210> 391
   <211> 117
   <212> PRT
   <213> Artificial Sequence
<220>
   <223> Synthetic
<400> 391
   Gln Val Gln Leu Val Gln Ser Gly Ala Glu Val Lys Lys Pro Gly Ala
    1               5                      10                      15
   Ser Val Lys Val Ser Cys Lys Ala Ser Gly Tyr Thr Phe Thr Asn Tyr
                   20                      25                      30
   Gly Ile Ser Trp Val Arg Gln Ala Pro Gly Gln Gly Leu Glu Trp Met
               35                      40                      45
   Gly Trp Ile Ser Val Tyr Asn Gly Lys Thr Asn Tyr Ala Gln Lys Leu
       50                      55                      60
   Gln Gly Arg Val Thr Met Thr Thr Asp Thr Ser Thr Thr Thr Ala Tyr
   65                      70                      75                      80
   Met Glu Met Arg Ser Leu Arg Ser Asp Asp Thr Ala Val Tyr Tyr Cys
                   85                      90                      95
   Ala Arg Gly Ser Gly Tyr Asp Leu Asp Tyr Trp Gly Gln Gly Thr Leu
               100                     105                     110
   Val Thr Val Ser Ser
               115
<210> 392
   <211> 322
   <212> DNA
   <213> Artificial Sequence
<220>
   <223> Synthetic
<400> 392
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gatttcaatt acccgtacac ttttggccag 300
   gggaccaagc tggagatcaa ac                                          322
<210> 393
   <211> 107
   <212> PRT
   <213> Artificial Sequence
<220>
   <223> Synthetic
<400> 393
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Phe Asn Tyr Pro Tyr
                   85                  90                  95
   Thr Phe Gly Gln Gly Thr Lys Leu Glu Ile Lys
               100                 105
<210> 394
   <211> 385
   <212> DNA
   <213> Artificial Sequence
<220>
   <223> Synthetic
<400> 394
   gaggtgcagc tggtggagtc tgggggaggc ttggtccagc ctggggggtc cctgagactc 60
   tcctgtgcag cctctggatt cacctttagt tccttctgga tgagctgggt ccgccaggct 120
   ccagggaagg ggctggagtg ggtggccaac ataaagcaag atggaagtga gaaatactat 180
   gtggactctg tgaagggccg attcaccatc tccagagaca acgccaagaa ctcactgtat 240
   ctgcaaatga acagcctgag agccgaggac acggctgtgt attactgtgc gagagatccc 300
   gggcgaacta tggttcgggg aggtataaga tattactatg gtatggacgt ctgggggcaa 360
   gggaccacgg tcaccgtctc ctcag 385
<210> 395
   <211> 128
   <212> PRT
   <213> Artificial Sequence
<220>
   <223> Synthetic
<400> 395
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Ser Phe
   20 25 30
   Trp Met Ser Trp Val Arg Gln Ala Pro Gly Lys Gly Leu Glu Trp Val
   35 40 45
   Ala Asn Ile Lys Gln Asp Gly Ser Glu Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Tyr Cys
   85 90 95
   Ala Arg Asp Pro Gly Arg Thr Met Val Arg Gly Gly Ile Arg Tyr Tyr
   100 105 110
   Tyr Gly Met Asp Val Trp Gly Gln Gly Thr Thr Val Thr Val Ser Ser
   115 120 125
<210> 396
   <211> 322
   <212> DNA
   <213> Artificial Sequence
<220>
   <223> Synthetic
<400> 396
   gacatccaga tgacccagtc tccatcttcc gtgtctgcat ctgtaggaga cagagtcacc 60
   atcacttgtc gggcgagtca gggtgttagt agctggttag cctggtatca gcagaaacca 120
   gggaaagccc ctaagctcct gatctatgct gcatccagtt tgcaaagtgg ggtcccatca 180
   aggttcagcg gcagtggatc tgggacagat ttcactctca ccatcagcag cctgcagcct 240
   gaagattttg caacttacta ttgtcaacag gctaacagtt tccctctcac tttcggcgga 300
   gggaccaagg tggagatcaa ac 322
<210> 397
   <211> 107
   <212> PRT
   <213> Artificial Sequence
<220>
   <223> Synthetic
<400> 397
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Gly Val Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ser Phe Pro Leu
                   85                  90                  95
   Thr Phe Gly Gly Gly Thr Lys Val Glu Ile Lys
               100                 105
<210> 398
   <211> 385
   <212> DNA
   <213> Artificial Sequence
<220>
   <223> Synthetic
<400> 398
   gaggtgcagc tggtggagtc tgggggaggc ttggtccagc ctggggggtc cctgagactc 60
   tcctgtgcag cctctggatt cacctttagt tccttctgga tgagctgggt ccgccaggct 120
   ccagggaagg ggctggagtg ggtggccaac ataaagcaag atggaagtga gaaatactat 180
   gtggactctg tgaagggccg attcaccatc tccagagaca acgccaagaa ctcactgtat 240
   ctgcaaatga acagcctgag agccgaggac acggctgtgt attactgtgc gagagatccc 300
   gggcgaacta tggttcgggg aggtataaga tattactatg gtatggacgt ctgggggcaa 360
   gggaccacgg tcaccgtctc ctcag                                       385
<210> 399
   <211> 128
   <212> PRT
   <213> Artificial Sequence
<220>
   <223> Synthetic
<400> 399
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Pro Gly Arg Thr Met Val Arg Gly Gly Ile Arg Tyr Tyr
               100                 105                 110
   Tyr Gly Met Asp Val Trp Gly Gln Gly Thr Thr Val Thr Val Ser Ser
           115                 120                 125
<210> 400
   <211> 322
   <212> DNA
   <213> Artificial Sequence
<220>
   <223> Synthetic
<400> 400
   gacatccaga tgacccagtc tccatcttcc gtgtctgcat ctgtaggaga cagagtcacc 60
   atcacttgtc gggcgagtca gggtgttagt agctggttag cctggtatca gcagaaacca 120
   gggaaagccc ctaagctcct gatctatgct gcatccagtt tgcaaagtgg ggtcccatca 180
   aggttcagcg gcagtggatc tgggacagat ttcactctca ccatcagcag cctgcagcct 240
   gaagattttg caacttacta ttgtcaacag gctaacagtt tccctctcac tttcggcgga 300
   gggaccaagg tggagatcaa ac                                          322
<210> 401
   <211> 107
   <212> PRT
   <213> Artificial Sequence
<220>
   <223> Synthetic
<400> 401
   Asp Ile Gln Met Thr Gln Ser Pro Ser Ser Val Ser Ala Ser Val Gly
    1               5                  10                  15
   Asp Arg Val Thr Ile Thr Cys Arg Ala Ser Gln Gly Val Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ser Phe Pro Leu
                   85                  90                  95
   Thr Phe Gly Gly Gly Thr Lys Val Glu Ile Lys
               100                 105
<210> 402
   <211> 367
   <212> DNA
   <213> Artificial Sequence
<220>
   <223> Synthetic
<400> 402
   gaggtgcagc tggtggagtc tgggggaggc ttggtccagc ctggggggtc cctgagactc 60
   tcctgtgcag cctctggatt cacctttagt agccattgga tgagctgggt ccgccaggct 120
   ccagggaagg ggctggagtg ggtggccaac ataaagcaag atggaagtga taaatactat 180
   gtggactctg tgaagggccg attcaccatc tccagagaca acgccaagaa ctcactgtat 240
   ctgcaaatga acagcctgag agccgaggac acggctgtgt attactgtgc gagagatcgg 300
   ggggttagac cacctcgtgg ggcttttgat atctggggcc aagggacaat ggtcaccgtc 360
   tcttcag                                                           367
<210> 403
   <211> 122
   <212> PRT
   <213> Artificial Sequence
<220>
   <223> Synthetic
<400> 403
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser His
               20                  25                  30
   Trp Met Ser Trp Val Arg Gln Ala Pro Gly Lys Gly Leu Glu Trp Val
           35                  40                  45
   Ala Asn Ile Lys Gln Asp Gly Ser Asp Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Gly Val Arg Pro Pro Arg Gly Ala Phe Asp Ile Trp
   100 105 110
   Gly Gln Gly Thr Met Val Thr Val Ser Ser
   115 120
<210> 404
   <211> 322
   <212> DNA
   <213> Artificial Sequence
<220>
   <223> Synthetic
<400> 404
   gacatccaga tgacccagtc tccatcttcc gtgtctgcat ctgtaggaga cagagtcacc 60
   atcacttgtc gggcgagtca gggtatcagc agctggttag cctggtatca gcagaaacca 120
   gggaaagccc ctaagctcct gatctatgct gcatccagtt tgcaaagtgg ggtcccatca 180
   aggttcagcg gcagtggatc tgggacagat ttcactctca ccatcagcag cctgcagcct 240
   gaagattttg caacttacta ttgtcaacag gctaacagtt tccctctcac tttcggcgga 300
   gggaccaagg tggagatcaa ac                                          322
<210> 405
   <211> 107
   <212> PRT
   <213> Artificial Sequence
<220>
   <223> Synthetic
<400> 405
   Asp Ile Gln Met Thr Gln Ser Pro Ser Ser Val Ser Ala Ser Val Gly
   1 5 10 15
   Asp Arg Val Thr Ile Thr Cys Arg Ala Ser Gln Gly Ile Ser Ser Trp
   20 25 30
   Leu Ala Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Ala Asn Ser Phe Pro Leu
   85 90 95
   Thr Phe Gly Gly Gly Thr Lys Val Glu Ile Lys
   100 105
<210> 406
   <211> 367
   <212> DNA
   <213> Artificial Sequence
<220>
   <223> Synthetic
<400> 406
   gaggtgcagc tggtggagtc tgggggaggc ttggtccagc cgggggggtc cctgagactc 60
   tcctgtgcag cctctggatt cacctttagt agccattgga tgaactgggt ccgccaggct 120
   ccagggaagg ggctggagtg ggtggccaac ataaagcaag atggaagtga taaatactat 180
   gtggactctg tgaagggccg attcaccatc tccagagaca acgccaagaa ctcactctat 240
   ctgcaattga acagcctgat agccgaggac acggctgtgt attactgtgc gagagatcgg 300
   ggggttagac cacctcgtgg ggcttttgat atctggggcc aagggacaat ggtcaccgtc 360
   tcttcag                                                           367
<210> 407
   <211> 122
   <212> PRT
   <213> Artificial Sequence
<220>
   <223> Synthetic
<400> 407
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser His
               20                  25                  30
   Trp Met Asn Trp Val Arg Gln Ala Pro Gly Lys Gly Leu Glu Trp Val
           35                  40                  45
   Ala Asn Ile Lys Gln Asp Gly Ser Asp Lys Tyr Tyr Val Asp Ser Val
       50                  55                  60
   Lys Gly Arg Phe Thr Ile Ser Arg Asp Asn Ala Lys Asn Ser Leu Tyr
   65                  70                  75                  80
   Leu Gln Leu Asn Ser Leu Ile Ala Glu Asp Thr Ala Val Tyr Tyr Cys
                   85                  90                  95
   Ala Arg Asp Arg Gly Val Arg Pro Pro Arg Gly Ala Phe Asp Ile Trp
               100                 105                 110
   Gly Gln Gly Thr Met Val Thr Val Ser Ser
           115                 120
<210> 408
   <211> 322
   <212> DNA
   <213> Artificial Sequence
<220>
   <223> Synthetic
<400> 408
   gacatccaga tgacccagtc tccatcttcc gtgtctgcat ctgtaggaga cagagtcacc 60
   atcacttgtc gggcgagtca gggtatcagc agctggttag cctggtatca gcagaaacca 120
   gggaaagccc ctaagctcct gatctatgct gcatccagtt ttcaaagtgg ggtcccatca 180
   aggttcagcg gcagtggatc tgggacagat ttcactctca ccatcagcag cctgcagcct 240
   gaagattttg caacttactt ttgtcaacag gctaacagtt tccctctcac tttcggcgga 300
   gggaccaagg tggagatcaa ac                                          322
<210> 409
   <211> 107
   <212> PRT
   <213> Artificial Sequence
<220>
   <223> Synthetic
<400> 409
   Asp Ile Gln Met Thr Gln Ser Pro Ser Ser Val Ser Ala Ser Val Gly
    1               5                  10                  15
   Asp Arg Val Thr Ile Thr Cys Arg Ala Ser Gln Gly Ile Ser Ser Trp
               20                  25                  30
   Leu Ala Trp Tyr Gln Gln Lys Pro Gly Lys Ala Pro Lys Leu Leu Ile
           35                  40                  45
   Tyr Ala Ala Ser Ser Phe Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Phe Cys Gln Gln Ala Asn Ser Phe Pro Leu
                   85                  90                  95
   Thr Phe Gly Gly Gly Thr Lys Val Glu Ile Lys
               100                 105
<210> 410
   <211> 355
   <212> DNA
   <213> Artificial Sequence
<220>
   <223> Synthetic
<400> 410
   gaagtgcagc tggtggagtc tgggggaggc ttggtacagc ctggcaggtc cctgagactc 60
   tcctgtgcag cctctggatt cacctttgat gattatgcca tgcactgggt ccggcaagct 120
   ccagggaagg gcctggagtg ggtctcaggt cttagtcgga caagtgtcag tataggctat 180
   gcggactctg tgaagggccg attcaccatc tccagagaca acgccaagaa ctccctgtat 240
   ctgcaaatga acagtctgag agctgaggac acggccttgt attactgtgc aaaatggggg 300
   acccgggggt attttgacta ctggggccaa ggaaccctgg tcaccgtctc ctcag      355
<210> 411
   <211> 118
   <212> PRT
   <213> Artificial Sequence
<220>
   <223> Synthetic
<400> 411
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Leu Ser Arg Thr Ser Val Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Trp Gly Thr Arg Gly Tyr Phe Asp Tyr Trp Gly Gln Gly Thr
               100                 105                 110
   Leu Val Thr Val Ser Ser
           115
<210> 412
   <211> 322
   <212> DNA
   <213> Artificial Sequence
<220>
   <223> Synthetic

<400> 412
   gacatccaga tgacccagtc tccatcttcc gtgtctgcat ctgtaggaga cagagtcacc 60
   atcacttgtc gggcgagtca ggatattagt atttggttag cctggtatca gcagaaacca 120
   gggaaagccc ctaagctcct gatctatgtt gcatccagtt tgcaaagtgg ggtcccatca 180
   aggttcagcg gcagtggatc tgggacagat ttcactctca ccatcagcag cctgcagcct 240
   gaagattttg caacttacta ttgtcaacag gctaacagtt tcccgatcac cttcggccaa 300
   gggacacgac tggagattaa ac                                          322
<210> 413
   <211> 107
   <212> PRT
   <213> Artificial Sequence
<220>
   <223> Synthetic
<400> 413
   Asp Ile Gln Met Thr Gln Ser Pro Ser Ser Val Ser Ala Ser Val Gly
    1               5                  10                  15
   Asp Arg Val Thr Ile Thr Cys Arg Ala Ser Gln Asp Ile Ser Ile Trp
               20                  25                  30
   Leu Ala Trp Tyr Gln Gln Lys Pro Gly Lys Ala Pro Lys Leu Leu Ile
           35                  40                  45
   Tyr Val Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ala Asn Ser Phe Pro Ile
                   85                  90                  95
   Thr Phe Gly Gln Gly Thr Arg Leu Glu Ile Lys
               100                 105
<210> 414
   <211> 355
   <212> DNA
   <213> Artificial Sequence
<220>
   <223> Synthetic
<400> 414
   gaagtgcagc tggtggagtc tgggggaggc ttggtacagc ctggcaggtc cctgagactc 60
   tcctgtgagg cctctggatt cacctttgat gattatgcca tgcactgggt ccggcaagct 120
   ccggggaagg gcctggaatg ggtctcaggt cttagtcgga caagtgtcag tataggctat 180
   gcggactctg tgaagggccg attcaccatc tccagagaca acgccaagaa ctccctttat 240
   ttggaaatga acagtctgag acctgaggac acggccttat attactgtgc aaaatggggg 300
   acccgggggt attttgacta ccggggccag ggaaccctgg tcaccgtctc ctcag      355
<210> 415
   <211> 118
   <212> PRT
   <213> Artificial Sequence
<220>
   <223> Synthetic
<400> 415
   Glu Val Gln Leu Val Glu Ser Gly Gly Gly Leu Val Gln Pro Gly Arg
    1               5                  10                  15
   Ser Leu Arg Leu Ser Cys Glu Ala Ser Gly Phe Thr Phe Asp Asp Tyr
                   20                      25                      30
   Ala Met His Trp Val Arg Gln Ala Pro Gly Lys Gly Leu Glu Trp Val
               35                      40                      45
   Ser Gly Leu Ser Arg Thr Ser Val Ser Ile Gly Tyr Ala Asp Ser Val
       50                      55                      60
   Lys Gly Arg Phe Thr Ile Ser Arg Asp Asn Ala Lys Asn Ser Leu Tyr
   65                      70                      75                      80
   Leu Glu Met Asn Ser Leu Arg Pro Glu Asp Thr Ala Leu Tyr Tyr Cys
                   85                      90                      95
   Ala Lys Trp Gly Thr Arg Gly Tyr Phe Asp Tyr Arg Gly Gln Gly Thr
               100                     105                     110
   Leu Val Thr Val Ser Ser
           115
<210> 416
   <211> 322
   <212> DNA
   <213> Artificial Sequence
<220>
   <223> Synthetic
<400> 416
   gacatccaga tgacccagtc tccatcttcc gtgtctgcat ctgtgggaga cagagtcacc 60
   atcacttgtc gggcgagtca ggatattagt atttggttag cctggtatca gcagagtcca 120
   gggaaagccc ctaaactcct gatcaatgtt gcatcccgtt tgcaaagtgg ggtcccatca 180
   aggttcagcg gcagtggatc tgggacagat ttcactctca ccatcaacag tctgcagcct 240
   gaagattttg taacttacta ttgtcaacag gctaacagtt tcccgatcac cttcggccaa 300
   gggacacgac tggagattaa ac                                          322
<210> 417
   <211> 107
   <212> PRT
   <213> Artificial Sequence
<220>
   <223> Synthetic
<400> 417
   Asp Ile Gln Met Thr Gln Ser Pro Ser Ser Val Ser Ala Ser Val Gly
   1               5                   10                  15
   Asp Arg Val Thr Ile Thr Cys Arg Ala Ser Gln Asp Ile Ser Ile Trp
               20                  25                  30
   Leu Ala Trp Tyr Gln Gln Ser Pro Gly Lys Ala Pro Lys Leu Leu Ile
           35                  40                  45
   Asn Val Ala Ser Arg Leu Gln Ser Gly Val Pro Ser Arg Phe Ser Gly
       50                  55                  60
   Ser Gly Ser Gly Thr Asp Phe Thr Leu Thr Ile Asn Ser Leu Gln Pro
   65                  70                  75                  80
   Glu Asp Phe Val Thr Tyr Tyr Cys Gln Gln Ala Asn Ser Phe Pro Ile
                   85                  90                  95
   Thr Phe Gly Gln Gly Thr Arg Leu Glu Ile Lys
               100                 105
<210> 418
   <211> 352
   <212> DNA
   <213> Artificial Sequence
<220>
   <223> Synthetic
<400> 418
   caggtgcagc tggtggagtc tgggggaggc ttggtcaagc ctggagggtc cctgagactc 60
   tcctgtgcag cctctggatt cacaatcagt gaccactaca tgagctggat ccgccaggct 120
   ccagggaagg ggctggagtg ggtttcatac attagtagta gtggtagtaa aatatactac 180
   gcagactctg tgaagggccg attcaccatc tccagggaca acgccaagaa ctcactgtat 240
   ctgcaaatga acagcctgag agccgaggac acggccgtgt attactgtgc gagaacacga 300
   cagctcgtgg gggactactg gggccaagga accctggtca ccgtctcctc ag        352
<210> 419
   <211> 117
   <212> PRT
   <213> Artificial Sequence
<220>
   <223> Synthetic
<400> 419
   Gln Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Ile Ser Asp His
               20                  25                  30
   Tyr Met Ser Trp Ile Arg Gln Ala Pro Gly Lys Gly Leu Glu Trp Val
           35                  40                  45
   Ser Tyr Ile Ser Ser Ser Gly Ser Lys Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Arg Gln Leu Val Gly Asp Tyr Trp Gly Gln Gly Thr Leu
               100                 105                 110
   Val Thr Val Ser Ser
           115
<210> 420
   <211> 322
   <212> DNA
   <213> Artificial Sequence
<220>
   <223> Synthetic
<400> 420
   gacatccagt tgacccagtc tccatccttc ctgtctgcat ctgtaggaga cagagtcacc 60
   atcacttgcc gggccagtca gggcattagc agttatttag cctggtatca gcaaaaacca 120
   gggaaagccc ctaagctcct gatctatgct gcatccactt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt acccgctcac tttcggcgga 300
   gggaccaagg tggagatcaa ac                                          322
<210> 421
   <211> 107
   <212> PRT
   <213> Artificial Sequence
<220>
   <223> Synthetic
<400> 421
   Asp Ile Gln Leu Thr Gln Ser Pro Ser Phe Leu Ser Ala Ser Val Gly
    1               5                  10                  15
   Asp Arg Val Thr Ile Thr Cys Arg Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Leu
                   85                  90                  95
   Thr Phe Gly Gly Gly Thr Lys Val Glu Ile Lys
               100                 105
<210> 422
   <211> 352
   <212> DNA
   <213> Artificial Sequence
<220>
   <223> Synthetic
<400> 422
   caggtgcagc tggtggagtc tgggggaggc ttggtcaagc ctggagggtc cctgagactc 60
   tcctgtgcag cctctggatt cacaatcagt gaccactaca tgagctggat ccgccaggct 120
   ccagggaagg ggctggagtg ggtttcatac attagtagta gtggtagtaa aatatactac 180
   gcagactctg tgaagggccg attcaccatc tccagggaca acgccaagaa ctcactgtat 240
   ctgcaaatga acagcctgag agccgaggac acggccgtgt attactgtgc gagaacacga 300
   cagctcgtgg gggactactg gggccaagga accctggtca ccgtctcctc ag         352
<210> 423
   <211> 117
   <212> PRT
   <213> Artificial Sequence
<220>
   <223> Synthetic
<400> 423
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Ile Ser Asp His
               20                  25                  30
   Tyr Met Ser Trp Ile Arg Gln Ala Pro Gly Lys Gly Leu Glu Trp Val
           35                  40                  45
   Ser Tyr Ile Ser Ser Ser Gly Ser Lys Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Thr Arg Gln Leu Val Gly Asp Tyr Trp Gly Gln Gly Thr Leu
               100                 105                 110
   Val Thr Val Ser Ser
           115
<210> 424
   <211> 322
   <212> DNA
   <213> Artificial Sequence
<220>
   <223> Synthetic
<400> 424
   gacatccagt tgacccagtc tccatccttc ctgtctgcat ctgtaggaga cagagtcacc 60
   atcacttgct gggccagtca gggcattagc agttatttag cctggtatca gcaaaaacca 120
   gggaaagccc ctaagctcct gatctttgct gcatccactt tgcaaagtgg ggtcccatca 180
   aggttcagcg gcagtggatc tgggacagaa ttcactctca caatcagcag cctgcagcct 240
   gaagattttg caacttatta ctgtcaacag cttaatagtt acccgctcac tttcggcgga 300
   gggaccaagg tggagatcaa ac                                          322
<210> 425
   <211> 107
   <212> PRT
   <213> Artificial Sequence
<220>
   <223> Synthetic
<400> 425
   Asp Ile Gln Leu Thr Gln Ser Pro Ser Phe Leu Ser Ala Ser Val Gly
   1               5                   10                  15
   Asp Arg Val Thr Ile Thr Cys Trp Ala Ser Gln Gly Ile Ser Ser Tyr
               20                  25                  30
   Leu Ala Trp Tyr Gln Gln Lys Pro Gly Lys Ala Pro Lys Leu Leu Ile
           35                  40                  45
   Phe Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys Gln Gln Leu Asn Ser Tyr Pro Leu
                   85                  90                  95
   Thr Phe Gly Gly Gly Thr Lys Val Glu Ile Lys
               100                 105
<210> 426
   <211> 373
   <212> DNA
   <213> Artificial Sequence
<220>
   <223> Synthetic
<400> 426
   gaagtgcagc tggtggagtc tgggggaggc ttggtacagc ctggcaggtc cctgagactc 60
   tcctgtgcag cctctggatt cacctttgat aattatgcca tgcactgggt ccggcaagct 120
   ccagggaagg gcctggagtg ggtctcaggt attaggtgga atagtggtag cataggctat 180
   gcggactctg tgaagggccg attcaccatc tccagagaca acgccaagaa ctccctgtat 240
   ctgcaaatga acagtctgag agctgaggac acggccttgt attactgtgc aaaagaaggt 300
   ggatatagtg gctaccgccc cggacccttc tttgactact ggggccaagg aaccctggtc 360
   accgtctcct cag                                                    373
<210> 427
   <211> 124
   <212> PRT
   <213> Artificial Sequence
<220>
   <223> Synthetic
<400> 427
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asn Tyr
               20                  25                  30
   Ala Met His Trp Val Arg Gln Ala Pro Gly Lys Gly Leu Glu Trp Val
           35                  40                  45
   Ser Gly Ile Arg Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Glu Gly Gly Tyr Ser Gly Tyr Arg Pro Gly Pro Phe Phe Asp
               100                 105                 110
   Tyr Trp Gly Gln Gly Thr Leu Val Thr Val Ser Ser
           115                 120
<210> 428
   <211> 322
   <212> DNA
   <213> Artificial Sequence
<220>
   <223> Synthetic
<400> 428
   gaaatagtga tgacgcagtc tccagccacc ctgtctgtgt ctccagggga aagagccacc 60
   ctctcctgca gggccagtca gagtgttaac tacaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggccgctcac tttcggcgga 300
   gggaccaagg tggagatcaa ac                                          322
<210> 429
   <211> 107
   <212> PRT
   <213> Artificial Sequence
<220>
   <223> Synthetic
<400> 429
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Asn Tyr Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
   100 105
<210> 430
   <211> 373
   <212> DNA
   <213> Artificial Sequence
<220>
   <223> Synthetic
<400> 430
   gaagtgcagc tggtggagtc tgggggaggc ttggtacagc ctggcaggtc cctgagactc 60
   tcctgtgcag cctctggatt cacctttgat aattatgcca tgcactgggt ccggcaagct 120
   ccagggaagg gcctggagtg ggtctcaggt attaggtgga atagtggtag cataggctat 180
   gcggactctg tgaagggccg attcaccatc tccagagaca acgccaagaa ctccctgtat 240
   ctgcaaatga atagtctgag agctgaggac acggccttgt attactgtgc aaaagaaggt 300
   ggatatagtg gctaccgccc cggacccttc tttgactact ggggccaggg aaccctggtc 360
   accgtctcct cag 373
<210> 431
   <211> 124
   <212> PRT
   <213> Artificial Sequence
<220>
   <223> Synthetic
<400> 431
   Glu Val Gln Leu Val Glu Ser Gly Gly Gly Leu Val Gln Pro Gly Arg
   1 5 10 15
   Ser Leu Arg Leu Ser Cys Ala Ala Ser Gly Phe Thr Phe Asp Asn Tyr
   20 25 30
   Ala Met His Trp Val Arg Gln Ala Pro Gly Lys Gly Leu Glu Trp Val
   35 40 45
   Ser Gly Ile Arg Trp Asn Ser Gly Ser Ile Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr Tyr Cys
   85 90 95
   Ala Lys Glu Gly Gly Tyr Ser Gly Tyr Arg Pro Gly Pro Phe Phe Asp
   100 105 110
   Tyr Trp Gly Gln Gly Thr Leu Val Thr Val Ser Ser
   115 120
<210> 432
   <211> 322
   <212> DNA
   <213> Artificial Sequence
<220>
   <223> Synthetic
<400> 432
   gaaatagtga tgacgcagtc tccagccacc ctgtctgtgt ctccagggga aagagccacc 60
   ctctcctgca gggccagtca gagtgttaac tacaacttag cctggtacca gcagaaacct 120
   ggccaggctc ccaggctcct catctatggt gcatccacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggccgctcac tttcggcgga 300
   gggaccaagg tggagatcaa ac                                          322
<210> 433
   <211> 107
   <212> PRT
   <213> Artificial Sequence
<220>
   <223> Synthetic
<400> 433
   Glu Ile Val Met Thr Gln Ser Pro Ala Thr Leu Ser Val Ser Pro Gly
   1               5                   10                  15
   Glu Arg Ala Thr Leu Ser Cys Arg Ala Ser Gln Ser Val Asn Tyr Asn
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
               100                 105
<210> 434
   <211> 352
   <212> DNA
   <213> Artificial Sequence
<220>
   <223> Synthetic
<400> 434
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtca cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactcctg gggccaagga accctggtca ccgtctcctc ag         352
<210> 435
   <211> 117
   <212> PRT
   <213> Artificial Sequence
<220>
   <223> Synthetic
<400> 435
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly His Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Ser Trp Gly Gln Gly Thr Leu
               100                 105                 110
   Val Thr Val Ser Ser
           115
<210> 436
   <211> 322
   <212> DNA
   <213> Artificial Sequence
<220>
   <223> Synthetic
<400> 436
   gccatccaga tgacccagtc tccatcctcc ctgtctgcat ctgtaggaga cagagtcacc 60
   atcacttgcc gggcaagtca ggccattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacgatt acccgtacac ttttggccag 300
   gggaccaagc tggagatcaa ac                                          322
<210> 437
   <211> 107
   <212> PRT
   <213> Artificial Sequence
<220>
   <223> Synthetic
<400> 437
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asp Tyr Pro Tyr
                   85                  90                  95
   Thr Phe Gly Gln Gly Thr Lys Leu Glu Ile Lys
               100                 105
<210> 438
   <211> 352
   <212> DNA
   <213> Artificial Sequence
<220>
   <223> Synthetic
<400> 438
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtca cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactcctg gggccaagga accctggtca ccgtctcctc ag         352
<210> 439
   <211> 117
   <212> PRT
   <213> Artificial Sequence
<220>
   <223> Synthetic
<400> 439
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly His Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Ser Trp Gly Gln Gly Thr Leu
               100                 105                 110
   Val Thr Val Ser Ser
           115
<210> 440
   <211> 322
   <212> DNA
   <213> Artificial Sequence
<220>
   <223> Synthetic
<400> 440
   gccatccaga tgacccagtc tccatcctcc ctgtctgcat ctgtaggaga cagagtcacc 60
   atcacttgcc gggcaagtca ggccattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacgatt acccgtacac ttttggccag 300
   gggaccaagc tggagatcaa ac                                          322
<210> 441
   <211> 107
   <212> PRT
   <213> Artificial Sequence
<220>
   <223> Synthetic
<400> 441
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Arg Asn Ala
                   20                      25                      30
   Leu Gly Trp Tyr Gln Gln Lys Pro Gly Lys Ala Pro Lys Leu Leu Ile
               35                      40                      45
   Tyr Ala Ala Ser Ser Leu Gln Ser Gly Val Pro Ser Arg Phe Ser Gly
       50                      55                      60
   Ser Gly Ser Gly Thr Asp Phe Thr Leu Thr Ile Ser Ser Leu Gln Pro
   65                      70                      75                      80
   Glu Asp Phe Ala Thr Tyr Tyr Cys Leu Gln Asp Tyr Asp Tyr Pro Tyr
                   85                      90                      95
   Thr Phe Gly Gln Gly Thr Lys Leu Glu Ile Lys
                   100                     105
<210> 442
   <211> 349
   <212> DNA
   <213> Artificial Sequence
<220>
   <223> Synthetic
<400> 442
   caggtgcagc tggtgcagtc tggggctgag gtgaagaagc ctggggcctc agtgaaggtc 60
   tcctgcaagg cttctagata caccttcacc agttatgata tcaactgggt gcgacaggcc 120
   actggacaag ggcttgagtg gatgggatgg atgaacccaa acagtggtaa cacaggctat 180
   gcacagaagt tccagggcag agtcaccatg accaggaaca cctccataag cacagcctac 240
   atggagctga gcagcctgag atctgaggac acggccgtgt attactgtgc gagagtacga 300
   cgcttttttg actactgggg ccaaggaacc ctggtcaccg tctcctcag             349
<210> 443
   <211> 116
   <212> PRT
   <213> Artificial Sequence
<220>
   <223> Synthetic
<400> 443
   Gln Val Gln Leu Val Gln Ser Gly Ala Glu Val Lys Lys Pro Gly Ala
   1               5                   10                  15
   Ser Val Lys Val Ser Cys Lys Ala Ser Arg Tyr Thr Phe Thr Ser Tyr
               20                  25                  30
   Asp Ile Asn Trp Val Arg Gln Ala Thr Gly Gln Gly Leu Glu Trp Met
           35                  40                  45
   Gly Trp Met Asn Pro Asn Ser Gly Asn Thr Gly Tyr Ala Gln Lys Phe
       50                  55                  60
   Gln Gly Arg Val Thr Met Thr Arg Asn Thr Ser Ile Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Val Arg Arg Phe Phe Asp Tyr Trp Gly Gln Gly Thr Leu Val
               100                 105                 110
   Thr Val Ser Ser
           115
<210> 444
   <211> 322
   <212> DNA
   <213> Artificial Sequence
<220>
   <223> Synthetic
<400> 444
   gacatccagt tgacccagtc tccatccttc ctgtctgcat ctgtaggaga cagagtcacc 60
   atcacttgcc gggccagtca gggcattatc agttatttag cctggtatca gcaaaaacca 120
   gggaaagccc ctaagctcct gatctatgct gcatccactt tgcaaagtgg ggtcccatca 180
   aggttcagcg gcagtggatc tgggacagaa ttcactctca caatcagcag cctgcagcct 240
   gaagattttg caacttatta ctgtcaccag cttaaaagtt acccgatcac cttcggccaa 300
   gggacacgac tggagattaa ac                                          322
<210> 445
   <211> 107
   <212> PRT
   <213> Artificial Sequence
<220>
   <223> Synthetic
<400> 445
   Asp Ile Gln Leu Thr Gln Ser Pro Ser Phe Leu Ser Ala Ser Val Gly
    1               5                  10                  15
   Asp Arg Val Thr Ile Thr Cys Arg Ala Ser Gln Gly Ile Ile Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys His Gln Leu Lys Ser Tyr Pro Ile
                   85                  90                  95
   Thr Phe Gly Gln Gly Thr Arg Leu Glu Ile Lys
               100                 105
<210> 446
   <211> 349
   <212> DNA
   <213> Artificial Sequence
<220>
   <223> Synthetic
<400> 446
   caggtgcagc tggtgcagtc tggggctgag gtgaagaagc ctggggcctc agtgaaggtc 60
   tcctgcaagg cttctagata caccttcacc agttatgata tcaactgggt gcgacaggcc 120
   actggacaag ggcttgagtg gatgggatgg atgaacccaa acagtggtaa cacaggctat 180
   gcacagaagt tccagggcag agtcaccatg accaggaaca cctccacaag cacagcctac 240
   atggagctga gcagcctgag atctgaggac acggccgtgt attactgtgc gagagtacga 300
   cgcttttttg actactgggg ccagggaacc ctggtcaccg tctcctcag            349
<210> 447
   <211> 116
   <212> PRT
   <213> Artificial Sequence
<220>
   <223> Synthetic
<400> 447
   Gln Val Gln Leu Val Gln Ser Gly Ala Glu Val Lys Lys Pro Gly Ala
   1               5                   10                  15
   Ser Val Lys Val Ser Cys Lys Ala Ser Arg Tyr Thr Phe Thr Ser Tyr
               20                  25                  30
   Asp Ile Asn Trp Val Arg Gln Ala Thr Gly Gln Gly Leu Glu Trp Met
           35                  40                  45
   Gly Trp Met Asn Pro Asn Ser Gly Asn Thr Gly Tyr Ala Gln Lys Phe
       50                  55                  60
   Gln Gly Arg Val Thr Met Thr Arg Asn Thr Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Val Arg Arg Phe Phe Asp Tyr Trp Gly Gln Gly Thr Leu Val
               100                 105                 110
   Thr Val Ser Ser
           115
<210> 448
   <211> 322
   <212> DNA
   <213> Artificial Sequence
<220>
   <223> Synthetic
<400> 448
   gacatccagt tgacccagtc tccatccttc ctgtctgcat ctgtaggaga cagagtcacc 60
   atcacttgcc gggccagtca gggcattatc agttatttag cctggtatca gcaaaaacca 120
   gggaaagccc ctaagctcct gatctatgct gcatccactt tgcaaagtgg ggtcccatca 180
   aggttcagcg gcagtggatc tgggacagaa ttcactctca caatcagcag cctgcagcct 240
   gaagattttg caacttatta ctgtcaccag cttaaaagtt acccgatcac cttcggccaa 300
   gggacacgac tggagattaa ac                                         322
<210> 449
   <211> 107
   <212> PRT
   <213> Artificial Sequence
<220>
   <223> Synthetic
<400> 449
   Asp Ile Gln Leu Thr Gln Ser Pro Ser Phe Leu Ser Ala Ser Val Gly
    1               5                  10                  15
   Asp Arg Val Thr Ile Thr Cys Arg Ala Ser Gln Gly Ile Ile Ser Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Glu Asp Phe Ala Thr Tyr Tyr Cys His Gln Leu Lys Ser Tyr Pro Ile
                   85                  90                  95
   Thr Phe Gly Gln Gly Thr Arg Leu Glu Ile Lys
               100                 105
<210> 450
   <211> 349
   <212> DNA
   <213> Artificial Sequence
<220>
   <223> Synthetic
<400> 450
   caggtgcagc tggtggagtc tgggggaggc ttggtcaagc ctggagggtc cctgagactc 60
   tcctgtgcag cctctggatt caccctcagt gactactaca tgagctggat ccgccaggct 120
   ccagggaagg ggctggagtg ggtttcatac attaggagaa gtggtaatac catatactac 180
   gcagactctg tgaagggccg attcaccatc tccagggaca acgccaagaa ctcactgtat 240
   ctgcaaatga acagcctgag agccgaggac acggccgtgt attactgtgc gagagggggg 300
   gactgggcgg acgtctgggg gcaagggacc acggtcaccg tctcctcag             349
<210> 451
   <211> 116
   <212> PRT
   <213> Artificial Sequence
<220>
   <223> Synthetic
<400> 451
   Gln Val Gln Leu Val Glu Ser Gly Gly Gly Leu Val Lys Pro Gly Gly
    1               5                  10                  15
   Ser Leu Arg Leu Ser Cys Ala Ala Ser Gly Phe Thr Leu Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Arg Arg Ser Gly Asn Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Trp Ala Asp Val Trp Gly Gln Gly Thr Thr Val
               100                 105                 110
   Thr Val Ser Ser
           115
<210> 452
   <211> 322
   <212> DNA
   <213> Artificial Sequence
<220>
   <223> Synthetic
<400> 452
   gacatccaga tgacccagtc tccatcctca ctgtctgcat ctgtaggaga cagagtcacc 60
   atcacttgtc gggcgagtca gggcattagc atttatttag cctggtttca gcagaaacca 120
   gggaaagccc ctaagtccct gatctatgct gcatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtt acccgtacac ttttggccag 300
   gggaccaagc tggagatcaa ac                                          322
<210> 453
   <211> 107
   <212> PRT
   <213> Artificial Sequence
<220>
   <223> Synthetic
<400> 453
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ile Tyr
               20                  25                  30
   Leu Ala Trp Phe Gln Gln Lys Pro Gly Lys Ala Pro Lys Ser Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 454
   <211> 349
   <212> DNA
   <213> Artificial Sequence
<220>
   <223> Synthetic
<400> 454
   caggtgcagc tggtggagtc tgggggagac ttggtcaggc ctggagggtc cctgagactc 60
   tcctgtacag cctctggatt caccctcagt gactactaca tgagctggat ccgtcaggct 120
   ccagggaagg ggctggagtg ggtttcatac attaggagaa gtggtaatac catatactac 180
   gcagactctg tgaagggccg attcaccatc tccagggaca acgccaagaa ctcactgtat 240
   ctgcaaatga acagcctgag agccgaggac acggccgtat attactgtgc gagagggggg 300
   gactgggcgg acgtctgggg ccaagggacc acggtcaccg tctcctcag             349
<210> 455
   <211> 116
   <212> PRT
   <213> Artificial Sequence
<220>
   <223> Synthetic
<400> 455
   Gln Val Gln Leu Val Glu Ser Gly Gly Asp Leu Val Arg Pro Gly Gly
   1               5                   10                  15
   Ser Leu Arg Leu Ser Cys Thr Ala Ser Gly Phe Thr Leu Ser Asp Tyr
               20                  25                  30
   Tyr Met Ser Trp Ile Arg Gln Ala Pro Gly Lys Gly Leu Glu Trp Val
           35                  40                  45
   Ser Tyr Ile Arg Arg Ser Gly Asn Thr Ile Tyr Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Asp Trp Ala Asp Val Trp Gly Gln Gly Thr Thr Val
               100                 105                 110
   Thr Val Ser Ser
<210> 456
   <211> 322
   <212> DNA
   <213> Artificial Sequence
<220>
   <223> Synthetic
<400> 456
   gacatccaga tgacccagtc tccatcctca ctgtctgcct ctgtgggaga cagagtcacc 60
   atcacttgtc gggcgagtca gggcattagc atttatttag cctggtttca gcagaaacca 120
   gggaaagccc ctaagtccct gatctatgct gcatccagtt tgcaacgtgg ggtcccatca 180
   aagttcagcg gcagtggatc tgggacagat ttcactctca ccatcagtag cctgcagcct 240
   gaagattttg caacttatta ctgccaacag tataatagtt acccgtacac ttttggccag 300
   gggaccaagc tggagatcaa ac                                          322
<210> 457
   <211> 107
   <212> PRT
   <213> Artificial Sequence
<220>
   <223> Synthetic
<400> 457
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Ser Ile Tyr
               20                  25                  30
   Leu Ala Trp Phe Gln Gln Lys Pro Gly Lys Ala Pro Lys Ser Leu Ile
           35                  40                  45
   Tyr Ala Ala Ser Ser Leu Gln Arg Gly Val Pro Ser Lys Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser Tyr Pro Tyr
                   85                  90                  95
   Thr Phe Gly Gln Gly Thr Lys Leu Glu Ile Lys
               100                 105
<210> 458
   <211> 352
   <212> DNA
   <213> Artificial Sequence
<220>
   <223> Synthetic
<400> 458
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa cataaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 459
   <211> 117
   <212> PRT
   <213> Artificial Sequence
<220>
   <223> Synthetic
<400> 459
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Ile Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 460
   <211> 322
   <212> DNA
   <213> Artificial Sequence
<220>
   <223> Synthetic
<400> 460
   gccatccaga tgacccagtc tccatcctcc ctgtctgcat ctgtaggaga cagagtcacc 60
   atcacttgcc gggcaagtca ggacattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt acccgtacac ttttggccag 300
   gggaccaagc tggagatcaa ac                                          322
<210> 461
   <211> 107
   <212> PRT
   <213> Artificial Sequence
<220>
   <223> Synthetic
<400> 461
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 462
   <211> 352
   <212> DNA
   <213> Artificial Sequence
<220>
   <223> Synthetic
<400> 462
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa cataaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 463
   <211> 117
   <212> PRT
   <213> Artificial Sequence
<220>
   <223> Synthetic
<400> 463
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Ile Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 464
   <211> 322
   <212> DNA
   <213> Artificial Sequence
<220>
   <223> Synthetic
<400> 464
   gccatccaga tgacccagtc tccatcctcc ctgtctgcat ctgtaggaga cagagtcacc 60
   atcacttgcc gggcaagtca ggacattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt acccgtacac ttttggccag 300
   gggaccaagc tggagatcaa ac                                          322
<210> 465
   <211> 107
   <212> PRT
   <213> Artificial Sequence
<220>
   <223> Synthetic
<400> 465
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Asp Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 466
   <211> 367
   <212> DNA
   <213> Artificial Sequence
<220>
   <223> Synthetic
<400> 466
   caggttcagc tggtgcagtc tggagctgag gtgaagaagc ctggggcctc agtgaaggtc 60
   tcctgcaagg cttctgctta cacctttaat agatatggta tcagctgggt gcgacaggcc 120
   cctggacaag ggcttgagtg gatgggatgg atcagcgctt acactggtaa cacaaactat 180
   gcacagaagc tccagggcag agtcaccatg accacagaca catccacgag cacagcctac 240
   atggagctga ggagcctgag atctgacgac acggccgtgt attactgtgc gagagataag 300
   tcgatttttg gagtggttag agggtttgac tactggggcc aaggaaccct ggtcaccgtc 360
   tcctcag                                                           367
<210> 467
   <211> 122
   <212> PRT
   <213> Artificial Sequence
<220>
   <223> Synthetic
<400> 467
   Gln Val Gln Leu Val Gln Ser Gly Ala Glu Val Lys Lys Pro Gly Ala
    1               5                  10                  15
   Ser Val Lys Val Ser Cys Lys Ala Ser Ala Tyr Thr Phe Asn Arg Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Thr Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Lys Ser Ile Phe Gly Val Val Arg Gly Phe Asp Tyr Trp
               100                 105                 110
   Gly Gln Gly Thr Leu Val Thr Val Ser Ser
           115                 120
<210> 468
   <211> 322
   <212> DNA
   <213> Artificial Sequence
<220>
   <223> Synthetic
<400> 468
   gaaatagtga tgacgcagtc tccagccacc ctgtctgtgt ctccagggga aagagccacc 60
   ctctcctgca gggccagtca gagtgttagc agcagcttag cctggtacca gcagaaacct 120
   ggccaggctc ccaggctcct catctatggt gcatctacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggcctctcac tttcggcgga 300
   gggaccaagg tggagatcaa ac                                          322
<210> 469
   <211> 107
   <212> PRT
   <213> Artificial Sequence
<220>
   <223> Synthetic
<400> 469
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Ser
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
               100                 105
<210> 470
   <211> 367
   <212> DNA
   <213> Artificial Sequence
<220>
   <223> Synthetic

<400> 470
   caggttcagc tggtgcagtc tggagctgag gtgaagaagc ctggggcctc agtgaaggtc 60
   tcctgcaagg cttctgctta cacctttaat agatatggta tcagctgggt gcgacaggcc 120
   cctggacaag ggcttgagtg gatgggatgg atcagcgctt acactggtaa cacaaactat 180
   gcacagaagc tccagggcag agtcaccatg accacagaca catccacgag cacagcctac 240
   atggagctga ggagcctgag atctgacgac acggccgtgt attactgtgc gagagataag 300
   tcgatttttg gagtggttag agggtttgac tactggggcc aaggaaccct ggtcaccgtc 360
   tcctcag                                                           367
<210> 471
   <211> 122
   <212> PRT
   <213> Artificial Sequence
<220>
   <223> Synthetic
<400> 471
   Gln Val Gln Leu Val Gln Ser Gly Ala Glu Val Lys Lys Pro Gly Ala
    1               5                  10                  15
   Ser Val Lys Val Ser Cys Lys Ala Ser Ala Tyr Thr Phe Asn Arg Tyr
               20                  25                  30
   Gly Ile Ser Trp Val Arg Gln Ala Pro Gly Gln Gly Leu Glu Trp Met
           35                  40                  45
   Gly Trp Ile Ser Ala Tyr Thr Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Asp Lys Ser Ile Phe Gly Val Val Arg Gly Phe Asp Tyr Trp
               100                 105                 110
   Gly Gln Gly Thr Leu Val Thr Val Ser Ser
           115                 120
<210> 472
   <211> 322
   <212> DNA
   <213> Artificial Sequence
<220>
   <223> Synthetic
<400> 472
   gaaatagtga tgacgcagtc tccagccacc ctgtctgtgt ctccagggga aagagccacc 60
   ctctcctgca gggccagtca gagtgttagc agcagcttag cctggtacca gcagaaacct 120
   ggccaggctc ccaggctcct catctatggt gcatctacca gggccactgg tatcccagcc 180
   aggttcagtg gcagtgggtc tgggacagag ttcactctca ccatcagcag cctgcagtct 240
   gaagattttg cagtttatta ctgtcagcag tataataact ggcctctcac tttcggcgga 300
   gggaccaagg tggagatcaa ac                                          322
<210> 473
   <211> 107
   <212> PRT
   <213> Artificial Sequence
<220>
   <223> Synthetic
<400> 473
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Ser
               20                  25                  30
   Leu Ala Trp Tyr Gln Gln Lys Pro Gly Gln Ala Pro Arg Leu Leu Ile
           35                  40                  45
   Tyr Gly Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Ser
   65                  70                  75                  80
   Glu Asp Phe Ala Val Tyr Tyr Cys Gln Gln Tyr Asn Asn Trp Pro Leu
                   85                  90                  95
   Thr Phe Gly Gly Gly Thr Lys Val Glu Ile Lys
               100                 105
<210> 474
   <211> 352
   <212> DNA
   <213> Artificial Sequence
<220>
   <223> Synthetic
<400> 474
   caggttcagc tggtgcagtc tggagctgag gtgaagaagc ctggggcctc agtgaaggtc 60
   tcctgcaagg cttctggtta cacctttacc aactatggca tcagctgggt gcgacaggcc 120
   cctggacaag ggcttgagtg gatgggatgg atcagtgttt acaatggcaa cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 475
   <211> 117
   <212> PRT
   <213> Artificial Sequence
<220>
   <223> Synthetic
<400> 475
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 476
   <211> 322
   <212> DNA
   <213> Artificial Sequence
<220>
   <223> Synthetic
<400> 476
   gccatccaga tgacccagtc tccatcctcc ctgtctgcat ctgtaggaga cagagtcacc 60
   atcacttgcc gggcaagtca ggacattaga aatgatttag gctggtatca gcagaaacca 120
   gggaaagccc ctaagctcct gatctatact gcttccagtt tacaaagtgg ggtcccatca 180
   aggttcagcg gcagtggatc tggcacagat ttcactctca ccatcagcag cctgcagcct 240
   gaagattttg caacttatta ctgtctccaa gataatagtt acccgtacac ttttggccag 300
   gggaccaagc tggagatcaa ac                                          322
<210> 477
   <211> 107
   <212> PRT
   <213> Artificial Sequence
<220>
   <223> Synthetic
<400> 477
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   Tyr Thr Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Asn Ser Tyr Pro Tyr
                   85                  90                  95
   Thr Phe Gly Gln Gly Thr Lys Leu Glu Ile Lys
               100                 105
<210> 478
   <211> 352
   <212> DNA
   <213> Artificial Sequence
<220>
   <223> Synthetic
<400> 478
   caggttcagc tggtgcagtc tggagctgag gtgaagaagc ctggggcctc agtgaaggtc 60
   tcctgcaagg cttctggtta cacctttacc aactatggca tcagctgggt gcgacaggcc 120
   cctggacaag ggcttgagtg gatgggatgg atcagtgttt acaatggcaa cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 479
   <211> 117
   <212> PRT
   <213> Artificial Sequence
<220>
   <223> Synthetic
<400> 479
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 480
   <211> 322
   <212> DNA
   <213> Artificial Sequence
<220>
   <223> Synthetic
<400> 480
   gccatccaga tgacccagtc tccttcctcc ctgtctgcat ctgttggaga cagagtcgcc 60
   atcacttgcc gggcaagtca ggacattaga aatgatttag gctggtatca gcagaaacca 120
   gggaaagccc ctaagcttct gatccatact gcttccagtt tacaaagtgg ggtcccatca 180
   aggttcagcg gcagtggatc tggcacagat ttcactctca ccatcagcaa cctgcagcct 240
   gaagattttg ccacttatta ctgtctccaa gataatagtt acccgtacac ttttggccag 300
   gggaccaagc tggagatcaa ac                                          322
<210> 481
   <211> 107
   <212> PRT
   <213> Artificial Sequence
<220>
   <223> Synthetic
<400> 481
   Ala Ile Gln Met Thr Gln Ser Pro Ser Ser Leu Ser Ala Ser Val Gly
    1               5                  10                  15
   Asp Arg Val Ala Ile Thr Cys Arg Ala Ser Gln Asp Ile Arg Asn Asp
               20                  25                  30
   Leu Gly Trp Tyr Gln Gln Lys Pro Gly Lys Ala Pro Lys Leu Leu Ile
           35                  40                  45
   His Thr Ala Ser Ser Leu Gln Ser Gly Val Pro Ser Arg Phe Ser Gly
       50                  55                  60
   Ser Gly Ser Gly Thr Asp Phe Thr Leu Thr Ile Ser Asn Leu Gln Pro
   65                  70                  75                  80
   Glu Asp Phe Ala Thr Tyr Tyr Cys Leu Gln Asp Asn Ser Tyr Pro Tyr
                   85                  90                  95
   Thr Phe Gly Gln Gly Thr Lys Leu Glu Ile Lys
               100                 105
<210> 482
   <211> 349
   <212> DNA
   <213> Artificial Sequence
<220>
   <223> Synthetic
<400> 482
   caggtgcagc tggtgcagtc tggggctgag gtgaagaagc ctggggcctc agtgaaggtc 60
   tcctgcaagg cttctggata caccttcacc ggccagtata tgcactgggt gcgacaggcc 120
   cctggacaag ggcttgagtg gatgggatgg atcaacccta acagtggtgg cacaaactat 180
   gcacagaagt ttcagggcag ggtcaccatg accagggaca cgtccatcag cacagcctac 240
   atggagctga gcaggctgag atctgacgac acggccgtgt attactgtgt gagagaaaag 300
   agaggttttg atatctgggg ccaagggaca atggtcaccg tctcttcag             349
<210> 483
   <211> 116
   <212> PRT
   <213> Artificial Sequence
<220>
   <223> Synthetic
<400> 483
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Gly Gln
               20                  25                  30
   Tyr Met His Trp Val Arg Gln Ala Pro Gly Gln Gly Leu Glu Trp Met
           35                  40                  45
   Gly Trp Ile Asn Pro Asn Ser Gly Gly Thr Asn Tyr Ala Gln Lys Phe
       50                  55                  60
   Gln Gly Arg Val Thr Met Thr Arg Asp Thr Ser Ile Ser Thr Ala Tyr
   65                  70                  75                  80
   Met Glu Leu Ser Arg Leu Arg Ser Asp Asp Thr Ala Val Tyr Tyr Cys
                   85                  90                  95
   Val Arg Glu Lys Arg Gly Phe Asp Ile Trp Gly Gln Gly Thr Met Val
               100                 105                 110
   Thr Val Ser Ser
           115
<210> 484
   <211> 319
   <212> DNA
   <213> Artificial Sequence
<220>
   <223> Synthetic
<400> 484
   gaaattgtgt tgacacagtc tccagccacc ctgtctttgt ctccagggga aagagccacc 60
   ctctcctgca gggccagtca gagtgttagg agctacttag cctggtacca acagaaacct 120
   ggccaggctc ccaggctcct catctatgat gcatccaaca gggccactgg catcccagcc 180
   aggttcagtg gcagtgggtc tgggacagac ttcactctca ccatcagcag cctagagcct 240
   gaagattttg cagtttatta ctgtcagcag cgtagcgact ggcctacttt cggccctggg 300
   accaaagtgg atatcaaac                                              319
<210> 485
   <211> 106
   <212> PRT
   <213> Artificial Sequence
<220>
   <223> Synthetic
<400> 485
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Arg Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asp Trp Pro Thr
                   85                  90                  95
   Phe Gly Pro Gly Thr Lys Val Asp Ile Lys
               100                 105
<210> 486
   <211> 349
   <212> DNA
   <213> Artificial Sequence
<220>
   <223> Synthetic
<400> 486
   caggtgcagc tggtgcagtc tggggctgag gtgaagaagc ctggggcctc agtgaaggtc 60
   tcctgcaagg cttctggata caccttcacc ggccagtata ttcactgggt tcgacaggcc 120
   cctggacaag gacttgagtg gatgggatgg atcaacccta acagtggtgg cacaaacttt 180
   gcacagaagt ttcaggacag ggtcaccatg accagggaca cgtccatcgg cacagtatac 240
   atggaactga ggaggctgac atctgacgac acggccgtat tttattgtgt gagagaaaag 300
   agaggttttg atatctgggg ccaagggaca atggtcaccg tttcttcag              349
<210> 487
   <211> 116
   <212> PRT
   <213> Artificial Sequence
<220>
   <223> Synthetic
<400> 487
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Gly Gln
               20                  25                  30
   Tyr Ile His Trp Val Arg Gln Ala Pro Gly Gln Gly Leu Glu Trp Met
           35                  40                  45
   Gly Trp Ile Asn Pro Asn Ser Gly Gly Thr Asn Phe Ala Gln Lys Phe
       50                  55                  60
   Gln Asp Arg Val Thr Met Thr Arg Asp Thr Ser Ile Gly Thr Val Tyr
   65                  70                  75                  80
   Met Glu Leu Arg Arg Leu Thr Ser Asp Asp Thr Ala Val Phe Tyr Cys
                   85                  90                  95
   Val Arg Glu Lys Arg Gly Phe Asp Ile Trp Gly Gln Gly Thr Met Val
   100 105 110
   Thr Val Ser Ser
   115
<210> 488
   <211> 319
   <212> DNA
   <213> Artificial Sequence
<220>
   <223> Synthetic
<400> 488
   gaaattgtgt tgacacagtc tccagccacc ctgtctttgt ctccagggga aagagccacc 60
   ctctcctgca gggccagtca gagtgttagg agctactttg cctggtacca acagaaacct 120
   ggccaggctc ccagggtcct catctatgat gcatccatca gggccaccgg catcccagcc 180
   aggttcagtg gcagtgggtc tgggacagac ttcagtctca ccattgacag cctagagcct 240
   gaagattttg cagtttatta ctgtcagcag cgtagcgact ggcctacttt cggccctggg 300
   accaaagtgg atatcaaac 319
<210> 489
   <211> 106
   <212> PRT
   <213> Artificial Sequence
<220>
   <223> Synthetic
<400> 489
   Glu Ile Val Leu Thr Gln Ser Pro Ala Thr Leu Ser Leu Ser Pro Gly
   1 5 10 15
   Glu Arg Ala Thr Leu Ser Cys Arg Ala Ser Gln Ser Val Arg Ser Tyr
   20 25 30
   Phe Ala Trp Tyr Gln Gln Lys Pro Gly Gln Ala Pro Arg Val Leu Ile
   35 40 45
   Tyr Asp Ala Ser Ile Arg Ala Thr Gly Ile Pro Ala Arg Phe Ser Gly
   50 55 60
   Ser Gly Ser Gly Thr Asp Phe Ser Leu Thr Ile Asp Ser Leu Glu Pro
   65 70 75 80
   Glu Asp Phe Ala Val Tyr Tyr Cys Gln Gln Arg Ser Asp Trp Pro Thr
   85 90 95
   Phe Gly Pro Gly Thr Lys Val Asp Ile Lys
   100 105
<210> 490
   <211> 352
   <212> DNA
   <213> Artificial Sequence
<220>
   <223> Synthetic
<400> 490
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagtattt acaatggtaa cacaaactat 180
   gcacagaagc tccagggcag agtcaccatg accacagaca catccacgag cacagcctac 240
   atggagctga ggagcctgag atctgacgac acggccgtgt attactgtgc gagaggtagt 300
   ggctacaatt tcgaccactg gggccaagga accctggtca ccgtctcctc ag         352
<210> 491
   <211> 117
   <212> PRT
   <213> Artificial Sequence
<220>
   <223> Synthetic
<400> 491
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Ile Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asn Phe Asp His Trp Gly Gln Gly Thr Leu
               100                 105                 110
   Val Thr Val Ser Ser
           115
<210> 492
   <211> 322
   <212> DNA
   <213> Artificial Sequence
<220>
   <223> Synthetic
<400> 492
   gccatccaga tgacccagtc tccatcctcc ctgtctgcat ctgtaggaga cagagtcacc 60
   atcacttgcc gggcaagtca gggcattaga aatgatttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gataacactt atccgtacac ttttggccag 300
   gggaccaagc tggagatcaa ac                                          322
<210> 493
   <211> 107
   <212> PRT
   <213> Artificial Sequence
<220>
   <223> Synthetic
<400> 493
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Asp
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Asn Thr Tyr Pro Tyr
                   85                  90                  95
   Thr Phe Gly Gln Gly Thr Lys Leu Glu Ile Lys
               100                 105
<210> 494
   <211> 352
   <212> DNA
   <213> Artificial Sequence
<220>
   <223> Synthetic
<400> 494
   caggttcagc tggtgcagtc tggagctgag gtgaagaagc ctggggcctc agtgaaggtc 60
   tcctgcaagg cttctggtta cacctttacc aactatggta tcggctgggt gcgacaggcc 120
   cctggacaag ggcttgagtg gatgggatgg atcagtattt acaatggtaa cacaaattat 180
   gcacagaggc tccagggcag agtcaccatg accacagaca catccacgag cacagccttc 240
   atggacctga ggagcctgaa atctgacgac acggccgttt attactgtgc gagaggtagt 300
   ggctacaatt tcgaccactg gggccaggga accctggtca ccgtctcctc ag         352
<210> 495
   <211> 117
   <212> PRT
   <213> Artificial Sequence
<220>
   <223> Synthetic
<400> 495
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Asn Tyr
               20                  25                  30
   Gly Ile Gly Trp Val Arg Gln Ala Pro Gly Gln Gly Leu Glu Trp Met
           35                  40                  45
   Gly Trp Ile Ser Ile Tyr Asn Gly Asn Thr Asn Tyr Ala Gln Arg Leu
       50                  55                  60
   Gln Gly Arg Val Thr Met Thr Thr Asp Thr Ser Thr Ser Thr Ala Phe
   65                  70                  75                  80
   Met Asp Leu Arg Ser Leu Lys Ser Asp Asp Thr Ala Val Tyr Tyr Cys
                   85                  90                  95
   Ala Arg Gly Ser Gly Tyr Asn Phe Asp His Trp Gly Gln Gly Thr Leu
               100                 105                 110
   Val Thr Val Ser Ser
           115
<210> 496
   <211> 322
   <212> DNA
   <213> Artificial Sequence
<220>
   <223> Synthetic
<400> 496
   gccatccaga tgacccagtc tccatcctcc ctgtctgcat ctgtaggaga cagagtcacc 60
   atcacttgcc gggcaagtca gggcattaga aatgatttag gctggtatca gcagataccg 120
   gggaaagccc ctaaactcct gatctatgct gcatccagtt tacaaagtgg ggtcccgtca 180
   aggttcagcg gcagtggatc tggcacagat ttcactctca ccatcagcaa cctgcagcct 240
   gaagattttg cagtttattt ctgtctacaa gataacactt atccgtacac ttttggccag 300
   gggaccaagc tggagatcaa ac                                          322
<210> 497
   <211> 107
   <212> PRT
   <213> Artificial Sequence
<220>
   <223> Synthetic
<400> 497
   Ala Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Asp
               20                  25                  30
   Leu Gly Trp Tyr Gln Gln Ile Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Asn Leu Gln Pro
   65                  70                  75                  80
   Glu Asp Phe Ala Val Tyr Phe Cys Leu Gln Asp Asn Thr Tyr Pro Tyr
                   85                  90                  95
   Thr Phe Gly Gln Gly Thr Lys Leu Glu Ile Lys
               100                 105
<210> 498
   <211> 352
   <212> DNA
   <213> Artificial Sequence
<220>
   <223> Synthetic
<400> 498
   caggttcagc tggtgcagtc tggagctgag gtgaagaagc ctggggcctc agtgaaggtc 60
   tcctgcaagg cttctggtta cacctttacc aactatggta tcagctgggt gcgacaggcc 120
   cctggacaag ggcttgagtg gatgggatgg atcagcgttt acaatggtaa aacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 499
   <211> 117
   <212> PRT
   <213> Artificial Sequence
<220>
   <223> Synthetic
<400> 499
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
   35 40 45
   Gly Trp Ile Ser Val Tyr Asn Gly Lys Thr Asn Tyr Ala Gln Lys Leu
   50 55 60
   Gln Gly Arg Val Thr Met Thr Thr Asp Thr Ser Thr Ser Thr Ala Tyr
   65 70 75 80
   Met Glu Leu Arg Ser Leu Arg Ser Asp Asp Thr Ala Val Tyr Tyr Cys
   85 90 95
   Ala Arg Gly Ser Gly Tyr Asp Phe Asp Tyr Trp Gly Gln Gly Thr Leu
   100 105 110
   Val Thr Val Ser Ser
   115
<210> 500
   <211> 322
   <212> DNA
   <213> Artificial Sequence
<220>
   <223> Synthetic
<400> 500
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct tcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattataatt acccgtacac ttttggccag 300
   gggaccaagc tggagatcaa ac 322
<210> 501
   <211> 107
   <212> PRT
   <213> Artificial Sequence
<220>
   <223> Synthetic
<400> 501
   Ala Ile Gln Met Thr Gln Ser Pro Ser Ser Leu Ser Ala Ser Val Gly
   1 5 10 15
   Asp Arg Val Thr Ile Thr Cys Arg Ala Ser Gln Gly Ile Arg Asn Ala
   20 25 30
   Leu Gly Trp Tyr Gln Gln Lys Pro Gly Lys Ala Pro Lys Leu Leu Ile
   35 40 45
   Tyr Ala Ser Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Leu Gln Asp Tyr Asn Tyr Pro Tyr
   85 90 95
   Thr Phe Gly Gln Gly Thr Lys Leu Glu Ile Lys
   100 105
<210> 502
   <211> 352
   <212> DNA
   <213> Artificial Sequence
<220>
   <223> Synthetic
<400> 502
   caggttcagc tggtgcagtc tggacctgag gtgaagaagc ctggggcctc agtgaaggtc 60
   tcctgcaagg cttctggtta cacctttacc aactatggta tcagttgggt gcgacaggcc 120
   cctggacaag ggcttgagtg gatgggatgg atcagcgttt acaatggtaa aacaaactat 180
   gcacagaagt tccagggcag agtcaccatg accacagaca catccacgag cacagcctac 240
   atggacctga ggagcctgag atctgacgac acggccgtat attactgtgc gagagggagt 300
   ggctacgatt ttgactactg gggccaggga accctggtca ccgtctcctc ag         352
<210> 503
   <211> 117
   <212> PRT
   <213> Artificial Sequence
<220>
   <223> Synthetic
<400> 503
   Gln Val Gln Leu Val Gln Ser Gly Pro Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Lys Thr Asn Tyr Ala Gln Lys Phe
       50                  55                  60
   Gln Gly Arg Val Thr Met Thr Thr Asp Thr Ser Thr Ser Thr Ala Tyr
   65                  70                  75                  80
   Met Asp Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 504
   <211> 322
   <212> DNA
   <213> Artificial Sequence
<220>
   <223> Synthetic
<400> 504
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct tcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattataatt acccgtacac ttttggccag 300
   gggaccaagc tggagatcaa ac                                         322
<210> 505
   <211> 107
   <212> PRT
   <213> Artificial Sequence
<220>
   <223> Synthetic
<400> 505
   Ala Ile Gln Met Thr Gln Ser Pro Ser Ser Leu Ser Ala Ser Val Gly
    1               5                   10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ser Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 506
   <211> 352
   <212> DNA
   <213> Artificial Sequence
<220>
   <223> Synthetic
<400> 506
   caggttcagc tggtgcagtc tggagctgag gtgaagaagc ctggggcctc agtgaaggtc 60
   tcctgcaagg cttctggtta cacctttacc agctatggta tcagctgggt gcgacaggcc 120
   cctggacaag ggcttgagtg gatgggatgg atcagtgttt acaatggtaa cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 507
   <211> 117
   <212> PRT
   <213> Artificial Sequence
<220>
   <223> Synthetic
<400> 507
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 508
   <211> 322
   <212> DNA
   <213> Artificial Sequence
<220>
   <223> Synthetic
<400> 508
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt acccgtacac ttttggccag 300
   gggaccaagc tggagatcaa ac                                          322
<210> 509
   <211> 107
   <212> PRT
   <213> Artificial Sequence
<220>
   <223> Synthetic
<400> 509
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 510
   <211> 352
   <212> DNA
   <213> Artificial Sequence
<220>
   <223> Synthetic
<400> 510
   caggttcagc tggtgcagtc tggagctgag gtgaagaagc ctggggcctc agtgaaggtc 60
   tcctgcaagg cttctggtta cacctttacc agctatggta tcagctgggt gcgacaggcc 120
   cctggacaag ggcttgagtg gatgggatgg atcagtgttt acaatggtaa cacaaactat 180
   gcacagaagc tccagggcag agtcaccatg accacagaca catccacgag cacagcctac 240
   atggagctga ggagcctgag atctgacgac acggccgtgt attactgtgc gagagggagt 300
   ggctacgatt ttgactactg gggccaagga accctggtca ccgtctcctc ag         352
<210> 511
   <211> 117
   <212> PRT
   <213> Artificial Sequence
<220>
   <223> Synthetic
<400> 511
   Gln Val Gln Leu Val Gln Ser Gly Ala Glu Val Lys Lys Pro Gly Ala
    1               5                  10                  15
   Ser Val Lys Val Ser Cys Lys Ala Ser Gly Tyr Thr Phe Thr Ser Tyr
               20                  25                  30
   Gly Ile Ser Trp Val Arg Gln Ala Pro Gly Gln Gly Leu Glu Trp Met
           35                  40                  45
   Gly Trp Ile Ser Val Tyr Asn Gly Asn Thr Asn Tyr Ala Gln Lys Leu
       50                  55                  60
   Gln Gly Arg Val Thr Met Thr Thr Asp Thr Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Ser Gly Tyr Asp Phe Asp Tyr Trp Gly Gln Gly Thr Leu
               100                 105                 110
   Val Thr Val Ser Ser
           115
<210> 512
   <211> 322
   <212> DNA
   <213> Artificial Sequence
<220>
   <223> Synthetic
<400> 512
   gccatccaga tgacccagtc tccatcctcc ctgtctgcat ctgtaggaga cagagtcacc 60
   atcacttgcc gggcaagtca gggcattaga aatgctttag gctggtatca gcagaaacca 120
   gggaaagccc ctaagctcct gatctatgct gcatccagtt tacaaagtgg ggtcccatca 180
   aggttcagcg gcagtggatc tggcacagat ttcactctca ccatcagcag cctgcagcct 240
   gaagattttg caacttatta ctgtctacaa gattacaatt acccgtacac ttttggccag 300
   gggaccaagc tggagatcaa ac                                           322
<210> 513
   <211> 107
   <212> PRT
   <213> Artificial Sequence
<220>
   <223> Synthetic
<400> 513
   Ala Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Ala
               20                  25                  30
   Leu Gly Trp Tyr Gln Gln Lys Pro Gly Lys Ala Pro Lys Leu Leu Ile
           35                  40                  45
   Tyr Ala Ala Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Leu Gln Asp Tyr Asn Tyr Pro Tyr
                   85                  90                  95
   Thr Phe Gly Gln Gly Thr Lys Leu Glu Ile Lys
               100                 105
<210> 514
   <211> 358
   <212> DNA
   <213> Artificial Sequence
<220>
   <223> Synthetic
<400> 514
   caggtgcagc tggtggagtc tgggggaggc gtggtccagc ctgggaggtc cctgagactc 60
   tcctgtgcag cctctggatt caccttcaga agctatggca tgcactgggt ccgccaggct 120
   ccaggcaagg ggctggagtg ggtggcagtt atatcatatg atggcagtaa aacatactat 180
   gcagactccg tgaagggccg attcaccatc tccagagaca attccaagaa cacgctgtat 240
   ctgcaaatga acagcctgag agctgaggac acggctgtgt attactgtgt gaaagaagga 300
   agaagtggga gttggttcga cccctggggc caaggaaccc tggtcaccgt ctcctcag   358
<210> 515
   <211> 119
   <212> PRT
   <213> Artificial Sequence
<220>
   <223> Synthetic
<400> 515
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Arg Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Lys Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Lys Glu Gly Arg Ser Gly Ser Trp Phe Asp Pro Trp Gly Gln Gly
               100                 105                 110
   Thr Leu Val Thr Val Ser Ser
           115
<210> 516
   <211> 322
   <212> DNA
   <213> Artificial Sequence
<220>
   <223> Synthetic
<400> 516
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 517
   <211> 107
   <212> PRT
   <213> Artificial Sequence
<220>
   <223> Synthetic
<400> 517
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 518
   <211> 358
   <212> DNA
   <213> Artificial Sequence
<220>
   <223> Synthetic
<400> 518
   caggtgcagc tggtggagtc tgggggaggc gtggtccagc ctggagggtc cctgagactc 60
   tcctgtgtag cctctggatt caccttcaga agctatggca tgcactgggt ccgccaggct 120
   ccaggcaggg gactggagtg ggtggcagtt atatcatatg atggcagtaa aacattctac 180
   gtagattccg tgaaggtccg attcaccatc tccagagaca attccaagaa cacgctttat 240
   ttggaaatga acagcctgag agttgacgac acggctgtgt atttctgtgt gaaagaagga 300
   agaagtggga gttggttcga cccctggggc cagggaaccc tggtcgccgt ctcctcag   358
<210> 519
   <211> 119
   <212> PRT
   <213> Artificial Sequence
<220>
   <223> Synthetic
<400> 519
   Gln Val Gln Leu Val Glu Ser Gly Gly Gly Val Val Gln Pro Gly Gly
    1               5                  10                  15
   Ser Leu Arg Leu Ser Cys Val Ala Ser Gly Phe Thr Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Thr Phe Tyr Val Asp Ser Val
       50                  55                  60
   Lys Val Arg Phe Thr Ile Ser Arg Asp Asn Ser Lys Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Ala Val Ser Ser
           115
<210> 520
   <211> 322
   <212> DNA
   <213> Artificial Sequence
<220>
   <223> Synthetic
<400> 520
   gacatccaga tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaactccct gatctttgct acatccagtt tacaaagtgg ggtcccatca 180
   aggttcagcg ccggtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 521
   <211> 107
   <212> PRT
   <213> Artificial Sequence
<220>
   <223> Synthetic
<400> 521
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Asn Ser Leu Ile
           35                  40                  45
   Phe Ala Thr Ser Ser Leu Gln Ser Gly Val Pro Ser Arg Phe Ser Ala
       50                  55                  60
   Gly Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 522
   <211> 358
   <212> DNA
   <213> Artificial Sequence
<220>
   <223> Synthetic
<400> 522
   caggtgcagc tggtggagtc tgggggaggc gtggtccagc ctgggaggtc cctgagactc 60
   tcctgtgcag cctctggatt catcttcaga agttatggca tgcactgggt ccgccaggct 120
   ccaggcaagg ggctggagtg ggtggcagtt atatcatatg atggcagtaa aacatactat 180
   gcagactccg tgaagggccg attcaccatc tccagagaca attccaagaa cacgctgtat 240
   ctgcaaatga acagcctgag agctgaggac acggctgtgt attactgtgt gaaagaagga 300
   agaagtggga gttggttcga cccctggggc caaggaaccc tggtcaccgt ctcctcag   358
<210> 523
   <211> 119
   <212> PRT
   <213> Artificial Sequence
<220>
   <223> Synthetic
<400> 523
   Gln Val Gln Leu Val Glu Ser Gly Gly Gly Val Val Gln Pro Gly Arg
    1               5                   10                  15
   Ser Leu Arg Leu Ser Cys Ala Ala Ser Gly Phe Ile Phe Arg Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Lys Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Lys Glu Gly Arg Ser Gly Ser Trp Phe Asp Pro Trp Gly Gln Gly
               100                 105                 110
   Thr Leu Val Thr Val Ser Ser
           115
<210> 524
   <211> 322
   <212> DNA
   <213> Artificial Sequence
<220>
   <223> Synthetic
<400> 524
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 525
   <211> 107
   <212> PRT
   <213> Artificial Sequence
<220>
   <223> Synthetic
<400> 525
   Asp Ile Gln Met Thr Gln Ser Pro Ser Ser Leu Ser Ala Ser Val Gly
    1               5                   10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Gln Tyr Asn Ser His Pro Trp
   85 90 95
   Thr Phe Gly Gln Gly Thr Lys Val Glu Ile Lys
   100 105
<210> 526
   <211> 358
   <212> DNA
   <213> Artificial Sequence
<220>
   <223> Synthetic
<400> 526
   caggtgcagc tggtggagtc tgggggaggc gtggtccagc ctggagggtc cctgagactc 60
   tcctgtactg cctctggatt catcttcaga agttatggca tgcactgggt ccgccaggct 120
   ccaggcaggg gactggagtg ggtggcagtc atatcatatg atggcagtaa aacattctac 180
   gtagattccg tgaaggtccg attcaccatc tccagagaca actccaagaa cacgctttat 240
   ttggagatga acagcctgag agttgacgac acggctgtat atttctgtgt gaaagaagga 300
   agaagtggga gttggttcga cccctggggc cagggaaccc tggtcaccgt ctcctcag 358
<210> 527
   <211> 119
   <212> PRT
   <213> Artificial Sequence
<220>
   <223> Synthetic
<400> 527
   Gln Val Gln Leu Val Glu Ser Gly Gly Gly Val Val Gln Pro Gly Gly
   1 5 10 15
   Ser Leu Arg Leu Ser Cys Thr Ala Ser Gly Phe Ile Phe Arg Ser Tyr
   20 25 30
   Gly Met His Trp Val Arg Gln Ala Pro Gly Arg Gly Leu Glu Trp Val
   35 40 45
   Ala Val Ile Ser Tyr Asp Gly Ser Lys Thr Phe Tyr Val Asp Ser Val
   50 55 60
   Lys Val Arg Phe Thr Ile Ser Arg Asp Asn Ser Lys Asn Thr Leu Tyr
   65 70 75 80
   Leu Glu Met Asn Ser Leu Arg Val Asp Asp Thr Ala Val Tyr Phe Cys
   85 90 95
   Val Lys Glu Gly Arg Ser Gly Ser Trp Phe Asp Pro Trp Gly Gln Gly
   100 105 110
   Thr Leu Val Thr Val Ser Ser
   115
<210> 528
   <211> 322
   <212> DNA
   <213> Artificial Sequence
<220>
   <223> Synthetic

<400> 528
   gacatccaga tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 529
   <211> 107
   <212> PRT
   <213> Artificial Sequence
<220>
   <223> Synthetic
<400> 529
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 530
   <211> 358
   <212> DNA
   <213> Artificial Sequence
<220>
   <223> Synthetic
<400> 530
   caggtgcagc tggtggagtc tgggggaggc gtggtccagc ctgggaggtc cctgagactc 60
   tcctgtgcag cctctggatt catcttcaga agctatggca tgcactgggt ccgccaggct 120
   ccaggcaagg ggctggagtg ggtggcagtt atatcatatg atggcagtaa aacatactat 180
   gcagactccg tgaagggccg attcaccatc tccagagaca attccaagaa cacgctgtat 240
   ctgcaaatga acagcctgag agctgaggac acggctgtgt attactgtgt gaaagaagga 300
   agaagtggga gttggttcga cccctggggc caaggaaccc tggtcaccgt ctcctcag   358
<210> 531
   <211> 119
   <212> PRT
   <213> Artificial Sequence
<220>
   <223> Synthetic
<400> 531
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Ile Phe Arg Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Lys Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Lys Glu Gly Arg Ser Gly Ser Trp Phe Asp Pro Trp Gly Gln Gly
               100                 105                 110
   Thr Leu Val Thr Val Ser Ser
           115
<210> 532
   <211> 322
   <212> DNA
   <213> Artificial Sequence
<220>
   <223> Synthetic
<400> 532
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 533
   <211> 107
   <212> PRT
   <213> Artificial Sequence
<220>
   <223> Synthetic
<400> 533
   Asp Ile Gln Met Thr Gln Ser Pro Ser Ser Leu Ser Ala Ser Val Gly
    1              5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 534
   <211> 358
   <212> DNA
   <213> Artificial Sequence
<220>
   <223> Synthetic
<400> 534
   caggtgcagc tggtggagtc tgggggaggc gtggtccagc ctggagggtc cctgagactc 60
   tcctgtgctg cctctggatt catcttcaga agctatggca tgcactgggt ccgccaggct 120
   ccaggcaggg gactggagtg ggtggcagtc atatcatatg atggcagtaa aacattctac 180
   gtagattccg tgaaggtccg attcaccata tccagagaca actccaagaa cacgctttat 240
   ttggaaatga acagcctgag agttgacgac acggctgtat atttctgtgt gaaagaagga 300
   agaagtggga gttggttcga cccctggggc cagggaaccc tggtcaccgt ctcctcag   358
<210> 535
   <211> 119
   <212> PRT
   <213> Artificial Sequence
<220>
   <223> Synthetic
<400> 535
   Gln Val Gln Leu Val Glu Ser Gly Gly Gly Val Val Gln Pro Gly Gly
   1 5 10 15
   Ser Leu Arg Leu Ser Cys Ala Ala Ser Gly Phe Ile Phe Arg Ser Tyr
   20 25 30
   Gly Met His Trp Val Arg Gln Ala Pro Gly Arg Gly Leu Glu Trp Val
   35 40 45
   Ala Val Ile Ser Tyr Asp Gly Ser Lys Thr Phe Tyr Val Asp Ser Val
   50 55 60
   Lys Val Arg Phe Thr Ile Ser Arg Asp Asn Ser Lys Asn Thr Leu Tyr
   65 70 75 80
   Leu Glu Met Asn Ser Leu Arg Val Asp Asp Thr Ala Val Tyr Phe Cys
   85 90 95
   Val Lys Glu Gly Arg Ser Gly Ser Trp Phe Asp Pro Trp Gly Gln Gly
   100 105 110
   Thr Leu Val Thr Val Ser Ser
   115
<210> 536
   <211> 322
   <212> DNA
   <213> Artificial Sequence
<220>
   <223> Synthetic
<400> 536
   gacatccaga tgacccagtc tccatcctca ctgtctgcgt ctgtaggaga cagagtcacc 60
   atcacttgtc gggcgagtca ggccattaac aattttttag cctggtttca gcagaaacca 120
   gggaaaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa ac 322
<210> 537
   <211> 107
   <212> PRT
   <213> Artificial Sequence
<220>
   <223> Synthetic
<400> 537
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 538
   <211> 355
   <212> DNA
   <213> Artificial Sequence
<220>
   <223> Synthetic
<400> 538
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt atatcatatg atggaagtat taaatactat 180
   gcagactccg tgaagggccg attcaccatc tccagagaca attccaagaa cacgctgtat 240
   ctgcaaatga acagcctgag agctgaggac acggctgtgt attactgtgc gaaggaaggt 300
   aggaagtacg gtatggacgt ctgggggcaa gggaccacgg tcaccgtctc ctcag      355
<210> 539
   <211> 118
   <212> PRT
   <213> Artificial Sequence
<220>
   <223> Synthetic
<400> 539
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Ile Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Gly Arg Lys Tyr Gly Met Asp Val Trp Gly Gln Gly Thr
               100                 105                 110
   Thr Val Thr Val Ser Ser
           115
<210> 540
   <211> 322
   <212> DNA
   <213> Artificial Sequence
<220>
   <223> Synthetic
<400> 540
   gacatccaga tgacccagtc tccttccacc ctgtctgcat ctgtaggaga cagagtcacc 60
   atcacttgcc gggccagtca gattattagt agcttattgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatcatagtt acatgtacac ttttggccag 300
   gggaccaagc tggagatcaa ac                                          322
<210> 541
   <211> 107
   <212> PRT
   <213> Artificial Sequence
<220>
   <223> Synthetic
<400> 541
   Asp Ile Gln Met Thr Gln Ser Pro Ser Thr Leu Ser Ala Ser Val Gly
    1               5                   10                  15
   Asp Arg Val Thr Ile Thr Cys Arg Ala Ser Gln Ile Ile Ser Ser Leu
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr His Ser Tyr Met Tyr
                   85                  90                  95
   Thr Phe Gly Gln Gly Thr Lys Leu Glu Ile Lys
               100                 105
<210> 542
   <211> 355
   <212> DNA
   <213> Artificial Sequence
<220>
   <223> Synthetic
<400> 542
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt atatcatatg atggaagtat taaatactat 180
   gcagactccg tgaagggccg attcaccatc tccagagaca attccaagaa cacgctgtat 240
   ctgcaaatga acagcctgag agctgaggac acggctgtgt attactgtgc gaaggaaggt 300
   aggaagtacg gtatggacgt ctgggggcaa gggaccacgg tcaccgtctc ctcag      355
<210> 543
   <211> 118
   <212> PRT
   <213> Artificial Sequence
<220>
   <223> Synthetic
<400> 543
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Ile Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Gly Arg Lys Tyr Gly Met Asp Val Trp Gly Gln Gly Thr
               100                 105                 110
   Thr Val Thr Val Ser Ser
           115
<210> 544
   <211> 322
   <212> DNA
   <213> Artificial Sequence
<220>
   <223> Synthetic
<400> 544
   gacatccaga tgacccagtc tccttccacc ctgtctgcat ctgtaggaga cagagtcacc 60
   atcacttgcc gggccagtca gattattagt agcttattgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tatcatagtt acatgtacac ttttggccag 300
   gggaccaagc tggagatcaa ac                                          322
<210> 545
   <211> 107
   <212> PRT
   <213> Artificial Sequence
<220>
   <223> Synthetic
<400> 545
   Asp Ile Gln Met Thr Gln Ser Pro Ser Thr Leu Ser Ala Ser Val Gly
   1               5                   10                  15
   Asp Arg Val Thr Ile Thr Cys Arg Ala Ser Gln Ile Ile Ser Ser Leu
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr His Ser Tyr Met Tyr
                   85                  90                  95
   Thr Phe Gly Gln Gly Thr Lys Leu Glu Ile Lys
   100 105
<210> 546
   <211> 358
   <212> DNA
   <213> Artificial Sequence
<220>
   <223> Synthetic
<400> 546
   caggtgcagc tggtggagtc tgggggaggc gtggtccagc ctgggaggtc cctgagactc 60
   tcctgtgcag cctctggatt caccttcagt agctttggca tgcactgggt ccgccaggct 120
   ccaggcaagg ggctggagtg ggtggcagtt atatcatatg atggcagtaa aaaatactat 180
   gcagactccg tgaagggccg attcaccatc tccagagaca attccaagaa cacgctgtat 240
   ctgcaaatga acagcctgag agctgaggac acggctgtgt attactgtgc gaaagaagga 300
   agaactggga attggttcga cccctggggc caaggaaccc tggtcaccgt ctcctcag   358
<210> 547
   <211> 119
   <212> PRT
   <213> Artificial Sequence
<220>
   <223> Synthetic
<400> 547
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Phe
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Lys Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Gly Arg Thr Gly Asn Trp Phe Asp Pro Trp Gly Gln Gly
               100                 105                 110
   Thr Leu Val Thr Val Ser Ser
           115
<210> 548
   <211> 322
   <212> DNA
   <213> Artificial Sequence
<220>
   <223> Synthetic
<400> 548
   gacatccaga tgacccagtc tccatcctca ctgtctgcat ctgtaggaga cagagtcacc 60
   atcacttgtc gggcgagtca ggccattaac aattatttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 549
   <211> 107
   <212> PRT
   <213> Artificial Sequence
<220>
   <223> Synthetic
<400> 549
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Tyr
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 550
   <211> 358
   <212> DNA
   <213> Artificial Sequence
<220>
   <223> Synthetic
<400> 550
   caggtgcagc tggtggagtc tgggggaggc gtggtccagc ctgggaggtc cctgagactc 60
   tcctgtacag cctctggatt caccttcagt agctttggca tgcactgggt ccgccaggct 120
   ccaggcaggg gactggagtg ggtggcagtt atatcatatg atggcagtaa aaaatactac 180
   gcagactccg tgaaggtccg attcaccatc tccagagaca actccaagaa cacgctttat 240
   ctgcaaatga acagcctgag agttgacgac acggctgtgt atttctgtgc gaaagaagga 300
   agaactggga attggttcga cccctggggc cagggaaccc tggtcaccgt ctcctcag   358
<210> 551
   <211> 119
   <212> PRT
   <213> Artificial Sequence
<220>
   <223> Synthetic
<400> 551
   Gln Val Gln Leu Val Glu Ser Gly Gly Gly Val Val Gln Pro Gly Arg
   1               5                   10                  15
   Ser Leu Arg Leu Ser Cys Thr Ala Ser Gly Phe Thr Phe Ser Ser Phe
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Lys Lys Tyr Tyr Ala Asp Ser Val
       50                  55                  60
   Lys Val Arg Phe Thr Ile Ser Arg Asp Asn Ser Lys Asn Thr Leu Tyr
   65                  70                  75                  80
   Leu Gln Met Asn Ser Leu Arg Val Asp Asp Thr Ala Val Tyr Phe Cys
                   85                  90                  95
   Ala Lys Glu Gly Arg Thr Gly Asn Trp Phe Asp Pro Trp Gly Gln Gly
               100                 105                 110
   Thr Leu Val Thr Val Ser Ser
           115
<210> 552
   <211> 322
   <212> DNA
   <213> Artificial Sequence
<220>
   <223> Synthetic
<400> 552
   gacatccaga tgacccagtc tccatcctca ctgtctgcat ctgtaggaga cagagtcacc 60
   atcacttgtc gggcgagtca ggccattaac aattatttag cctggtttca gcagaaacca 120
   gggaaaaccc ctaagtccct gatctttgct acatccagtt tacaaagtgg ggtcccatca 180
   aggttcagcg gcactggatc tgggacagac ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 553
   <211> 107
   <212> PRT
   <213> Artificial Sequence
<220>
   <223> Synthetic
<400> 553
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Tyr
               20                  25                  30
   Leu Ala Trp Phe Gln Gln Lys Pro Gly Lys Thr Pro Lys Ser Leu Ile
           35                  40                  45
   Phe Ala Thr Ser Ser Leu Gln Ser Gly Val Pro Ser Arg Phe Ser Gly
       50                  55                  60
   Thr Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 554
   <211> 358
   <212> DNA
   <213> Artificial Sequence
<220>
   <223> Synthetic
<400> 554
   caggtgcagc tggtggagtc tgggggaggc gtggtccagc ctgggaggtc cctgagactc 60
   tcctgtgcag cctctggatt caccttcaga agttatggca tgcactgggt ccgccaggct 120
   ccaggcaagg ggctggagtg ggtggcagtt atatcatatg atggcagtca aacatactat 180
   gcagactccg tgaagggccg attcaccatc tccagagaca attccaagaa cacgctgtat 240
   ctgcaaatga acagcctgag agctgaggac acggctgtgt attactgtgt gaaagaagga 300
   agaagtggga gttggttcga cccctggggc caaggaaccc tggtcaccgt ctcctcag   358
<210> 555
   <211> 119
   <212> PRT
   <213> Artificial Sequence
<220>
   <223> Synthetic
<400> 555
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Arg Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Gln Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Val Lys Glu Gly Arg Ser Gly Ser Trp Phe Asp Pro Trp Gly Gln Gly
               100                 105                 110
   Thr Leu Val Thr Val Ser Ser
           115
<210> 556
   <211> 322
   <212> DNA
   <213> Artificial Sequence
<220>
   <223> Synthetic
<400> 556
   gacatccaga tgacccagtc tccatcctca ctgtctgcat ctgtaggaga cagagtcacc 60
   atcacttgtc gggcgagtca ggccattaat aattatttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 557
   <211> 107
   <212> PRT
   <213> Artificial Sequence
<220>
   <223> Synthetic
<400> 557
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Tyr
               20                  25                  30
   Leu Ala Trp Phe Gln Gln Lys Pro Gly Lys Ala Pro Lys Ser Leu Ile
               35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 558
   <211> 358
   <212> DNA
   <213> Artificial Sequence
<220>
   <223> Synthetic
<400> 558
   caggtgcagc tggtggagtc tgggggaggc gtggtccagc ctggaaggtc cctgagactc 60
   tcctgtgtag cctctggatt caccttcaga agttatggca tgcactgggt ccgccaggct 120
   ccaggcaggg gactggagtg ggtggcagtc atatcatatg atggcagtca aacattctat 180
   gtagattccg tgaaggtccg attcaccatc tccagagaca actccaacaa cacgctttat 240
   ttggaaatga acagcctgag agttgacgac acggctgttt atttctgtgt gaaagaagga 300
   agaagtggga gttggttcga cccctggggc cagggaaccc tggtcaccgt ctcctcag   358
<210> 559
   <211> 119
   <212> PRT
   <213> Artificial Sequence
<220>
   <223> Synthetic
<400> 559
   Gln Val Gln Leu Val Glu Ser Gly Gly Gly Val Val Gln Pro Gly Arg
   1               5                   10                  15
   Ser Leu Arg Leu Ser Cys Val Ala Ser Gly Phe Thr Phe Arg Ser Tyr
               20                  25                  30
   Gly Met His Trp Val Arg Gln Ala Pro Gly Arg Gly Leu Glu Trp Val
           35                  40                  45
   Ala Val Ile Ser Tyr Asp Gly Ser Gln Thr Phe Tyr Val Asp Ser Val
       50                  55                  60
   Lys Val Arg Phe Thr Ile Ser Arg Asp Asn Ser Asn Asn Thr Leu Tyr
   65                  70                  75                  80
   Leu Glu Met Asn Ser Leu Arg Val Asp Asp Thr Ala Val Tyr Phe Cys
                   85                  90                  95
   Val Lys Glu Gly Arg Ser Gly Ser Trp Phe Asp Pro Trp Gly Gln Gly
               100                 105                 110
   Thr Leu Val Thr Val Ser Ser
           115
<210> 560
   <211> 322
   <212> DNA
   <213> Artificial Sequence
<220>
   <223> Synthetic
<400> 560
   gacatccaga tgacccagtc tccatcctca ctgtctgcgt ctgttggaga cagagtcacc 60
   atcacttgtc gggcgagtca ggccattaat aattatttag cctggtttca gcagaaacca 120
   gggagaaccc ctaagtccct gatctttgct acatccagtt tacaaagtgg ggtcccatca 180
   aggttcagcg gcagtggatc tgggacagac ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 561
   <211> 107
   <212> PRT
   <213> Artificial Sequence
<220>
   <223> Synthetic
<400> 561
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ala Ile Asn Asn Tyr
               20                  25                  30
   Leu Ala Trp Phe Gln Gln Lys Pro Gly Arg Thr Pro Lys Ser Leu Ile
           35                  40                  45
   Phe Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 562
   <211> 355
   <212> DNA
   <213> Artificial Sequence
<220>
   <223> Synthetic
<400> 562
   caggtgcagc tggtggagtc tgggggaggc gtggtccagc ctgggaggtc cctgagactc 60
   tcctgtgcag cctctggatt caccttcagt agttatgcca tgcactgggt ccgccaggct 120
   ccaggcaagg ggctggagtg ggtggcagtt atatcatatg atggaagtaa taaatactat 180
   gcagactccg tgaagggccg attcaccatc tccagagaca attccaagaa cacgctgtat 240
   ctgcaaatga acagcctgag agctgaggac acggctgtgt attactgtgc gaaagaaagt 300
   tcttcgtacg gtttggacgt ctgggggcaa gggaccacgg tcaccgtctc ctcag      355
<210> 563
   <211> 118
   <212> PRT
   <213> Artificial Sequence
<220>
   <223> Synthetic
<400> 563
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Ser Ser Ser Tyr Gly Leu Asp Val Trp Gly Gln Gly Thr
               100                 105                 110
   Thr Val Thr Val Ser Ser
           115
<210> 564
   <211> 322
   <212> DNA
   <213> Artificial Sequence
<220>
   <223> Synthetic
<400> 564
   gacatccaga tgacccagtc tccttccacc ctgtctgcat ctgtaggaga cagagtcacc 60
   atcacttgcc gggccagtca gagtattagt aactggttgg cctggtatca gcagaaacca 120
   gggaaagccc ctaagctcct gatctataag gcgtctagtt tagaaagtgg ggtcccatca 180
   aggttcagcg gcagtggatc tgggacagaa ttcactctca ccatcagcag cctgcagcct 240
   gatgattttg caacttatta ctgccaacag tataatagtt attcgtacac ttttggccag 300
   gggaccaagc tggagatcaa ac                                          322
<210> 565
   <211> 107
   <212> PRT
   <213> Artificial Sequence
<220>
   <223> Synthetic
<400> 565
   Asp Ile Gln Met Thr Gln Ser Pro Ser Thr Leu Ser Ala Ser Val Gly
    1               5                  10                  15
   Asp Arg Val Thr Ile Thr Cys Arg Ala Ser Gln Ser Ile Ser Asn Trp
               20                  25                  30
   Leu Ala Trp Tyr Gln Gln Lys Pro Gly Lys Ala Pro Lys Leu Leu Ile
           35                  40                  45
   Tyr Lys Ala Ser Ser Leu Glu Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Thr Tyr Tyr Cys Gln Gln Tyr Asn Ser Tyr Ser Tyr
                   85                  90                  95
   Thr Phe Gly Gln Gly Thr Lys Leu Glu Ile Lys
               100                 105
<210> 566
   <211> 355
   <212> DNA
   <213> Artificial Sequence
<220>
   <223> Synthetic
<400> 566
   caggtgcagc tggtggagtc tgggggaggc gtggtccagc ctgggaggtc cctgagactc 60
   tcctgtgcag cctctggatt caccttcagt agttatgcca tgtactgggt ccgccaggct 120
   ccaggcaagg ggctggagtg ggtggcagtg atatcatatg atggaagtaa taaatactat 180
   acagactccg tgaagggccg attcaccgtc tccagagaca attccaaaaa cacgctgttt 240
   ctgcaaatga acagcctgag agctgaggac acggctctgt attactgtgc gaaagaaagt 300
   tcttcgtacg gtttggacgt ctggggtcaa gggaccacgg tcaccgtctc ctcag      355
<210> 567
   <211> 118
   <212> PRT
   <213> Artificial Sequence
<220>
   <223> Synthetic
<400> 567
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Ala Met Tyr Trp Val Arg Gln Ala Pro Gly Lys Gly Leu Glu Trp Val
           35                  40                  45
   Ala Val Ile Ser Tyr Asp Gly Ser Asn Lys Tyr Tyr Thr Asp Ser Val
       50                  55                  60
   Lys Gly Arg Phe Thr Val Ser Arg Asp Asn Ser Lys Asn Thr Leu Phe
   65                  70                  75                  80
   Leu Gln Met Asn Ser Leu Arg Ala Glu Asp Thr Ala Leu Tyr Tyr Cys
                   85                  90                  95
   Ala Lys Glu Ser Ser Ser Tyr Gly Leu Asp Val Trp Gly Gln Gly Thr
               100                 105                 110
   Thr Val Thr Val Ser Ser
           115
<210> 568
   <211> 322
   <212> DNA
   <213> Artificial Sequence
<220>
   <223> Synthetic
<400> 568
   gacatccaga tgacccagtc tccttccacc ctgtctgcat ctgtaggaga cagagtcgcc 60
   atcacttgcc gggccagtca gagtattagt aactggttgg cctggtatca gcagaaacca 120
   gggaaagccc ctaaactcct gatctctaag gcgtctagtt taaaaagtgg ggtcccatca 180
   aggttcagcg gcagtggatc tgggacagaa ttcactctca ccatcagcag cctgcagcct 240
   gatgattttg cagcttatta ctgccaacag tataatagtt attcgtacac ttttggccag 300
   gggaccaagc tggagatcaa ac                                          322
<210> 569
   <211> 107
   <212> PRT
   <213> Artificial Sequence
<220>
   <223> Synthetic
<400> 569
   Asp Ile Gln Met Thr Gln Ser Pro Ser Thr Leu Ser Ala Ser Val Gly
    1               5                  10                  15
   Asp Arg Val Ala Ile Thr Cys Arg Ala Ser Gln Ser Ile Ser Asn Trp
               20                  25                  30
   Leu Ala Trp Tyr Gln Gln Lys Pro Gly Lys Ala Pro Lys Leu Leu Ile
           35                  40                  45
   Ser Lys Ala Ser Ser Leu Lys Ser Gly Val Pro Ser Arg Phe Ser Gly
       50                  55                  60
   Ser Gly Ser Gly Thr Glu Phe Thr Leu Thr Ile Ser Ser Leu Gln Pro
   65                  70                  75                  80
   Asp Asp Phe Ala Ala Tyr Tyr Cys Gln Gln Tyr Asn Ser Tyr Ser Tyr
                   85                  90                  95
   Thr Phe Gly Gln Gly Thr Lys Leu Glu Ile Lys
               100                 105
<210> 570
   <211> 358
   <212> DNA
   <213> Artificial Sequence
<220>
   <223> Synthetic
<400> 570
   caggtgcagc tggtggagtc tgggggaggc gtggtccagc ctgggaggtc cctgagactc 60
   tcctgtgcag cctctggatt caccttcagt agctatggca tgcactgggt ccgccaggct 120
   ccaggcaagg ggctggagtg ggtggcagtt ttatcatatg atggcagtaa agaatactat 180
   gcagactccg tgaagggccg attcaccatc tccagagaca attccaagaa cacgctgtat 240
   ctgcaaatga acagcctgag agctgaggac acggctgtgt attactgtgc gaaagaagga 300
   agaactggga attggttcga cccctggggc caaggaaccc tggtcaccgt ctcctcag   358
<210> 571
   <211> 119
   <212> PRT
   <213> Artificial Sequence
<220>
   <223> Synthetic
<400> 571
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Leu Ser Tyr Asp Gly Ser Lys Glu Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Glu Gly Arg Thr Gly Asn Trp Phe Asp Pro Trp Gly Gln Gly
               100                 105                 110
   Thr Leu Val Thr Val Ser Ser
           115
<210> 572
   <211> 322
   <212> DNA
   <213> Artificial Sequence
<220>
   <223> Synthetic
<400> 572
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 573
   <211> 107
   <212> PRT
   <213> Artificial Sequence
<220>
   <223> Synthetic
<400> 573
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
               100                 105
<210> 574
   <211> 358
   <212> DNA
   <213> Artificial Sequence
<220>
   <223> Synthetic
<400> 574
   caggtgcagc tggtggagtc tgggggaggc gtggtccagc ctggggggtc cctgagactc 60
   tcctgtgcag cctctggatt caccttcagt agctatggca tgcactgggt ccgccaggct 120
   ccaggcaggg gactggagtg ggtggcagtt ttatcatatg atggcagtaa agaattctac 180
   gcagactccg tgaaggtccg attcaccgtc tccagagaca actccaagaa cacgctttat 240
   ctgcaaatga gcagcctgag agttgaggac acggctatat atttctgtgc gaaagaagga 300
   agaactggga attggttcga cccctggggc cagggaaccc tggtcaccgt ctcctcag   358
<210> 575
   <211> 119
   <212> PRT
   <213> Artificial Sequence
<220>
   <223> Synthetic
<400> 575
   Gln Val Gln Leu Val Glu Ser Gly Gly Gly Val Val Gln Pro Gly Gly
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Arg Gly Leu Glu Trp Val
           35                  40                  45
   Ala Val Leu Ser Tyr Asp Gly Ser Lys Glu Phe Tyr Ala Asp Ser Val
       50                  55                  60
   Lys Val Arg Phe Thr Val Ser Arg Asp Asn Ser Lys Asn Thr Leu Tyr
   65                  70                  75                  80
   Leu Gln Met Ser Ser Leu Arg Val Glu Asp Thr Ala Ile Tyr Phe Cys
                   85                  90                  95
   Ala Lys Glu Gly Arg Thr Gly Asn Trp Phe Asp Pro Trp Gly Gln Gly
               100                 105                 110
   Thr Leu Val Thr Val Ser Ser
           115
<210> 576
   <211> 322
   <212> DNA
   <213> Artificial Sequence
<220>
   <223> Synthetic
<400> 576
   gacatccaga tgacccagtc tccatcctca ctgtctgcat ctgtaggaga cagagtcacc 60
   atcacttgtc gggcgagtca ggccattaac aattttttag cctggtttca gcagaaacca 120
   gggaaagccc ctaagtccct gatctatgct acatccagtt tgcaaagtgg ggtcccatca 180
   aggttcagcg gcagtggatc tgggacagat ttcactctca ccatcagcag cctgcagcct 240
   gaagattttg caacttatta ctgccaacag tataatagtc acccgtggac gttcggccaa 300
   gggaccaagg tggaaatcaa ac                                          322
<210> 577
   <211> 107
   <212> PRT
   <213> Artificial Sequence
<220>
   <223> Synthetic
<400> 577
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Ala Ile Asn Asn Phe
               20                  25                  30
   Leu Ala Trp Phe Gln Gln Lys Pro Gly Lys Ala Pro Lys Ser Leu Ile
           35                  40                  45
   Tyr Ala Thr Ser Ser Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ser His Pro Trp
                   85                  90                  95
   Thr Phe Gly Gln Gly Thr Lys Val Glu Ile Lys
   100 105
<210> 578
   <211> 355
   <212> DNA
   <213> Artificial Sequence
<220>
   <223> Synthetic
<400> 578
   gaagtgcacc tggtggaatc tgggggaggc ttggtacagc ctggcaggtc cctgagactc 60
   tcctgtgagg cctctggatt cacctttgat gattatgcca tgcactgggt ccggcaagct 120
   ccggggaagg gcctggaatg ggtctcaggt cttagtcgga caagtgtcag tataggctat 180
   gcggactctg tgaagggccg attcaccatc tccagagaca acgccaagaa ctccctttat 240
   ttggaaatga acagtctgag acctgaggac acggccttat attactgtgc aaaatggggg 300
   acccgggggt attttgacta ctggggccag ggaaccctgg tcaccgtctc ctcag      355
<210> 579
   <211> 118
   <212> PRT
   <213> Artificial Sequence
<220>
   <223> Synthetic
<400> 579
   Glu Val His Leu Val Glu Ser Gly Gly Gly Leu Val Gln Pro Gly Arg
   1               5                   10                  15
   Ser Leu Arg Leu Ser Cys Glu Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Leu Ser Arg Thr Ser Val Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Glu Met Asn Ser Leu Arg Pro Glu Asp Thr Ala Leu Tyr Tyr Cys
                   85                  90                  95
   Ala Lys Trp Gly Thr Arg Gly Tyr Phe Asp Tyr Trp Gly Gln Gly Thr
               100                 105                 110
   Leu Val Thr Val Ser Ser
           115
<210> 580
   <211> 355
   <212> DNA
   <213> Artificial Sequence
<220>
   <223> Synthetic
<400> 580
   gaagtgcagc tggtggagtc tgggggaggc ttggtacagc ctggcaggtc cctgagactc 60
   tcctgtgagg cctctggatt cacctttgat gattatgcca tgcactgggt ccggcaagct 120
   ccggggaagg gcctggaatg ggtctcaggt cttagtcgga caagtgtcag tataggctat 180
   gcggactctg tgaagggccg attcaccatc tccagagaca acgccaagaa ctccctttat 240
   ttggaaatga acagtctgag acctgaggac acggccttat attactgtgc aaaatggggg 300
   acccgggggt attttgacta ctggggccag ggaaccctgg tcaccgtctc ctcag      355
<210> 581
   <211> 118
   <212> PRT
   <213> Artificial Sequence
<220>
   <223> Synthetic
<400> 581
   Glu Val Gln Leu Val Glu Ser Gly Gly Gly Leu Val Gln Pro Gly Arg
    1               5                  10                  15
   Ser Leu Arg Leu Ser Cys Glu Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Leu Ser Arg Thr Ser Val Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Glu Met Asn Ser Leu Arg Pro Glu Asp Thr Ala Leu Tyr Tyr Cys
                   85                  90                  95
   Ala Lys Trp Gly Thr Arg Gly Tyr Phe Asp Tyr Trp Gly Gln Gly Thr
               100                 105                 110
   Leu Val Thr Val Ser Ser
           115
<210> 582
   <211> 8
   <212> PRT
   <213> Artificial Sequence
<220>
   <223> Synthetic
<220>
   <221> VARIANT
   <222> (1)...(8)
   <223> Xaa = Any amino acid
<400> 582
   Xaa Xaa Xaa Xaa Xaa Xaa Xaa Xaa
    1               5
<210> 583
   <211> 8
   <212> PRT
   <213> Artificial Sequence
<220>
   <223> Synthetic
<220>
   <221> VARIANT
   <222> (1)...(8)
   <223> Xaa = Any amino acid
<400> 583
   Xaa Xaa Xaa Xaa Xaa Xaa Xaa Xaa
    1               5
<210> 584
   <211> 21
   <212> PRT
   <213> Artificial Sequence
<220>
   <223> Synthetic
<220>
   <221> VARIANT
   <222> (1)...(21)
   <223> Xaa = ANy amino acid
<400> 584
   Xaa Xaa Xaa Xaa Xaa Xaa Xaa Xaa Xaa Xaa Xaa Xaa Xaa Xaa Xaa Xaa
    1               5                  10                  15
   Xaa Xaa Xaa Xaa Xaa
               20
<210> 585
   <211> 6
   <212> PRT
   <213> Artificial Sequence
<220>
   <223> Synthetic
<220>
   <221> VARIANT
   <222> (1)...(6)
   <223> Xaa = Any amino acid
<400> 585
   Xaa Xaa Xaa Xaa Xaa Xaa
    1               5
<210> 586
   <211> 3
   <212> PRT
   <213> Artificial Sequence
<220>
   <223> Synthetic
<220>
   <221> VARIANT
   <222> (1)...(3)
   <223> Xaa = Any amino acid
<400> 586
   Xaa Xaa Xaa
   1
<210> 587
   <211> 9
   <212> PRT
   <213> Artificial Sequence

<220>
   <223> Synthetic
<220>
   <221> VARIANT
   <222> (1)...(9)
   <223> Xaa = Any amino acid
<400> 587
   Xaa Xaa Xaa Xaa Xaa Xaa Xaa Xaa Xaa
   1 5
<210> 588
   <211> 330
   <212> PRT
   <213> Artificial Sequence
<220>
   <223> Synthetic
<400> 588
<210> 589
   <211> 327
   <212> PRT
   <213> Artificial Sequence
<220>
   <223> Synthetic
<400> 589
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 590
   <211> 327
   <212> PRT
   <213> Artificial Sequence
<220>
   <223> Synthetic
<400> 590
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325